# EUROPEAN PATENT APPLICATION

(11) **EP 4 644 374 A1**
(43) Date of publication of application: **05.11.2025**
(21) Application number: 23910675.0
(22) Date of filing: 27.12.2023
(51) Int. Cl.: C07D 215/22, A61K 31/47, A61K 31/44, A61P 35/00

(54) **NAPHTHYLAMIDE COMPOUND, AND PREPARATION METHOD THEREFOR AND USE THEREOF**

(30) Priority: 28.12.2022 CN 202211694771
(71) Applicant: Shenzhen Chipscreen Biosciences Co., Ltd., Guangdong 518057 (CN)
(72) Inventor: YANG, Qianjiao, Shenzhen, Guangdong 518057 (CN); SHAN, Song, Shenzhen, Guangdong 518057 (CN); ZHOU, You, Shenzhen, Guangdong 518057 (CN); DENG, Zhiting, Shenzhen, Guangdong 518057 (CN); ZHANG, Yu, Shenzhen, Guangdong 518057 (CN); WANG, Xiaoliang, Shenzhen, Guangdong 518057 (CN); SONG, Yonglian, Shenzhen, Guangdong 518057 (CN); PAN, Desi, Shenzhen, Guangdong 518057 (CN)
(74) Representative: Lavoix
(86) International application number: PCT/CN2023/142173
(87) International publication number: WO 2024/140754

(57) **Abstract**

A naphthylamide compound as represented by formula (I), a preparation method therefor, and the use thereof in the treatment and/or prevention of diseases related to the biological activity of the protein kinase.

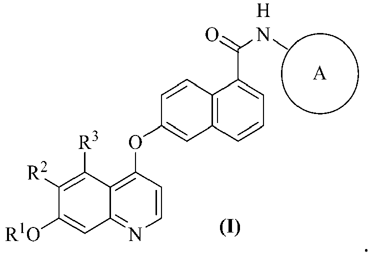

## Description

### TECHNICAL FIELD

The present disclosure pertains to the field of pharmaceutical chemistry and particularly relates to a naphthylamide compound as an inhibitor of a protein kinase, a preparation method therefor, and use thereof in the treatment and/or prevention of a disease associated with the bioactivity of the protein kinase.

### BACKGROUND

Vascular endothelial growth factor receptors (VEGFRs) are a family of receptor tyrosine kinases (RTKs) that includes three structurally similar transmembrane protein members: VEGFR1 (also known as feline McDonough sarcoma (fms)-related tyrosine kinase-1, Flt-1), VEGFR2 (also known as kinase insert domain receptor (KDR)), and VEGFR3 (also known as Flt-4) (Moser C, et al., Clin. Colorectal Cancer., 2007, 6(8):564-71). VEGFRs are expressed in endothelial cells and non-endothelial cells including tumor cells, and these receptors are capable of binding to 5 natural ligands: VEGF-A, VEGF-B, VEGF-C, VEGF-D, and PlGF (placental growth factor). VEGF-A plays an irreplaceable role in the angiogenesis and maintenance processes, and VEGF-C and VEGF-D are ligands mediating lymphangiogenesis. Among all these receptors, VEGFR2 plays the most central role and is pivotal in the regulation of angiogenesis, vascular development, vascular permeability, and embryonic hematopoiesis.

The diffusion limit of oxygen in mammalian tissue is about 100-200 µM. If there are no new blood vessels supplying sufficient blood oxygen but only tissue diffusion of oxygen, the growth diameter of metabolically active solid tumor tissues will be confined to the range of 0.2-2.0 mm due to a lack of oxygen. Thus, anti-angiogenesis has become an effective treatment method for various solid tumors (Adair TH, et al., Angiogenesis, Morgan & Claypool Life Sciences: San Rafael, CA, USA, 2010).

Currently, there are two major categories of approved drugs targeting tumor angiogenesis and VEGF-VEGFR pathways. One category is monoclonal antibodies, such as bevacizumab, which was first approved in 2004, and ramucirumab, which was approved in 2014. The other category is VEGFR small-molecule inhibitors, mainly including sorafenib, axitinib, apatinib, sunitinib, regorafenib, vandetanib, pazopanib, lenvatinib, ponatinib, cabozantinib, fruquintinib, etc.

Anti-angiogenic drugs have greatly improved the therapeutic prospects for various solid tumors. However, hypertension is one of the most common toxic side effects of VEGF-VEGFR inhibitors, and almost 100% of patients treated with these drugs experienced elevations in blood pressure. Studies have shown that hypertension induced by VEGF-VEGFR inhibitors is a mechanism-dependent target toxicity that often results in dose adjustments during treatment or treatment interruption (Camarda N, et al., Curr. Oncol. Rep., 2022 Apr; 24(4):463-74).

Aurora kinases (AURKs) are an important class of serine/threonine protein kinases that regulate cell cycles. The human AURK family includes three members: Aurora A, Aurora B, and Aurora C, and only Aurora A and Aurora B are expressed at detectable levels in all somatic cells (Tang A, et al., Oncotarget., 2017, 8:23937-54).

Relative to normal paracancerous tissues, Aurora B is overexpressed in tumors of various tissue origins. This kinase is encoded by the AURORA B gene located on chromosome 17 and is also referred to as AIK2, AIM1, ARK2, AIRK2, IPL1, STK1, STK5, and STK12. The phosphorylation of site Ser10 of histone H3 by this kinase is crucial for sister chromatid separation. Exogenous overexpression of Aurora B increases histone H3 phosphorylation and chromosomal aneuploidy in normal cells, and injection of these Aurora B-overexpressing cells into BALB/c nu/nu mice results in tumor formation, indicating that Aurora B plays a driving role in tumorigenesis (Ota T, et al., Cancer Res., 2002, 62 (18):5168-77).

Inhibiting the activity of Aurora B, as an anti-tumor target, blocks the abnormal connection of kinetochores and microtubules, preventing chromosome arrangement and separation. This further causes mitotic catastrophe, leading to apoptosis. In addition, the loss of Aurora B kinase activity leads to cytokinesis failure and the formation of tetraploid (or polyploid) cells, and such severe genomic instability eventually results in cell death. Aurora B is only expressed in dividing and proliferating cells and is mostly in a stable state in normal cells. Therefore, the targeted inhibition of Aurora B has strong selectivity for actively dividing and proliferating tumor cells, offering greater advantages over other non-specific cytotoxic drugs.

To date, no AURK inhibitors have been approved globally. However, clinical research on such inhibitors is relatively active, including pan-AURK inhibitors such as VX-680/MK-0475 (tozasertib), PHA-793358 (danusertib), AT-9283, AMG 900, KW-2449, ABT-348 (ilorasertib), and TT00420; Aurora A selective inhibitors such as MLN8237, ENMD 2076, VX-689, and LY3295668; and Aurora B selective inhibitors such as AZD1152 (barasertib) and chiauranib (Jing X, et al., Expert. Opin. Ther. Pat., 2021, 31(7):625-44). The existing evidence indicates, to some degree, that Aurora A or Aurora B selective inhibitors demonstrate better druggability than pan-AURK inhibitors by avoidance of more toxicity.

Chiauranib is a small-molecule inhibitor targeting multiple kinase pathways including VEGFR, PDGFR, Aurora B, etc. developed by Shenzhen Chipscreen Biosciences Co., Ltd., and it is undergoing clinical trials for the treatment of tumors. According to the phase I clinical trial results of chiauranib, the dose-limiting toxicity (DLT) of chiauranib occurred in the 65-mg treatment group as grade 3 hypertension, a toxicity associated with VEGFR pathway inhibition. Its recommended phase II clinical trial dose was 50 mg. At this dose, once-daily administration for 28 consecutive days achieved a maximum plasma exposure (Cₘₐₓ) of about 2.6 µM and a steady-state plasma exposure (C_{trough}) of about 1 µM (Sun Y, et al., J. Hematol. Oncol., 2019, 12(1):9). The DLT of chiauranib in the human body is related to VEGFR pathway inhibition, suggesting that the VEGFR pathway may be the dominant pathway for the *in vivo* efficacy of chiauranib. According to *in vitro* cytological assays, chiauranib exhibited relatively weak inhibitory activity against Aurora B kinase at 1-3 µM (comparable to *in vivo* drug exposure levels), suggesting that the drug target mechanism may be limited in the treatment of related Aurora B kinase-dependent tumors. Therefore, multi-target kinase inhibitors showing stronger selectivity for Aurora B kinase and higher inhibitory activity against Aurora B than against VEGFR2 may achieve better therapeutic effects on Aurora B kinase-dependent tumors within similar human exposure and maximum tolerated dose (MTD) ranges.

### SUMMARY

### Problems to be Solved by the Present Disclosure:

The inhibitory activity against Aurora B kinase in the prior art is relatively weak, and excessive inhibition of VEGFR2 can lead to toxic side effects such as hypertension. Particularly, where the selectivity for Aurora B kinase is weaker than that for VEGFR2, the full therapeutic effect is limited in the treatment of tumors associated with the bioactivity of Aurora B kinase. In addition, the treatment of the primary and metastatic tumors of the central nervous system requires that the therapeutic drugs be able to cross the blood-brain barrier and achieve effective intracranial exposure to exert their anti-tumor effects. Therefore, the present disclosure aims to provide a compound showing higher selectivity for Aurora B kinase than for VEGFR2. The related compound exhibits more significant inhibitory activity against Aurora B kinase at the cellular level under reachable plasma exposure conditions and can provide better comprehensive benefits in terms of efficacy/safety for related diseases. In addition, the compound of the present disclosure has a good ability to cross the blood-brain barrier and is suitable for use in the treatment and/or prevention of a disease of the central nervous system.

### Solutions to Problems:

To solve the above problems, the inventors of the present application carried out intensive research and found that a naphthylamide compound with a specific structure can achieve the aims, thus resulting in the present disclosure.

The present disclosure relates to the following naphthylamide compound:
a compound represented by formula (I) or a stereoisomer, tautomer, polymorph, co-crystal, solvate, metabolite, prodrug, deuterated compound, or pharmaceutically acceptable salt thereof: 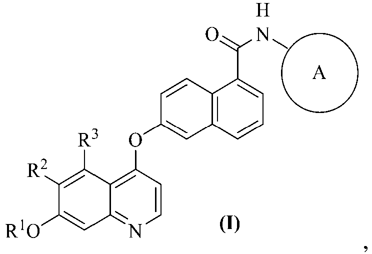 wherein:
R¹ is selected from methyl and ethyl; the methyl or ethyl is unsubstituted or substituted with one or more identical or different R^{a};
each R^{a} is independently selected from halogen, C₁₋₆ alkoxy, -NR'R", and halogenated C₁₋₆ alkoxy; wherein R' and R" are each independently selected from H, C₁₋₆ alkyl, and C₃₋₆ cycloalkyl, or R' and R", together with the N atom to which they are attached, form 4-8 membered nitrogen-containing heterocyclyl; the 4-8 membered nitrogen-containing heterocyclyl may additionally comprise 1-2 heteroatoms selected from N, O, and S;
R² and R³ are each independently selected from: H, halogen, and C₁₋₆ alkyl;
ring A is selected from C₄₋₁₀ cycloalkyl and 4-10 membered heterocyclyl; the 4-10 membered heterocyclyl comprises 1 to 4 heteroatoms selected from N, O, and S; the C₄₋₁₀ cycloalkyl or 4-10 membered heterocyclyl is unsubstituted or substituted with one or more identical or different R^{b};
each R^{b} is independently selected from halogen, C₁₋₆ alkyl, and halogenated C₁₋₆ alkyl; or, ring A is selected from the groups: 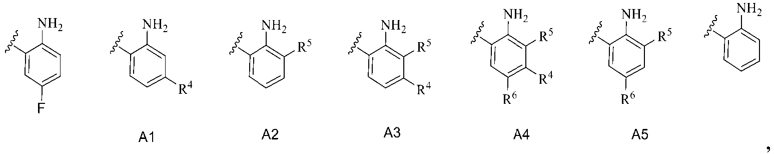 wherein:
   in A1, R⁴ is selected from F, Cl, C₁₋₆ alkyl, and halogenated C₁₋₆ alkyl;
   in A2, R⁵ is selected from halogen, C₁₋₆ alkyl, and halogenated C₁₋₆ alkyl;
   in A3, R⁴ and R⁵ are each independently selected from halogen, C₁₋₆ alkyl, and halogenated C₁₋₆ alkyl;
   in A4, R⁶ is selected from F and Cl, and R⁴ and R⁵ are each independently selected from halogen, C₁₋₆ alkyl, and halogenated C₁₋₆ alkyl; when R⁶ is Cl, R¹ is methyl or ethyl;
   in A5, R⁵ and R⁶ are each independently selected from halogen, C₁₋₆ alkyl, and halogenated C₁₋₆ alkyl;
   furthermore, when ring A is 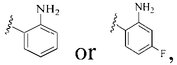 R¹ is ethyl, or methyl or ethyl substituted with one or more R^{a}.

In some specific embodiments, R¹ is selected from methyl and ethyl; the methyl or ethyl is unsubstituted or substituted with 1, 2, or 3 identical or different R^{a}.

In some specific embodiments, each R^{a} is independently selected from halogen, C₁₋₄ alkoxy, -NR'R", and halogenated C₁₋₄ alkoxy; wherein R' and R" are each independently selected from H, C₁₋₄alkyl, and C₃₋₅ cycloalkyl, or R' and R", together with the N atom to which they are attached, form 4-8 membered nitrogen-containing heterocyclyl; the 4-8 membered nitrogen-containing heterocyclyl may additionally comprise 1-2 heteroatoms selected from N, O, and S.

In some specific embodiments, each R^{a} is independently selected from halogen, C₅₋₆ alkoxy, -NR'R", and halogenated C₅₋₆ alkoxy; wherein R' and R" are each independently selected from H, C₅₋₆ alkyl, and C₅₋₆ cycloalkyl, or R' and R", together with the N atom to which they are attached, form 4-8 membered nitrogen-containing heterocyclyl; the 4-8 membered nitrogen-containing heterocyclyl may additionally comprise 1-2 heteroatoms selected from N, O, and S.

In some specific embodiments, each R^{a} is independently selected from halogen, C₁₋₃ alkoxy, -NR'R", and halogenated C₁₋₃ alkoxy; wherein R' and R" are each independently selected from H, C₁₋₃ alkyl, and C₃₋₄ cycloalkyl, or R' and R", together with the N atom to which they are attached, form 4-8 membered nitrogen-containing heterocyclyl; the 4-8 membered nitrogen-containing heterocyclyl may additionally comprise 1-2 heteroatoms selected from N, O, and S.

In some specific embodiments, each R^{a} is independently selected from halogen, methoxy, ethoxy, propoxy, -NR'R", halogenated methoxy, halogenated ethoxy, and halogenated propoxy; wherein R' and R" are each independently selected from H, methyl, and ethyl, or R' and R", together with the N atom to which they are attached, form 4-8 membered nitrogen-containing heterocyclyl; the 4-8 membered nitrogen-containing heterocyclyl may additionally comprise 1-2 heteroatoms selected from N, O, and S.

In some specific embodiments, each R^{a} is independently selected from F, Cl, Br, methoxy, ethoxy, propoxy, -NR'R", halogenated methoxy, halogenated ethoxy, and halogenated propoxy; wherein R' and R" are each independently selected from H, methyl, and ethyl, or R' and R", together with the N atom to which they are attached, form 4-7 membered nitrogen-containing heterocyclyl; the 4-7 membered nitrogen-containing heterocyclyl may additionally comprise 1-2 heteroatoms selected from N, O, and S.

In some specific embodiments, each R^{a} is independently selected from F, Cl, Br, methoxy, ethoxy, -NR'R", and methoxy or ethoxy substituted with 1-3 F or Cl; wherein R' and R" are each independently selected from methyl and ethyl, or R' and R", together with the N atom to which they are attached, form 4-7 membered nitrogen-containing heterocyclyl; the 4-7 membered nitrogen-containing heterocyclyl may additionally comprise 1-2 heteroatoms selected from N, O, and S.

In some specific embodiments, each R^{a} is independently selected from F, Cl, methoxy, ethoxy, -NR'R", -OCH₂F, -OCHF₂, -OCF₃, -OCH₂CH₂F, -OCH₂CHF₂, and -OCH₂CF₃; wherein R' and R" are each independently selected from methyl and ethyl, or R' and R", together with the N atom to which they are attached, form 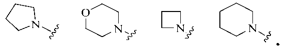 In some specific embodiments, each R^{a} is independently selected from F, Cl, methoxy, N,N-dimethylamino, N-methylamino, -OCHF₂, and 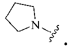 In some specific embodiments, R¹ is selected from methyl, ethyl, -CH₂CH₂F, -CH₂CHF₂, -CH₂CH₂Cl, methoxyethyl, and

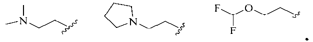

In some specific embodiments, when ring A is 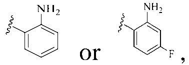 R¹ is ethyl or -CH₂CH₂F.

In some specific embodiments, R¹ is selected from methyl and ethyl, and the methyl or ethyl is unsubstituted.

In some specific embodiments, ring A is selected from C₄₋₇ monocycloalkyl, C₅₋₁₀ spirobicycloalkyl, 4-7 membered monoheterocyclyl, and 6-10 membered spirobiheterocyclyl; the 4-7 membered monoheterocyclyl or 6-10 membered spirobiheterocyclyl comprises 1 to 4 heteroatoms selected from N, O, and S; the C₄₋₇ monocycloalkyl, C₅₋₁₀ spirobicycloalkyl, 4-7 membered monoheterocyclyl, or 6-10 membered spirobiheterocyclyl is unsubstituted or substituted with 1, 2, or 3 identical or different R^{b}.

In some specific embodiments, ring A is selected from C₄₋₆ monocycloalkyl, C₆₋₈ spirobicycloalkyl, 4-7 membered monoheterocyclyl, and 6-8 membered spirobiheterocyclyl; the 4-7 membered monoheterocyclyl or 6-8 membered spirobiheterocyclyl comprises 1 to 2 heteroatoms selected from N, O, and S; the C₄₋₆ monocycloalkyl, C₆₋₈ spirobicycloalkyl, 4-7 membered monoheterocyclyl, or 6-8 membered spirobiheterocyclyl is unsubstituted or substituted with 1, 2, or 3 identical or different R^{b}.

In some specific embodiments, ring A is selected from 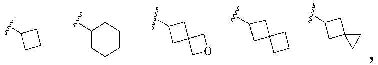 and the 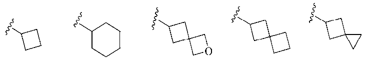 are unsubstituted or substituted with 1, 2, or 3 identical or different R^{b}.

In some specific embodiments, each R^{b} is independently selected from halogen and C₁₋₆ alkyl.

In some specific embodiments, each R^{b} is independently selected from F, Cl, Br, C₁₋₄ alkyl, and C₅₋₆ alkyl.

In some specific embodiments, each R^{b} is independently selected from F, Cl, Br, methyl, ethyl, C₃ alkyl, C₄ alkyl, C₅ alkyl, and C₆ alkyl.

In some specific embodiments, each R^{b} is independently selected from F, Cl, Br, methyl, ethyl, propyl, isopropyl, butyl, tert-butyl, and sec-butyl.

In some specific embodiments, each R^{b} is independently selected from F, Cl, Br, and methyl.

In some specific embodiments, each R^{b} is independently selected from F.

In some specific embodiments, ring A is selected from

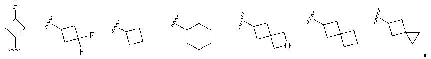

In some specific embodiments, in A1, R⁴ is selected from F, Cl, C₁₋₄ alkyl, C₅₋₆ alkyl, halogenated C₁₋₄ alkyl, and halogenated C₅₋₆ alkyl.

In some specific embodiments, in A1, R⁴ is selected from F, Cl, C₁₋₄ alkyl, and halogenated C₁₋₄ alkyl.

In some specific embodiments, in A1, R⁴ is selected from F, Cl, methyl, ethyl, propyl, isopropyl, C₁₋₄ alkyl, halogenated methyl, halogenated ethyl, halogenated propyl, halogenated isopropyl, and halogenated C₄ alkyl.

In some specific embodiments, in A1, R⁴ is selected from F, Cl, methyl, ethyl, methyl substituted with fluorine or chlorine, and ethyl substituted with fluorine or chlorine.

In some specific embodiments, in A1, R⁴ is selected from F, Cl, and methyl.

In some specific embodiments, in A2, R⁵ is selected from F, Cl, Br, C₁₋₄ alkyl, C₅₋₆ alkyl, halogenated C₁₋₄ alkyl, and halogenated C₅₋₆ alkyl.

In some specific embodiments, in A2, R⁵ is selected from F, Cl, Br, C₁₋₄ alkyl, and halogenated C₁₋₄ alkyl.

In some specific embodiments, in A2, R⁵ is selected from F, Cl, Br, C₅₋₆ alkyl, and halogenated C₅₋₆ alkyl.

In some specific embodiments, in A2, R⁵ is selected from F, Cl, Br, methyl, ethyl, propyl, isopropyl, C₄ alkyl, halogenated methyl, halogenated ethyl, halogenated propyl, halogenated isopropyl, and halogenated C₄ alkyl.

In some specific embodiments, in A2, R⁵ is selected from F, Cl, Br, methyl, ethyl, methyl substituted with fluorine or chlorine, and ethyl substituted with fluorine or chlorine.

In some specific embodiments, in A2, R⁵ is selected from F, Cl, Br, and methyl.

In some specific embodiments, in A2, R⁵ is selected from F.

In some specific embodiments, in A3, R⁴ and R⁵ are each independently selected from halogen, C₁₋₄ alkyl, C₅₋₆ alkyl, halogenated C₁₋₄ alkyl, and halogenated C₅₋₆ alkyl.

In some specific embodiments, in A3, R⁴ and R⁵ are each independently selected from F, Cl, Br, C₁₋₄ alkyl, and halogenated C₁₋₄ alkyl.

In some specific embodiments, in A3, R⁴ and R⁵ are each independently selected from F, Cl, Br, methyl, ethyl, propyl, isopropyl, C₄ alkyl, halogenated methyl, halogenated ethyl, halogenated propyl, halogenated isopropyl, and halogenated C₄ alkyl.

In some specific embodiments, in A3, R⁴ and R⁵ are each independently selected from F, Cl, Br, methyl, ethyl, methyl substituted with fluorine or chlorine, and ethyl substituted with fluorine or chlorine.

In some specific embodiments, in A3, R⁴ and R⁵ are each independently selected from F and Cl.

In some specific embodiments, in A3, R⁴ is F, and R⁵ is F or Cl.

In some specific embodiments, ring A is selected from

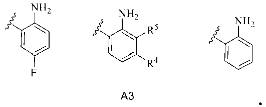

In some specific embodiments, in A4, R⁶ is selected from F and Cl, and R⁴ and R⁵ are each independently selected from halogen, C₁₋₄ alkyl, C₅₋₆ alkyl, halogenated C₁₋₄ alkyl, and halogenated C₅₋₆ alkyl; when R⁶ is Cl, R¹ is methyl or ethyl.

In some specific embodiments, in A4, R⁶ is selected from F and Cl, and R⁴ and R⁵ are each independently selected from F, Cl, Br, C₁₋₄ alkyl, and halogenated C₁₋₄ alkyl; when R⁶ is Cl, R¹ is methyl or ethyl.

In some specific embodiments, in A4, R⁶ is selected from F and Cl, and R⁴ and R⁵ are each independently selected from F, Cl, Br, methyl, ethyl, propyl, isopropyl, C₄ alkyl, halogenated methyl, halogenated ethyl, halogenated propyl, halogenated isopropyl, and halogenated C₄ alkyl; when R⁶ is Cl, R¹ is methyl or ethyl.

In some specific embodiments, in A4, R⁶ is selected from F and Cl; R⁴ and R⁵ are each independently selected from F, Cl, Br, methyl, ethyl, methyl substituted with fluorine or chlorine, and ethyl substituted with fluorine or chlorine; when R⁶ is Cl, R¹ is methyl or ethyl.

In some specific embodiments, in A4, R⁶ is selected from F and Cl, and R⁴ and R⁵ are each independently selected from F and Cl; when R⁶ is Cl, R¹ is methyl or ethyl.

In some specific embodiments, in A5, R⁵ and R⁶ are each independently selected from halogen, C₁₋₄ alkyl, C₅₋₆ alkyl, halogenated C₁₋₄ alkyl, and halogenated C₅₋₆ alkyl.

In some specific embodiments, in A5, R⁵ and R⁶ are each independently selected from F, Cl, Br, C₁₋₄ alkyl, and halogenated C₁₋₄ alkyl.

In some specific embodiments, in A5, R⁵ and R⁶ are each independently selected from F, Cl, Br, methyl, ethyl, propyl, isopropyl, C₄ alkyl, halogenated methyl, halogenated ethyl, halogenated propyl, halogenated isopropyl, and halogenated C₄ alkyl.

In some specific embodiments, in A5, R⁵ and R⁶ are each independently selected from F, Cl, Br, methyl, ethyl, methyl substituted with fluorine or chlorine, and ethyl substituted with fluorine or chlorine.

In some specific embodiments, in A5, R⁵ and R⁶ are each independently selected from F and Cl.

In some specific embodiments, ring A is selected from

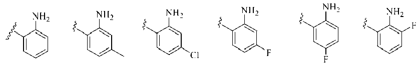

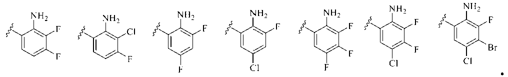

In some specific embodiments, ring A is selected from 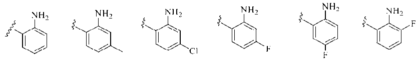 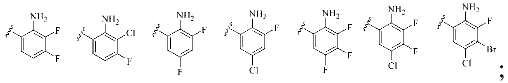 when ring A is selected from 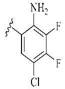 and 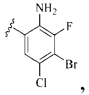 R¹ is methyl or ethyl.

In some embodiments, ring A is selected from

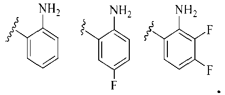

In some specific embodiments, ring A is selected from

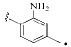

In some specific embodiments, ring A is selected from

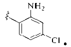

In some specific embodiments, ring A is selected from

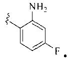

In some specific embodiments, ring A is selected from

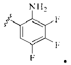

In some specific embodiments, ring A is selected from 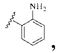 and R¹ is ethyl or -CH₂CH₂F.

In some specific embodiments, ring A is selected from 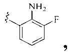 and R¹ is ethyl or -CH₂CH₂F.

In some specific embodiments, ring A is selected from

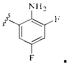

In some specific embodiments, ring A is selected from

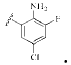

In some specific embodiments, ring A is selected from

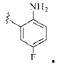

In some specific embodiments, ring A is selected from

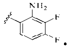

In some specific embodiments, ring A is selected from

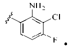

In some specific embodiments, ring A is selected from 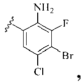 and R¹ is methyl or ethyl.

In some specific embodiments, ring A is selected from 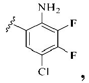 and R¹ is methyl or ethyl.

In some specific embodiments, R² and R³ are each independently selected from H, halogen, C₁₋₄ alkyl, and C₅₋₆ alkyl.

In some specific embodiments, R² and R³ are each independently selected from H, F, Cl, Br, methyl, ethyl, propyl, isopropyl, butyl, pentyl, and hexyl.

In some specific embodiments, R² and R³ are each independently selected from H, F, Cl, and Br.

In some specific embodiments, R² and R³ are each independently selected from H and F. In some specific embodiments, R² and R³ are both H.

In some specific embodiments, one of R² and R³ is H, and the other is F.

In some specific embodiments, R² is H, and R³ is F.

In some specific embodiments, R² is F, and R³ is H.

In some specific embodiments, the above compound is selected from:

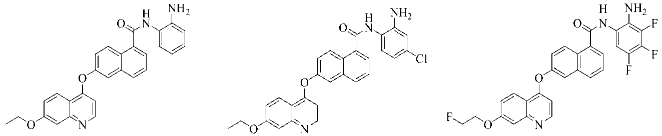

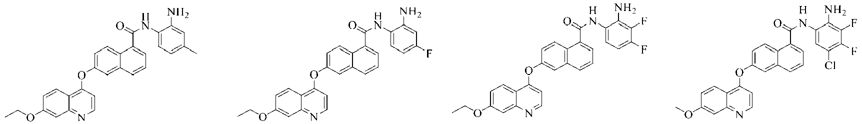

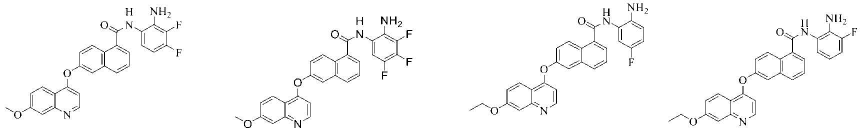

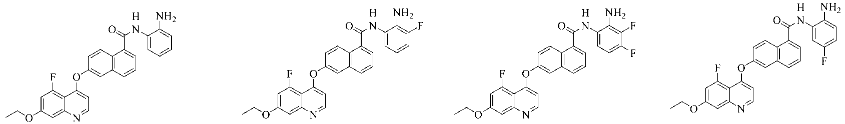

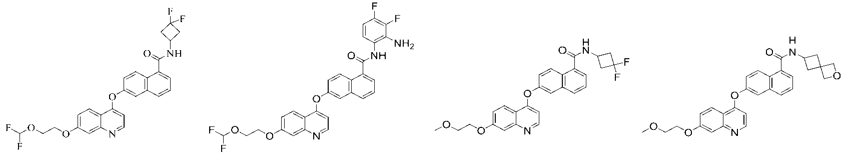

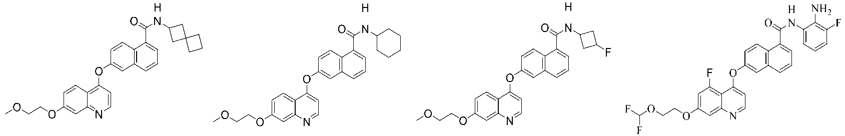

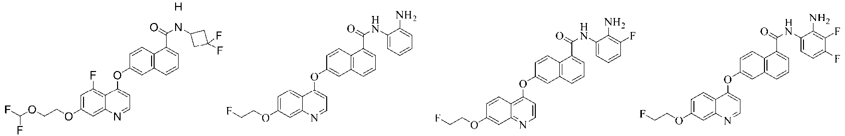

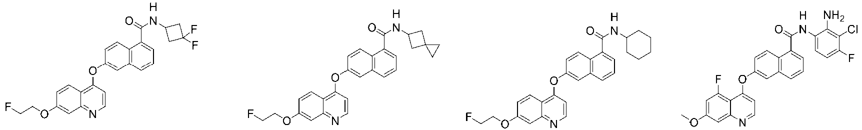

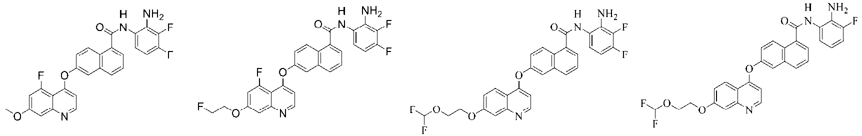

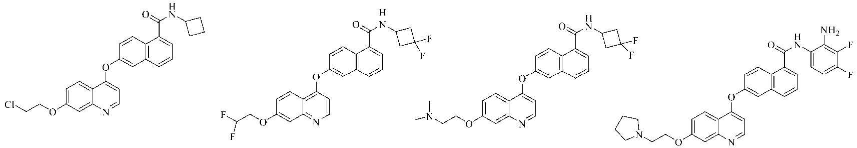

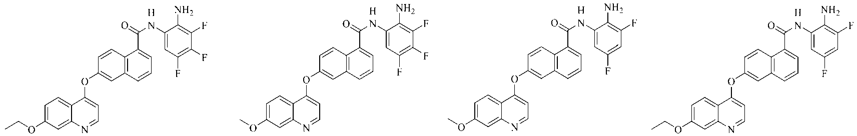

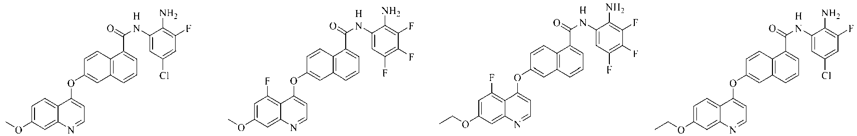

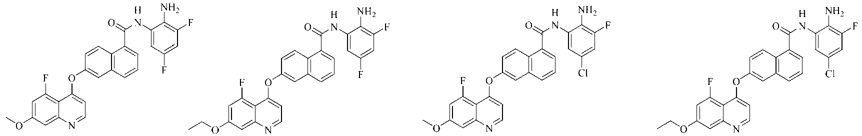

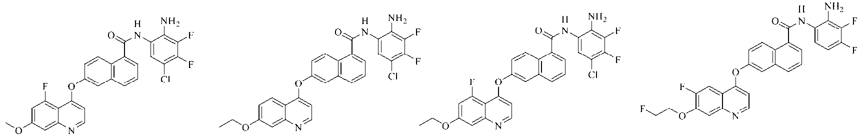

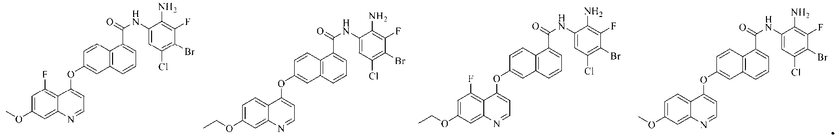

As used herein, an example of the term "pharmaceutically acceptable salt of the compound represented by formula (I)" is an organic acid addition salt formed from an organic acid that forms a pharmaceutically acceptable anion.

The term "pharmaceutically acceptable salt" refers to a salt of the compound of the present disclosure prepared from the compound with specific substituents discovered in the present disclosure and a relatively non-toxic acid or base. When the compound of the present disclosure comprises a relatively acidic functional group, a base addition salt may be obtained by contacting a sufficient amount of base with the compound in a pure solution or a suitable inert solvent. When the compound of the present disclosure comprises a relatively basic functional group, an acid addition salt may be obtained by contacting a sufficient amount of acid with the compound in a pure solution or a suitable inert solvent.

The term "prodrug" refers to derivatives of the compound represented by formula (I) with particular substituents discovered in the present disclosure. These derivatives may themselves exhibit weak activity or even no activity, but upon administration, they are converted under physiological conditions (e.g., by metabolism, solvolysis, or other means) into the compound with specific substituents discovered in the present disclosure, thereby producing the corresponding bioactivity *in vivo.*

The term "metabolite" refers to a product obtained *in vivo* through the metabolism of the compound represented by formula (I) with specific substituents discovered in the present disclosure. A metabolite of a compound may be identified by techniques well known in the art to which it pertains, and its activity may be characterized by experimental methods as described in the present disclosure. Such products may be obtained through the oxidation, reduction, hydrolysis, amidation, deamidation, esterification, deesterification, enzymatic cleavage, etc. of the administered compound. Accordingly, the present disclosure includes metabolites of the compound, including metabolites produced by contacting the compound of the present disclosure with a mammal for a sufficient period of time.

The term "deuterated compound" means that the compound of the present disclosure comprises at least one deuterium atom, and specifically means that one or more hydrogen atoms in the compound of the present disclosure can be replaced or substituted by deuterium atoms. In some embodiments, the compound comprises two or more deuterium atoms. In some embodiments, the compound comprises 1, 2, 3, 4, 5, 6, 7, 8, 9, 10, 11, or 12 deuterium atoms. Synthetic methods for including an isotope in an organic compound are known in the art.

When the structural formula of the compound of general formula (I) of the present disclosure is not in accordance with the Chinese name, the structural formula shall prevail.

### Preparation Method:

Thus, another aspect of the present disclosure further provides a preparation method for the above compound according to the present disclosure. The compound of general formula (I) of the present disclosure may be prepared by the following exemplary methods and examples; however, these methods and examples are not construed in any way as limiting the scope of the present disclosure. The compound of the present disclosure may also be synthesized by synthetic techniques known to those skilled in the art, or comprehensively using synthetic methods known in the art and the method of the present disclosure. The product from each reaction step is obtained by separation techniques known in the art, including but not limited to extraction, filtration, distillation, crystallization, chromatography separation, etc. The starting materials and chemical reagents required for the synthesis may be conventionally synthesized according to literature (as provided by Scifinder) or purchased.

The synthetic scheme below describes a preparation method for the compound of formula (I) of the present disclosure, and the starting materials, reagents, catalysts, solvents, etc. used in the synthetic scheme may be prepared by methods well known to those of ordinary skill in the art of organic chemistry or commercially available. All final derivatives of the present disclosure can be prepared by the method described in the scheme or similar methods, and all these methods are well known to those of ordinary skill in the art of organic chemistry. All variable factors employed in the scheme are as defined in the context.

### Preparation Method

The following variables are as previously defined, and the new variables are as defined in the content of this section. In addition, the compound of general formula (I) and the involved intermediates can all be purified by common separation methods, such as extraction, recrystallization, silica gel column chromatography separation, etc. The 200-300 mesh silica gel and thin-layer chromatography silica gel plates used were all produced by Qingdao Haiyang Chemical Co., Ltd. The chemical reagents used were analytically or chemically pure commercially available products of general reagents and were used without further purification.

The present disclosure provides a preparation method for the compound represented by general formula (I), comprising the following steps: 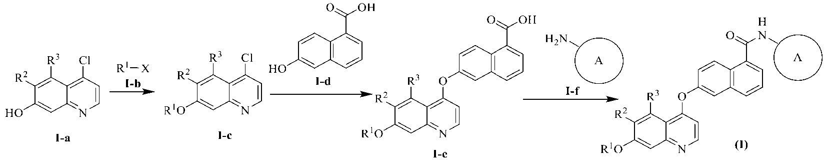
1) subjecting a compound represented by formula (I-a) and a compound represented by formula (I-b) to a nucleophilic substitution reaction in a first solvent under catalysis by a first base to give a compound represented by formula (I-c); or subjecting the compound represented by formula (I-a) and the compound represented by formula (I-b) to a Mitsunobu reaction under the action of triphenylphosphine (PPh₃)/diethyl azodicarboxylate (DEAD) or triphenylphosphine (PPh₃)/diisopropyl azodicarboxylate (DIAD) to give the compound represented by formula (I-c);
2) subjecting the compound represented by formula (I-c) to a nucleophilic substitution reaction with a compound represented by (I-d) in a second solvent under the action of a second base to give a compound represented by formula (I-e); and
3) subjecting the compound represented by formula (I-e) to a condensation reaction with a compound represented by (I-f) in a third solvent under the action of a condensing agent and a third base to give the compound represented by formula (I),
   wherein:
   R¹, R², R³, and ring A are as previously defined;
   X is selected from bromine, iodine, -OMs, -OTf, -OTs, and -OH.

In some embodiments, in step 1), the first solvent is selected from dichloromethane (DCM), 1,2-dichloroethane, 1,4-dioxane, tetrahydrofuran (THF), acetonitrile (MeCN), *N,N*-dimethylformamide (DMF), *N*-methylpyrrolidone (NMP), and a combination thereof.

In some embodiments, in step 1), the first base is selected from potassium carbonate (K₂CO₃), cesium carbonate (Cs₂CO₃), and a combination thereof.

In some embodiments, in step 2), the second solvent is selected from 1,2-dichloroethane, 1,4-dioxane, tetrahydrofuran (THF), acetonitrile (MeCN), *N*,*N*'-dimethylformamide (DMF), *N*-methylpyrrolidone (NMP), isopropanol, dimethylsulfoxide (DMSO), and a combination thereof.

In some embodiments, in step 2), the second base is selected from potassium carbonate (K₂CO₃), cesium carbonate (Cs₂CO₃), diisopropylethylamine (DIEA), and a combination thereof.

In some embodiments, in step 3), the condensing agent is selected from 1-(3-dimethylaminopropyl)-3-ethylcarbodiimide hydrochloride (EDCI HCl), 1-hydroxybenzotriazole (HOBt), 2-(7-azabenzotriazol-1-yl)-*N*,*N*,*N*',*N*'-tetramethyluronium hexafluorophosphate (HATU), *N*,*N*,*N*',*N*'-tetramethylchloroformamidinium hexafluorophosphate (TCFH), *N*-methylimidazole, benzotriazol-1-yloxytripyrrolidinophosphonium hexafluorophosphate (PyBOP), and a combination thereof, wherein TCFH is specifically suitable for condensation of an amine with relatively large steric hindrance.

In some embodiments, in step 3), the third solvent is selected from dichloromethane (DCM), 1,4-dioxane, tetrahydrofuran (THF), acetonitrile (MeCN), N,N-dimethylformamide (DMF), and a combination thereof.

In some embodiments, the third base is selected from triethylamine (TEA) and diisopropylethylamine (DIEA).

### Pharmaceutical Composition:

The present disclosure further provides a pharmaceutical composition comprising any aforementioned compound or stereoisomer, tautomer, polymorph, co-crystal, solvate, metabolite, prodrug, deuterated compound, or pharmaceutically acceptable salt thereof, and optionally a pharmaceutically acceptable carrier and/or adjuvant and/or diluent.

In some embodiments, the pharmaceutical composition may further comprise an additional drug for treating and/or preventing a disease associated with the bioactivity of Aurora B kinase.

Methods of preparing various pharmaceutical compositions comprising certain amounts of active ingredients are known or, according to the content of the present disclosure, apparent to those skilled in the art. Methods of preparing the pharmaceutical composition comprise incorporating a suitable pharmaceutical excipient, carrier, diluent, etc., as described in REMINGTON'S PHARMACEUTICAL SCIENCES, Martin, E.W., ed., Mack Publishing Company, 19th ed. (1995).

### Pharmaceutical Use:

Another aspect of the present disclosure relates to the compound or the stereoisomer, tautomer, polymorph, co-crystal, solvate, metabolite, prodrug, deuterated compound, or pharmaceutically acceptable salt thereof described above according to the present disclosure and the pharmaceutical composition described above for use in the treatment and/or prevention of diseases associated with the bioactivity of Aurora B kinase.

Another aspect of the present disclosure relates to use of the compound or the stereoisomer, tautomer, polymorph, co-crystal, solvate, metabolite, prodrug, deuterated compound, or pharmaceutically acceptable salt thereof described above according to the present disclosure and the pharmaceutical composition described above in the manufacture of drugs for treating and/or preventing diseases associated with the bioactivity of Aurora B kinase.

A method for treating and/or preventing diseases associated with the bioactivity of Aurora B kinase, comprising administering to an individual in need thereof a therapeutically/prophylactically effective amount of the compound or the stereoisomer, tautomer, polymorph, co-crystal, solvate, metabolite, prodrug, deuterated compound, or pharmaceutically acceptable salt thereof described above or the composition described above.

According to a preferred embodiment of the present disclosure, the diseases associated with the bioactivity of Aurora B kinase are selected from tumors and hyperproliferative diseases.

In the present disclosure, "treatment" or "treating" refers to administering to a subject drugs or other auxiliary therapy to obtain a desired pharmacological and/or physiological effect. The effect may be prophylactic in terms of completely or partially preventing the disease or symptoms thereof, and/or therapeutic in terms of partially or completely stabilizing or curing the disease and/or side effects due to the disease. As used herein, "treatment" or "treating" encompasses any treatment of a disease in a patient, including: (a) preventing a disease or symptom in a patient who is susceptible to the disease or symptom but has not yet been diagnosed as having the disease; (b) inhibiting symptoms of the disease, that is, preventing its development; or (c) alleviating symptoms of the disease, that is, causing regression of the disease or symptom.

In the present disclosure, "subject" refers to a vertebrate. In certain embodiments, the vertebrate refers to a mammal. The mammal includes, but is not limited to, livestock (such as cattle), pets (such as cats, dogs, and horses), primates, mice, and rats. In certain embodiments, the mammal refers to a human.

In the present disclosure, "effective amount" refers to an amount effective, at doses and for periods of time necessary, in achieving a desired therapeutic or prophylactic effect. The "therapeutically effective amount" of a substance/molecule of the present disclosure may vary depending on factors such as the disease state, age, sex, and body weight of the individual, as well as the ability of the substance/molecule to elicit a desired response in the individual. A therapeutically effective amount also encompasses an amount in which any toxic or detrimental consequence of the substance/molecule is outweighed by its therapeutically beneficial effects. "Prophylactically effective amount" refers to an amount effective, at doses and for periods of time necessary, in achieving a desired prophylactic effect. Typically but not necessarily, the prophylactically effective amount will be less than the therapeutically effective amount as the prophylactic dose is used in the subject prior to the onset of the disease or at an early stage of the disease. In the case of cancer, the therapeutically effective amount of the drug can reduce the number of cancer cells, reduce the tumor volume, inhibit (that is, slow to some extent, preferably stop) the infiltration of cancer cells into peripheral organs, inhibit (that is, slow to some extent, preferably stop) tumor metastasis, inhibit tumor growth to some extent, and/or palliate one or more cancer-associated symptoms to some extent.

### Definitions of Terms:

According to a convention in the art, 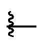 is used in the structural formulas herein to depict the bond that is the point of attachment of the moiety or substituent to the core or main structure.

A dash "-" that is not between two letters or symbols is used to indicate a point of attachment for a substituent. For example, -NR'R" is attached via the N atom.

In each part of the specification, the substituents of the compound of the present disclosure are disclosed according to the group types or ranges. It is specifically noted that the present disclosure includes every individual subcombination of the members of these group types and ranges. For example, the term "C₁₋₆ alkyl" specifically refers to individually disclosed methyl, ethyl, C₃ alkyl, C₄ alkyl, C₅ alkyl, and C₆ alkyl, or individually disclosed "C₁₋₄ alkyl", or individually disclosed "C₁₋₃ alkyl".

The term "alkyl" as used herein is meant to include branched and linear saturated aliphatic hydrocarbon groups with a specified number of carbon atoms. For example, "C₁-₆ alkyl" refers to C₁, C₂, C₃, C₄, C₅, and C₆. In addition, for example, "C₁₋₆ alkyl" refers to an alkyl group having 1 to 6 carbon atoms. An alkyl group may be unsubstituted or substituted such that one or more hydrogen atoms thereof are replaced with another chemical group. Examples of alkyl groups include, but are not limited to, methyl, ethyl, propyl (e.g., n-propyl and isopropyl), butyl (e.g., n-butyl, isobutyl, and tert-butyl), pentyl (e.g., n-pentyl, isopentyl, and neopentyl), etc.

The term "alkoxy" refers to any of the above alkyl groups (e.g., C₁-₆ alkyl, C₁-₄ alkyl, C₁-₃ alkyl, etc.) attached to the remainder of the molecule via an oxygen atom (-O-).

The term "halogenated C₁₋₆ alkyl" or "halogenated C₁₋₆ alkoxy" means that one or more (e.g., 2 or 3) hydrogen atoms in the alkyl or alkoxy are substituted with halogen atoms, such as fluorine, chlorine, bromine, or iodine. The alkyl or alkoxy are as defined above. In some embodiments, the term "halogenated C₁₋₆ alkyl" preferably refers to fluoro- or chloro-substituted C₁₋₆ alkyl; examples include -CF₃, -CHF₂, -CH₂F, -CH₂CH₂F, -CH₂CH₂Cl, -CH₂CHF₂, -CH₂CF₃, etc. In some embodiments, the term "halogenated C₁₋₆ alkoxy" preferably refers to fluoro-substituted C₁₋₆ alkoxy; examples include -OCF₃, -OCHF₂, -OCH₂F, -OCH₂CH₂F, -OCH₂CHF₂, -OCH₂CF₃, etc.

The term "substituted" as used herein means that any one or more hydrogen atoms on a specified atom or group are replaced with a selection from the specified group, provided that the normal valence of the specified atom is not exceeded.

The term "cycloalkyl" refers to a cyclized alkyl group, including monocyclic, bicyclic, or polycyclic ring systems. When a cycloalkyl group is bicyclic or polycyclic, each ring thereof should be a saturated carbocycle or carbocyclic residue. Every two rings of a bicyclic or polycyclic cycloalkyl group may be connected in manners including bridging, fusion, or spiro-linkage. For example, C₃₋₁₀ cycloalkyl is meant to include C₃, C₄, C₅, C₆, C₇, C₈, C₉, and C₁₀ cycloalkyl. Examples of cycloalkyl groups include, but are not limited to, cyclopropyl, cyclobutyl, cyclopentyl, cyclohexyl, 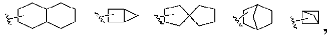 etc.

The term "carbocycle" or "carbocyclic residue" refers to any stable 3-, 4-, 5-, 6-, or 7-membered monocyclic ring or 5-, 6-, 7-, 8-, 9-, 10-, 11-, 12-, 13-, or 14-membered bicyclic or polycyclic ring, wherein any ring may be saturated, partially saturated, unsaturated, or aromatic. Every two rings of a bicyclic or polycyclic carbocycle may be connected in manners including bridging, fusion, or spiro-linkage. Examples of these carbocycles include, but are not limited to, cyclopropyl, cyclobutyl, cyclobutenyl, cyclopentyl, cyclopentenyl, cyclohexyl, cyclohexenyl, cycloheptenyl, cycloheptyl, adamantyl, cyclooctyl, phenyl, naphthyl, [2,2,2]bicyclooctane, 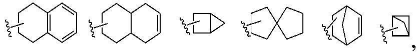 etc.

The terms "heterocycle", "heterocyclic", or "heterocyclyl" are used interchangeably and refer to substituted and unsubstituted 4-8 membered monocyclic or bicyclic groups, 8-10 membered bicyclic or tricyclic groups, and 10-14 membered tricyclic or polycyclic groups, wherein at least one ring has at least one heteroatom (O, S, or N), and the ring containing the heteroatom preferably has 1, 2, or 3 heteroatoms selected from O, S, and N. In these groups, each heteroatom-containing ring may contain 1 to 2 oxygen or sulfur atoms and/or 1 to 4 nitrogen atoms, provided that the total number of heteroatoms in each ring is 4 or less, and further provided that the ring contains at least one carbon atom. In some preferred embodiments, the heteroatom refers only to N or O, and the total number thereof does not exceed 3; preferably, only 1-2 heteroatoms are contained. Carbon and sulfur atoms may be optionally oxidized, and nitrogen atoms may be optionally quaternized. Where the valence permits, a ring atom on a heterocycle may be optionally substituted with =O (oxo) (e.g.: 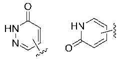 ). Fused rings completing bicyclic and tricyclic groups may contain only carbon atoms and may be saturated, partially saturated, fully unsaturated, aromatic, or non-aromatic. A heterocyclyl group may be attached at any available nitrogen or carbon atom. The term "heterocycle" as used herein does not include a fully aromatic ring.

The term "halogen" refers to fluorine, chlorine, bromine, or iodine.

The term "heteroatom" shall include oxygen, sulfur, and nitrogen.

The term "XXX is unsubstituted or substituted with one or more identical or different YYY" means that XXX may be unsubstituted or substituted with YYY. "Substituted with one or more YYY" means that it may be substituted with one YYY (that is, there is only one YYY substituent) or with multiple YYY substituents, and the YYY substituents are independent of each other; they may be identical or different substituents selected from the corresponding range, and they may substitute at any substitutable position. "More" means 2 or more, preferably 2, 3, or 4, and more preferably 2 or 3.

Throughout the specification, groups and substituents thereof may be chosen by one skilled in the art to provide stable moieties and compounds and compounds useful as pharmaceutically acceptable compounds and/or intermediate compounds useful in preparing pharmaceutically acceptable compounds.

### Effects of the Present Disclosure:

The naphthylamide compound with a specific structure of formula (I) of the present disclosure exhibits excellent inhibitory activity against Aurora B kinase and higher selectivity for Aurora B kinase inhibition than for VEGFR2 inhibition, and can affect the tumor cell cycle to some degree, thereby achieving better comprehensive benefits in terms of efficacy/safety for related diseases. In addition, in a brain permeation assay, the compound of the present disclosure exhibited a ratio of compound concentration in brain tissue to plasma concentration significantly higher than those of control molecule 01 (chiauranib) and control molecule 02. The results of this assay show that: the compound of the present disclosure has superior brain barrier permeability, and thus is capable of crossing the blood-brain barrier and has the potential to achieve an effective plasma concentration in brain tissue, thereby demonstrating its applicability in the treatment and prevention of diseases associated with the central nervous system. In addition, in an anti-tumor efficacy experiment in mice, compared to control molecules 01 and 02, the compound of the present disclosure significantly reduced the tumor volumes and exhibited a significantly improved tumor volume inhibition rate. The results of this experiment show that: the compound of the present disclosure has superior *in vivo* anti-tumor efficacy and is capable of significantly inhibiting tumor growth.

### BRIEF DESCRIPTION OF THE DRAWING:

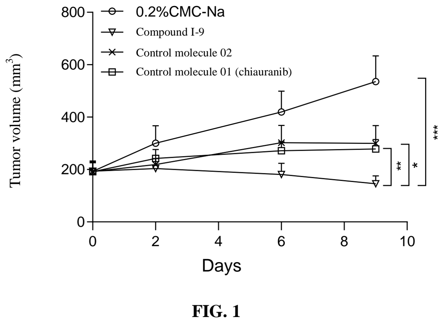

FIG. 1 shows changes in subcutaneous tumor volume of various groups of mice during treatment with the compound I-9 of the present disclosure, control molecule 01 (chiauranib), and control molecule 02. The vertical axis represents the mean tumor volume (mm³), and the horizontal axis represents the number of treatment days post-grouping.

### DETAILED DESCRIPTION

The examples and preparation examples provided in the present disclosure further illustrate and exemplify the compound of the present disclosure and the preparation method therefor. It should be understood that the following preparation examples and examples do not limit the scope of the present disclosure in any way.

It should also be understood that the terms used herein are for the purpose of describing specific examples only, and are not intended to be limiting. In addition, although any methods, apparatus, and materials similar or equivalent to those described herein may be used for practicing or testing the present disclosure, the preferred methods, apparatus, and materials are now described.

### LC-MS Analysis Method:

Mass spectrometry conditions: instrument: Thermo ISQ EC; ion source: ESI (EA+ EA-); source temperature: 300 °C; sheath gas pressure: 50.0 psi; auxiliary gas pressure: 5.0 psi; purge pressure: 0.5 psi; vaporization chamber temperature: 300 °C.

Chromatographic conditions: instrument: Thermo U3000; detector: DAD-3000 (RS) (diode array detector); column: FLM Titank C18 3 µm 4.6 × 50 mm; flow rate: 2.0 mL/min, split flow; column temperature: 35 °C; mobile phase A: water containing 0.05% formic acid and 5% acetonitrile; mobile phase B: acetonitrile containing 0.05% formic acid; elution method: linearly elute with phase A from 100% to 5% within 0-1.0 min, and then maintain 5% phase A for 1.2 min.

### HPLC Analysis Method:

Instrument: Thermo U3000; detector: VWD-3 ×00 (RS) (ultraviolet detector); wavelength: 254 nm; column: Shimadzu inertsil 3 µm 4.6 × 150 mm; flow rate: 0.8 mL/min; column temperature: 35 °C; mobile phase A: water containing 0.05% formic acid and 5% acetonitrile; mobile phase B: acetonitrile containing 0.05% formic acid; elution method: maintain 100% phase A for 1.0 min, linearly elute with phase A from 100% to 5% within 1.0-8.0 min, and finally maintain 5% phase A for 4.0 min.

### ¹H-NMR Analysis Method:

¹H-NMR analysis was performed at room temperature using a BRUKER AVANCE-400 MHz model nuclear magnetic resonance spectrometer in DMSO-d₆, CDCl₃, or the like, with TMS as an internal standard, and signal peaks are represented as s (singlet), d (doublet), t (triplet), q (quartet), m (multiplet), dd (doublet of doublets), and dt (doublet of triplets). The coupling constant (J) is expressed in Hertz (Hz).

In the present disclosure, according to the methods described above, representative compounds **I-1** to **I-44** (see **Table 1**) were prepared, and their purities were measured and recorded. Five compounds (I-7, I-11, I-12, I-13, and I-14) were used as control compounds.

**Table 1**

| **Compound (example)** | **Structural formula** | **% Purity (HPLC)** | **Retention time (min)** | **Compound (example)** | **Structural formula** | **% Purity (HPLC)** | **Retention time (min)** |
|---|---|---|---|---|---|---|---|
| I-1 | 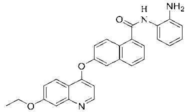 | 99.21 | 5.82 | I-2 | 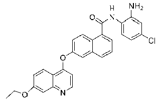 | 93.42 | 6.28 |
| I-3 | 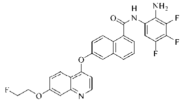 | 93.63 | 7.32 | I-4 | 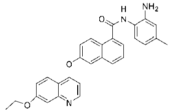 | 97.97 | 6.00 |
| I-5 | 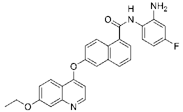 | 99.14 | 6.06 | I-6 | 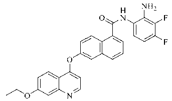 | 98.15 | 6.20 |
| I-7 (comparative example) | 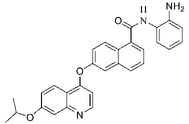 | 97.63 | 5.95 | I-8 | 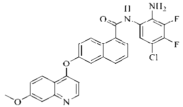 | 94.95 | 7.44 |
| I-9 | 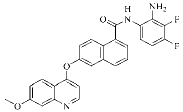 | 99.20 | 5.95 | I-10 | 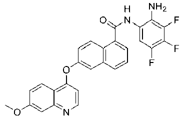 | 98.49 | 6.55 |
| I-11 (comparative example) | 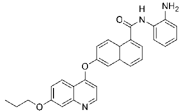 | 99.31 | 6.11 | I-12 (comparative example) | 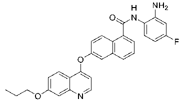 | 98.28 | 6.37 |
| I-13 (comparative example) | 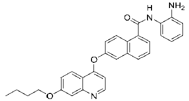 | 91.62 | 6.53 | I-14 (comparative example) | 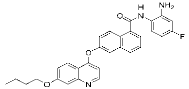 | 98.92 | 6.64 |
| I-15 | 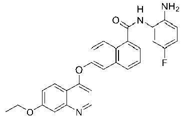 | 99.50 | 6.29 | I-16 | 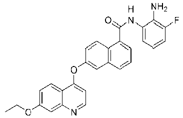 | 99.38 | 6.17 |
| I-17 | 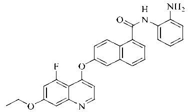 | 98.84 | 6.51 | I-18 | 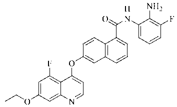 | 96.45 | 6.97 |
| I-19 | 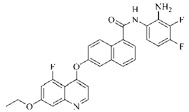 | 98.11 | 7.08 | I-20 | 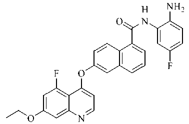 | 98.34 | 6.78 |
| I-21 | 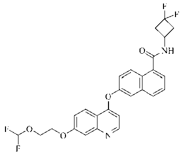 | 97.63 | 6.25 | I-22 | 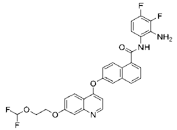 | 98.33 | 6.42 |
| I-23 | 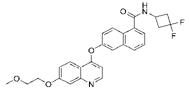 | 99.20 | 5.95 | I-24 | 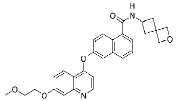 | 99.43 | 4.07 |
| I-25 | 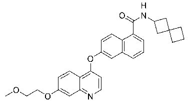 | 98.45 | 5.35 | I-26 | 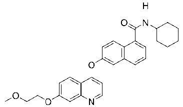 | 98.82 | 5.05 |
| I-27 | 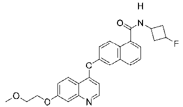 | 98.42 | 4.19 | I-28 | 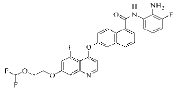 | 91.65 | 7.02 |
| I-29 | 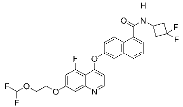 | 96.92 | 7.03 | I-30 | 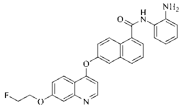 | 98.84 | 5.68 |
| I-31 | 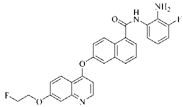 | 95.50 | 6.01 | I-32 | 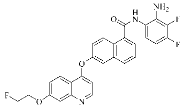 | 98.21 | 5.99 |
| I-33 | 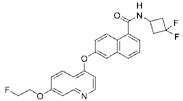 | 93.70 | 7.41 | 1-34 | 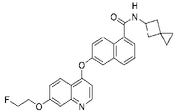 | 94.04 | 7.97 |
| I-35 | 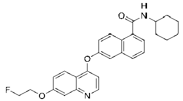 | 98.02 | 6.59 | I-36 | 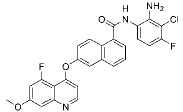 | 95.39 | 7.05 |
| I-37 | 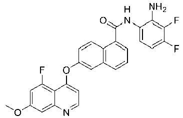 | 95.25 | 6.90 | I-38 | 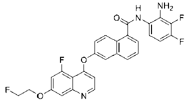 | 97.58 | 7.20 |
| I-39 | 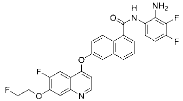 | 97.02 | 6.70 | I-40 | 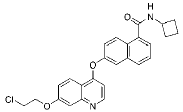 | 98.09 | 4.94 |
| I-41 | 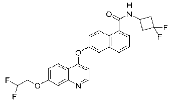 | 89.21 | 12.32 | I-42 | 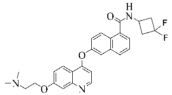 | 95.76 | 6.39 |
| I-43 | 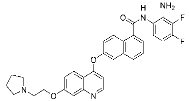 | 95.93 | 4.99 | I-44 | 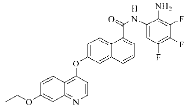 | 92.42 | 6.86 |

The present disclosure is further elucidated below with reference to specific examples. However, the protection scope of the present disclosure is not limited to these examples only. The percentages described in the present disclosure are all weight percentages, unless otherwise indicated. The numerical ranges described in the specification, such as units of measurement, reaction conditions, physical states of compounds, or percentages, are intended to provide an unambiguous written reference. When one skilled in the art practices the present disclosure, using temperatures, concentrations, quantities, numbers of carbon atoms, etc. that fall outside these ranges or differ from the individual values may still result in the desired results. In addition, unless otherwise specified, the starting materials in the following examples are all commercially available; for example, they can be purchased from Shanghai Bide Pharmatech Co., Ltd., Jiangsu Aikon Biopharmaceutical R&D Co., Ltd., Nanjing PharmaBlock Sciences Inc., Shanghai Accela ChemBio Co., Ltd., and He Chun Biological Technology (Shanghai) Co., Ltd.

### Preparation of intermediate: I-1c

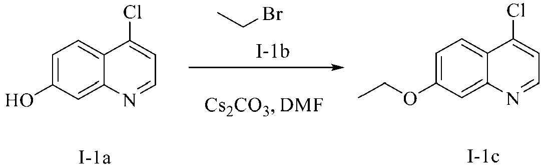

Commercially available I-1a (2.0 g, 11.13 mmol, 1.0 eq) was dissolved in DMF (30 mL), and Cs₂CO₃ (10.88 g, 33.39 mmol, 3.0 eq) and I-1b (3.64 g, 33.39 mmol, 3.0 eq) were added. The mixture was stirred at room temperature for 3 h. The reaction mixture was quenched with water (150 mL), and the precipitated solid was collected by filtration, rinsed 3 times with water (20 mL), and dried in a blast drying oven at 50 °C for 8 h to give intermediate I-1c as a yellow solid (2.23 g, yield: 96.5%). LC-MS MS-ESI (m/z) 208.0 [M+H]⁺.

### Preparation of intermediate: I-1e

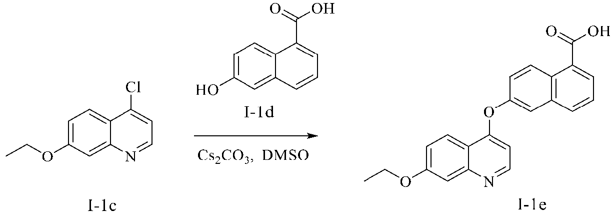

Intermediate I-1c (2.23 g, 10.74 mmol, 1.0 eq) was dissolved in DMSO (30 mL), and Cs₂CO₃ (11.55 g, 35.44 mmol, 3.3 eq) and commercially available I-1d (2.02 g, 10.74 mmol, 1.0 eq) were added. The mixture was heated to 140 °C, stirred for 3 h, and cooled to room temperature. The reaction was quenched with water (150 mL), and the pH was adjusted to 6-7 with 1 N dilute hydrochloric acid. The precipitated solid was collected by filtration, rinsed 3 times with water (20 mL), and dried in a blast drying oven at 50 °C for 8 h to give intermediate I-1e as a brown solid (3.49 g, yield: 90.4%). LC-MS MS-ESI (m/z) 360.1 [M+H]⁺.

### Example 1: Preparation of Compound I-1

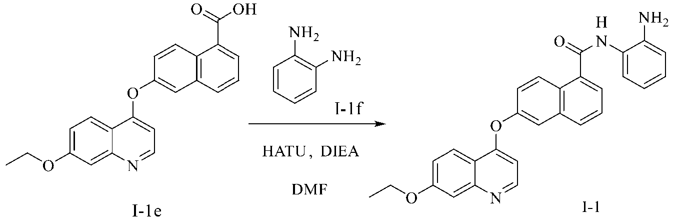

Intermediate I-1e (3.49 g, 9.71 mmol, 1.0 eq) was dissolved in super-dry DMF (40 mL), and commercially available 2-(7-azabenzotriazol-1-yl)-*N*,*N*,*N*',*N*'-tetramethyluronium hexafluorophosphate (HATU, 5.54 g, 14.56 mmol, 1.5 eq), *N,N-*diisopropylethylamine (DIEA, 3.76 g, 29.13 mmol, 3.0 eq), and I-1f (1.05 g, 9.71 mmol, 1.0 eq) were added. The mixture was stirred at room temperature for 18 h. The reaction was quenched with water (200 mL), and the precipitated solid was collected by filtration, rinsed 3 times with water (20 mL), and dried in a blast drying oven at 50 °C for 8 h. The crude product was separated through a silica gel column (200- to 300-mesh silica gel, elution with ethyl acetate (EA)/petroleum ether (PE) = 0-1) and then slurried with EA/PE = 1:1 (30 mL) to give I-1 as a yellow solid (2.30 g, yield: 52.7%). LC-MS MS-ESI (m/z) 450.2 [M+H]⁺. ¹H-NMR (400 MHz, DMSO-d₆) δ ppm 9.91 (s, 1H), 8.64 (d, *J =* 5.2 Hz, 1H), 8.44 (d, *J =* 9.2 Hz, 1H), 8.25 (d, *J =* 9.1 Hz, 1H), 8.09 (d, *J =* 8.2 Hz, 1H), 7.96-7.83 (m, 2H), 7.67 (t, *J =* 7.6 Hz, 1H), 7.58 (dd, *J* = 9.2, 2.0 Hz, 1H), 7.43 (d, *J =* 2.0 Hz, 1H), 7.38 (d, *J =* 7.6 Hz, 1H), 7.32 (dd, *J* = 9.1, 2.2 Hz, 1H), 7.01 (t, *J =* 7.4 Hz, 1H), 6.84 (d, *J =* 7.8 Hz, 1H), 6.66 (t, *J =* 7.4 Hz, 1H), 6.59 (d, *J =* 5.1 Hz, 1H), 5.00 (s, 2H), 4.23 (q, *J =* 6.9 Hz, 2H), 1.44 (t, *J =* 6.9 Hz, 3H).

### Example 2: Preparation of Compound I-2

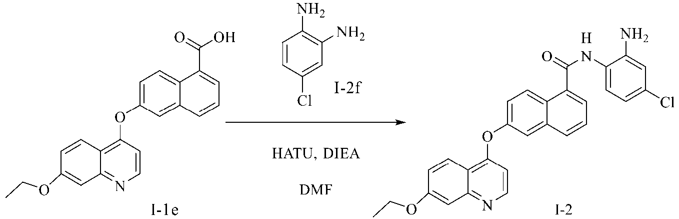

I-2 (yellow solid) was prepared from intermediate I-1e (200.00 mg, 0.56 mmol, 1.0 eq), HATU (319.28 mg, 0.84 mmol, 1.5 eq), DIEA (217.06 mg, 1.68 mmol, 3.0 eq), and I-2f (79.86 mg, 0.56 mmol, 1.0 eq) by steps similar to those in Example I-1. (26.0 mg, yield: 9.6%). LC-MS MS-ESI (m/z) 484.1 [M+H]⁺. ¹H-NMR (400 MHz, DMSO-d₆) δ ppm 9.86 (s, 1H), 8.63 (d, *J* = 5.1 Hz, 1H), 8.42 (d, *J =* 9.2 Hz, 1H), 8.24 (d, *J =* 9.1 Hz, 1H), 8.08 (d, *J =* 8.2 Hz, 1H), 7.96-7.87 (m, 2H), 7.66 (t, *J =* 7.6 Hz, 1H), 7.62-7.50 (m, 1H), 7.46-7.36 (m, 2H), 7.30 (d, *J =* 9.1 Hz, 1H), 6.85 (s, 1H), 6.65 (d, *J =* 8.3 Hz, 1H), 6.58 (d, *J =* 5.1 Hz, 1H), 5.33 (s, 2H), 4.23 (q, *J =* 6.9 Hz, 2H), 1.43 (t, *J =* 6.9 Hz, 3H).

### Preparation of intermediate: I-3c

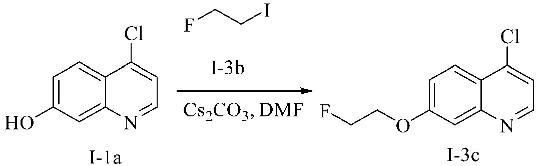

Intermediate I-3c (yellow solid) was prepared from commercially available I-1a (1.79 g, 10.00 mmol, 1.0 eq), Cs₂CO₃ (4.15 g, 30.00 mmol, 3.0 eq), and I-3b (5.22 g, 30.00 mmol, 3.0 eq) by steps similar to those in Example Intermediate I-1c. (1.86 g, yield: 82.4%). LC-MS MS-ESI (m/z) 226.0 [M+H]⁺.

### Preparation of intermediate: I-3e

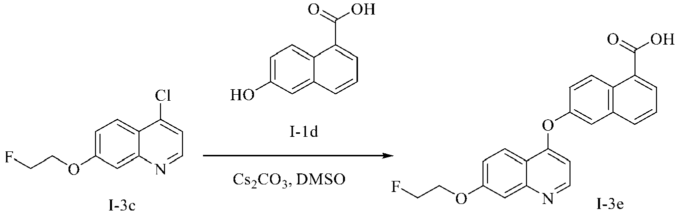

Intermediate I-3e (brown solid) was prepared from intermediate I-3c (1.86 g, 8.24 mmol, 1.0 eq), Cs₂CO₃ (8.86 g, 27.19 mmol, 3.3 eq), and commercially available I-1d (1.55 g, 8.24 mmol, 1.0 eq) by steps similar to those in Example Intermediate I-1e. (2.25 g, yield: 72.3%). LC-MS MS-ESI (m/z) 378.1 [M+H]⁺.

### Example 3: Preparation of Compound I-3

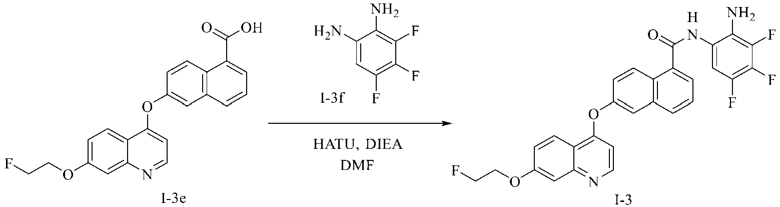

I-3 (yellow solid) was prepared from intermediate I-3e (377.00 mg, 1.00 mmol, 1.0 eq), HATU (570.36 mg, 1.50 mmol, 1.5 eq), DIEA (387.82 mg, 3.00 mmol, 3.0 eq), and I-3f (162.12 mg, 1.00 mmol, 1.0 eq) by steps similar to those in Example I-1. (130.0 mg, yield: 24.9%). LC-MS MS-ESI (m/z) 522.1 [M+H]⁺. ¹H-NMR (400 MHz, DMSO-d₆) δ ppm 10.04 (s, 1H), 8.66 (d, *J =* 5.2 Hz, 1H), 8.44 (d, *J =* 9.2 Hz, 1H), 8.27 (d, *J =* 9.1 Hz, 1H), 8.11 (d, *J =* 8.3 Hz, 1H), 7.95 (d, *J =* 7.0 Hz, 1H), 7.92 (d, *J =* 2.2 Hz, 1H), 7.67 (t, *J* = 7.7 Hz, 1H), 7.58 (dd, *J =* 9.2, 2.4 Hz, 1H), 7.51 (dd, *J =* 14.2, 6.1 Hz, 2H), 7.36 (dd, *J* = 9.1, 2.4 Hz, 1H), 6.60 (d, *J =* 5.2 Hz, 1H), 5.28 (s, 2H), 4.98-4.74 (m, 2H), 4.57- 4.35 (m, 2H).

### Example 4: Preparation of Compound I-4

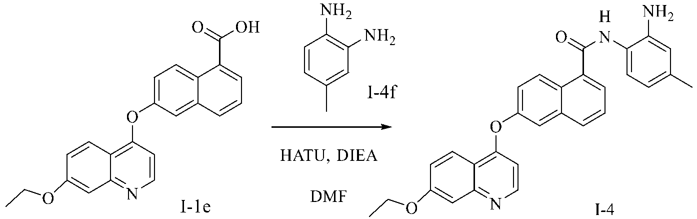

I-4 (yellow solid) was prepared from intermediate I-1e (200.00 mg, 0.56 mmol, 1.0 eq), HATU (319.28 mg, 0.84 mmol, 1.5 eq), DIEA (217.06 mg, 1.68 mmol, 3.0 eq), and I-4f (68.43 mg, 0.56 mmol, 1.0 eq) by steps similar to those in Example I-1. (120.0 mg, yield: 46.5%). LC-MS MS-ESI (m/z) 464.2 [M+H]⁺. ¹H-NMR (400 MHz, DMSO-d₆) δ ppm 9.80 (s, 1H), 8.63 (d, *J =* 4.8 Hz, 1H), 8.42 (d, *J =* 9.0 Hz, 1H), 8.24 (d, *J =* 9.1 Hz, 1H), 8.07 (d, *J =* 8.0 Hz, 1H), 7.96-7.81 (m, 2H), 7.65 (t, *J =* 7.4 Hz, 1H), 7.57 (d, *J =* 8.5 Hz, 1H), 7.42 (s, 1H), 7.30 (d, *J =* 8.4 Hz, 1H), 7.23 (d, *J =* 7.9 Hz, 1H), 6.63 (s, 1H), 6.58 (d, *J =* 4.7 Hz, 1H), 6.46 (d, *J =* 7.5 Hz, 1H), 4.92 (s, 2H), 4.23 (d, *J =* 6.8 Hz, 2H), 2.21 (s, 3H), 1.43 (t, *J =* 6.7 Hz, 3H).

### Example 5: Preparation of Compound I-5

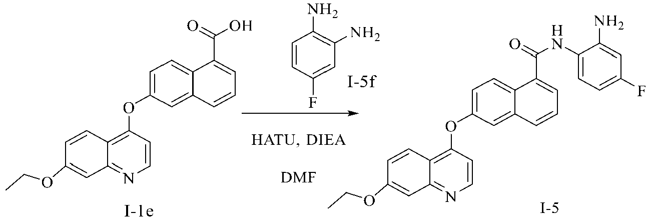

I-5 (yellow solid) was prepared from intermediate I-1e (200.00 mg, 0.56 mmol, 1.0 eq), HATU (319.28 mg, 0.84 mmol, 1.5 eq), DIEA (217.06 mg, 1.68 mmol, 3.0 eq), and I-5f (141.12 mg, 1.12 mmol, 2.0 eq) by steps similar to those in Example I-1. (55.0 mg, yield: 21.0%). LC-MS MS-ESI (m/z) 468.2 [M+H]⁺. ¹H-NMR (400 MHz, DMSO-d₆) δ ppm 9.81 (s, 1H), 8.64 (d, *J =* 5.2 Hz, 1H), 8.44 (d, *J =* 9.2 Hz, 1H), 8.24 (d, *J =* 9.1 Hz, 1H), 8.08 (d, *J =* 8.3 Hz, 1H), 7.94-7.88 (m, 2H), 7.70-7.62 (m, 1H), 7.57 (dd, *J =* 9.2, 2.5 Hz, 1H), 7.43 (d, *J =* 2.4 Hz, 1H), 7.38-7.26 (m, 2H), 6.64-6.54 (m, 2H), 6.42 (td, *J =* 8.5, 2.8 Hz, 1H), 5.33 (s, 2H), 4.24 (q, *J =* 6.9 Hz, 2H), 1.44 (t, *J =* 7.0 Hz, 3H).

### Example 6: Preparation of Compound I-6

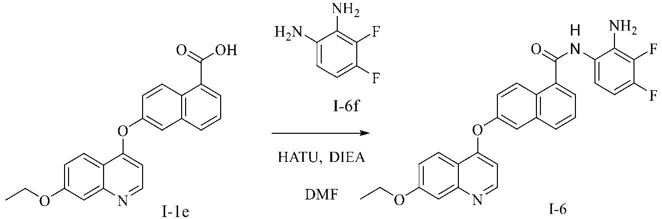

Intermediate I-1e (1.08 g, 3.00 mmol, 1.0 eq) was dissolved in super-dry DMF (15 mL), and HATU (1.71 g, 4.50 mmol, 1.5 eq), DIEA (1.16 g, 9.00 mmol, 3.0 eq), and I-6f (432.00 mg, 3.00 mmol, 1.0 eq) were added. The mixture was stirred at room temperature for 18 h. The reaction was quenched with water (150 mL), and the precipitated solid was collected by filtration, rinsed 3 times with water (5 mL), and dried in a blast drying oven at 50 °C for 8 h. The crude product was separated through a silica gel column (200- to 300-mesh silica gel, elution with EA/PE = 0-1) and then slurried with EA/PE = 1:1 (30 mL) to give I-6 as a yellow solid (682.00 mg, yield: 46.8%). LC-MS MS-ESI (m/z) 486.2 [M+H]⁺. ¹H-NMR (400 MHz, DMSO-d₆) δ ppm 9.93 (s, 1H), 8.62 (d, *J* = 5.1 Hz, 1H), 8.41 (d, *J* = 9.2 Hz, 1H), 8.22 (d, *J =* 9.1 Hz, 1H), 8.07 (d, *J =* 8.1 Hz, 1H), 7.93 (d, *J =* 6.9 Hz, 1H), 7.88 (s, 1H), 7.65 (t, *J =* 7.6 Hz, 1H), 7.55 (d, *J =* 8.9 Hz, 1H), 7.40 (s, 1H), 7.29 (d, *J* = 9.1 Hz, 1H), 7.20 (s, 1H), 6.70-6.52 (m, 2H), 5.35 (s, 2H), 4.21 (q, *J* = 6.8 Hz, 2H), 1.41 (t, *J =* 6.9 Hz, 3H).

### Preparation of intermediate: I-7c

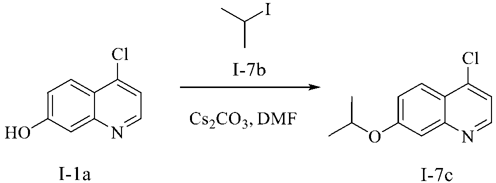

Intermediate I-7c (yellow solid) was prepared from commercially available I-1a (230.00 mg, 1.28 mmol, 1.0 eq), Cs₂CO₃ (1.25 g, 3.84 mmol, 3.0 eq), and I-7b (652.80 mg, 3.84 mmol, 3.0 eq) by steps similar to those in Example Intermediate I-1c. (263.00 mg, yield: 92.7%). LC-MS MS-ESI (m/z) 222.1 [M+H]⁺.

### Preparation of intermediate: I-7e

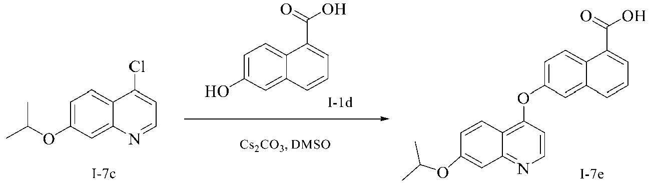

Intermediate I-7e (brown solid) was prepared from intermediate I-7c (prepared in-house, 263.00 mg, 1.19 mmol, 1.0 eq), Cs₂CO₃ (1.28 g, 3.93 mmol, 3.3 eq), and commercially available I-1d (223.96 mg, 1.19 mmol, 1.0 eq) by steps similar to those in Example Intermediate I-1e. (392.00 mg, yield: 88.2%). LC-MS MS-ESI (m/z) 374.2 [M+H]⁺.

### Example 7: Preparation of Compound I-7

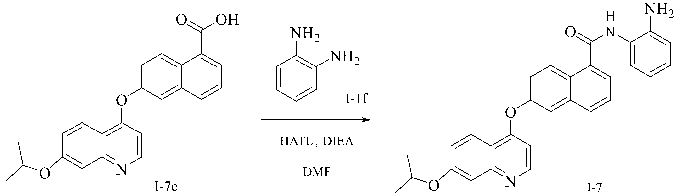

I-7 (yellow solid) was prepared from intermediate I-7e (392.00 mg, 1.05 mmol, 1.0 eq), HATU (596.76 mg, 1.57 mmol, 1.5 eq), DIEA (406.98 mg, 3.15 mmol, 3.0 eq), and I-1f (113.55 mg, 1.05 mmol, 1.0 eq) by steps similar to those in Example I-1. (174.0 mg, yield: 35.7%). LC-MS MS-ESI (m/z) 464.2 [M+H]⁺. ¹H-NMR (400 MHz, DMSO-d₆) δ ppm 9.89 (s, 1H), 8.63 (d, *J =* 5.0 Hz, 1H), 8.44 (d, *J =* 9.2 Hz, 1H), 8.23 (d, *J =* 9.1 Hz, 1H), 8.08 (d, *J =* 8.2 Hz, 1H), 7.89 (s, 2H), 7.66 (t, *J =* 7.6 Hz, 1H), 7.57 (d, *J =* 9.0 Hz, 1H), 7.43 (s, 1H), 7.38 (d, *J =* 7.6 Hz, 1H), 7.28 (d, *J =* 9.0 Hz, 1H), 7.00 (t, *J =* 7.3 Hz, 1H), 6.83 (d, *J =* 7.8 Hz, 1H), 6.65 (t, *J =* 7.3 Hz, 1H), 6.58 (d, *J =* 5.0 Hz, 1H), 5.01 (s, 2H), 4.87 (dt, *J =* 11.7, 5.8 Hz, 1H), 1.37 (d, *J =* 5.9 Hz, 6H).

### Preparation of intermediate: I-8c

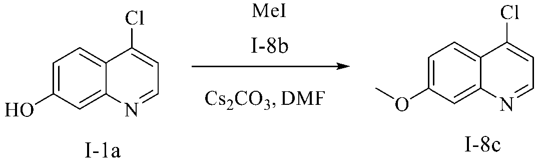

Intermediate I-8c (yellow solid) was prepared from commercially available I-1a (1.80 g, 10.02 mmol, 1.0 eq), Cs₂CO₃ (9.79 g, 30.06 mmol, 3.0 eq), and I-8b (4.27 g, 30.06 mmol, 3.0 eq) by steps similar to those in Example Intermediate I-1c. (1.77 g, yield: 91.2%). LC-MS MS-ESI (m/z)194.0 [M+H]⁺.

### Preparation of intermediate: I-8e

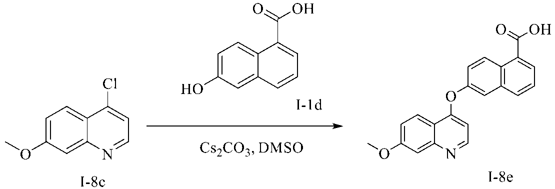

Intermediate I-8e (brown solid) was prepared from I-8c (prepared in-house, 1.00 g, 5.16 mmol, 1.0 eq), Cs₂CO₃ (5.55 g, 17.03 mmol, 3.3 eq), and commercially available I-1d (971.11 mg, 5.16 mmol, 1.0 eq) by steps similar to those in Example Intermediate I-1e. (1.41 g, yield: 79.2%). LC-MS MS-ESI (m/z) 346.1 [M+H]⁺.

### Example 8: Preparation of Compound I-8

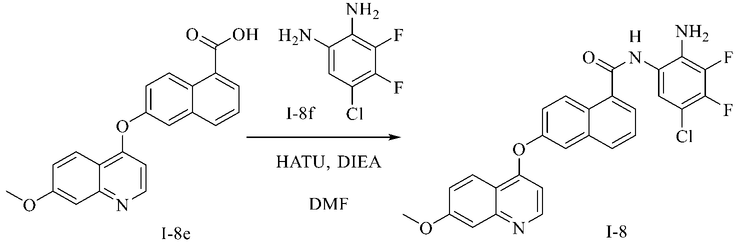

I-8 (yellow solid) was prepared from intermediate I-8e (104.0 mg, 0.30 mmol, 1.0 eq), HATU (171.10 mg, 0.45 mmol, 1.5 eq), DIEA (116.32 mg, 0.90 mmol, 3.0 eq), and I-8f (53.57 mg, 0.3 mmol, 1.0 eq) by steps similar to those in Example I-1. (42.00 mg, yield: 27.67%). LC-MS MS-ESI (m/z) 506.1 [M+H]⁺. ¹H-NMR (400 MHz, DMSO-d₆) δ ppm 9.98 (s, 1H), 8.63 (d, *J =* 5.2 Hz, 1H), 8.41 (d, *J =* 9.2 Hz, 1H), 8.23 (d, *J =* 9.1 Hz, 1H), 8.08 (d, *J* = 8.3 Hz, 1H), 7.94 (d, *J =* 6.9 Hz, 1H), 7.88 (d, *J =* 2.4 Hz, 1H), 7.69-7.61 (m, 1H), 7.58-7.49 (m, 2H), 7.43 (d, *J =* 2.5 Hz, 1H), 7.30 (dd, *J* = 9.1, 2.5 Hz, 1H), 6.57 (d, *J* = 5.2 Hz, 1H), 5.57 (s, 2H), 3.93 (s, 3H).

### Preparation of intermediate: I-9f-2

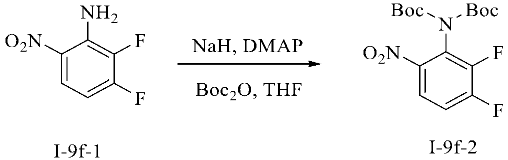

I-9f-1 (8.0 g, 0.46 mol, 1.0 eq) and 4-dimethylaminopyridine (DMAP, 0.281 g, 2.29 mmol, 0.05 eq) were dissolved in THF (500 mL). The solution was cooled to 0 °C in an ice bath, and NaH (60% in mineral oil, 3.68 g, 0.092 mol, 2.0 eq) was added. After the mixture was warmed to room temperature, di-tert-butyl dicarbonate (Boc₂O, 25.07 g, 0.115 mol, 2.5 eq) was added dropwise, and the mixture was stirred at room temperature for 18 h. The reaction mixture was quenched with ice/water (300 mL) and extracted twice with ethyl acetate (300.0 mL). The organic phases were combined, washed once with saturated brine (300 mL), and concentrated. The crude product was separated through a silica gel column (200- to 300-mesh silica gel, elution with EA/PE = 1/15) to give product I-9f-2 as a light yellow solid (15.2 g, yield: 88.4%). ¹H-NMR (400MHz, CDCl₃) δ ppm 7.97-7.93 (m, 1H), 7.35-7.29 (m, 1H), 1.43 (s, 18H).

### Preparation of intermediate: I-9f-3

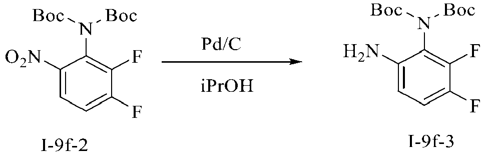

I-9f-2 (15.20 g, 40.63 mmol, 1.0 eq) was dissolved in isopropanol (iPrOH, 170 mL), and Pd/C (2.0 g) was added. The resulting mixture was stirred at room temperature for 20 h in a hydrogen atmosphere. The insoluble substance was removed by filtration through diatomaceous earth and rinsed 3 times with isopropanol (10 mL). The filtrate was concentrated to give product I-9f-3 as a yellow solid (13.44 g, yield: 96.1%). LC-MS MS-ESI (m/z) 345.1 [M+H]⁺.

### Preparation of intermediate: I-9f

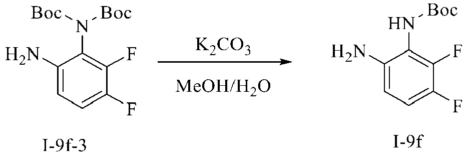

I-9f-3 (13.44 g, 39.02 mmol, 1.0 eq) was dissolved in methanol (MeOH, 150 mL), and water (10 mL) and K₂CO₃ (15.00 g, 107.91 mmol, 2.77 eq) were added. The mixture was heated to 60 °C, stirred for 24 h, and cooled to room temperature. The reaction mixture was concentrated, and the residue was diluted with water (200 mL) and extracted 3 times with ethyl acetate (200 mL). The organic phases were combined, washed once with saturated brine (300 mL), and concentrated. The crude product was separated through a silica gel column (200- to 300-mesh silica gel, elution with EA/PE = 1/2) to give product I-9f as a light yellow solid (9.41 g, yield: 98.7%). LC-MS MS-ESI (m/z) 189.1 [M-55]⁺. ¹H-NMR (400MHz, CDCl₃) δ ppm 8.28 (brs, 1H), 7.02-6.95 (m, 1H), 6.46-6.43 (m, 1H), 4.93 (s, 2H), 1.43 (s, 9H).

### Preparation of intermediate: I-9g

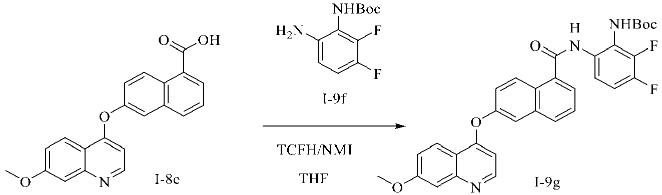

Intermediate I-8e (200.00 mg, 0.58 mmol, 1.0 eq) was dissolved in super-dry THF (10 mL), and commercially available N-methylimidazole (NMI, 214.28 mg, 2.61 mmol, 4.5 eq), I-9f (212.45 mg, 0.87 mmol, 1.5 eq), and N,N,N',N'-tetramethylchloroformamidinium hexafluorophosphate (TCFH, 243.60 mg, 0.87 mmol, 1.5 eq) were added. The mixture was stirred at room temperature for 24 h. The reaction mixture was concentrated, and the crude product was separated through a silica gel column (200- to 300-mesh silica gel, elution with EA/PE = 0-1) to give intermediate I-9g as a yellow solid (157.0 mg, yield: 47.3%). LC-MS MS-ESI (m/z) 572.2 [M+H]⁺.

### Example 9: Preparation of Compound I-9

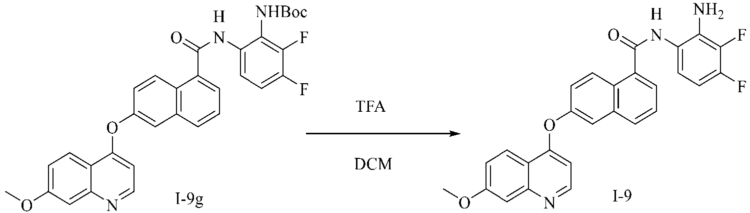

Intermediate I-9g (157.00 mg, 0.27 mmol, 1.0 eq) was dissolved in dichloromethane (DCM, 5 mL), and trifluoroacetic acid (TFA, 1.5 mL) was added. The mixture was stirred at room temperature for 18 h. The reaction was quenched with saturated sodium bicarbonate solution (100 mL) and extracted twice with DCM. The organic phases were combined and concentrated. The crude product was separated through a silica gel column (200- to 300-mesh silica gel, elution with EA/PE = 0-1) to give I-9 as a yellow solid (97.00 mg, yield: 76.2%). LC-MS MS-ESI (m/z) 472.2 [M+H]⁺. ¹H-NMR (400 MHz, DMSO-d₆) δ ppm 9.94 (s, 1H), 8.63 (d, *J =* 5.2 Hz, 1H), 8.41 (d, *J =* 9.2 Hz, 1H), 8.23 (d, *J* = 9.1 Hz, 1H), 8.07 (d, *J* = 8.3 Hz, 1H), 7.93 (d, *J* = 6.9 Hz, 1H), 7.88 (d, *J* = 2.3 Hz, 1H), 7.72-7.60 (m, 1H), 7.55 (dd, *J =* 9.2, 2.4 Hz, 1H), 7.43 (d, *J =* 2.4 Hz, 1H), 7.30 (dd, *J =* 9.1, 2.5 Hz, 1H), 7.26-7.15 (m, 1H), 6.74-6.51 (m, 2H), 5.36 (s, 2H), 3.94 (s, 3H).

### Example 10: Preparation of Compound I-10

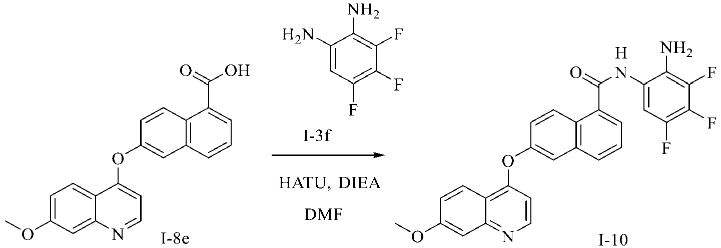

I-10 (yellow solid) was prepared from intermediate I-8e (138.14 mg, 0.40 mmol, 1.0 eq), HATU (228.14 mg, 0.6 mmol, 1.5 eq), DIEA (155.0 mg, 1.2 mmol, 3.0 eq), and I-3f (97.27 mg, 0.60 mmol, 1.5 eq) by steps similar to those in Example I-1. (45.60 mg, yield: 23.29%). LC-MS MS-ESI (m/z) 490.1 [M+H]⁺. ¹H-NMR (400 MHz, DMSO-d₆) δ ppm 10.01 (s, 1H), 8.63 (d, *J* = 5.1 Hz, 1H), 8.41 (d, *J =* 9.2 Hz, 1H), 8.23 (d, *J =* 9.1 Hz, 1H), 8.08 (d, *J =* 8.1 Hz, 1H), 7.97-7.85 (m, 2H), 7.65 (t, *J =* 7.5 Hz, 1H), 7.59-7.46 (m, 2H), 7.43 (d, *J* = 2.2 Hz, 1H), 7.30 (dd, *J =* 9.1, 2.2 Hz, 1H), 6.58 (d, *J =* 5.1 Hz, 1H), 5.24 (s, 2H), 3.94 (s, 3H).

### Preparation of intermediate: I-11c

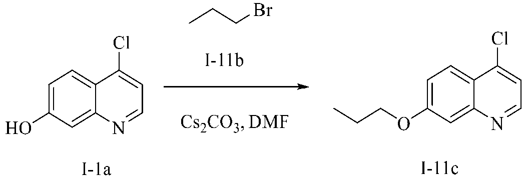

Intermediate I-11c (yellow solid) was prepared from commercially available I-1a (1.80 g, 10.00 mmol, 1.0 eq), Cs₂CO₃ (4.15 g, 30.00 mmol, 3.0 eq), and I-11b (3.66 g, 30.00 mmol, 3.0 eq) by steps similar to those in Example Intermediate I-1c. (1.65 g, yield: 74.3%). LC-MS MS-ESI (m/z) 222.0 [M+H]⁺.

### Preparation of intermediate: I-11e

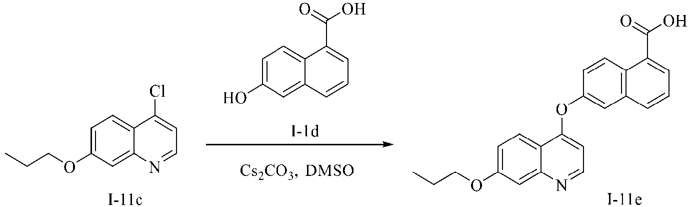

Intermediate I-11e (brown solid) was prepared from intermediate I-11c (prepared in-house, 1.65 g, 7.44 mmol, 1.0 eq), Cs₂CO₃ (8.00 g, 24.55 mmol, 3.3 eq), and commercially available I-1d (1.40 g, 7.44 mmol, 1.0 eq) by steps similar to those in Example Intermediate I-1e. (2.30 g, yield: 82.8%). LC-MS MS-ESI (m/z) 374.1 [M+H]⁺.

### Example 11: Preparation of Compound I-11

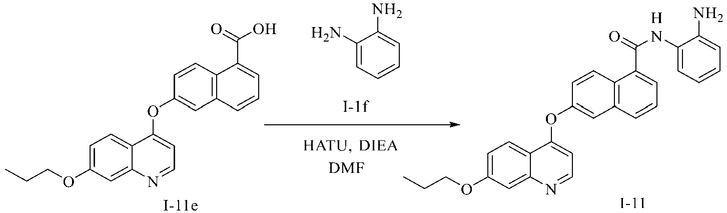

I-11 (yellow solid) was prepared from intermediate I-11e (1.30 g, 3.48 mmol, 1.0 eq), HATU (1.98 g, 5.22 mmol, 1.5 eq), DIEA (1.35 g, 10.44 mmol, 3.0 eq), and I-1f (376.19 mg, 3.48 mmol, 1.0 eq) by steps similar to those in Example I-1. (590.00 mg, yield: 36.6%). LC-MS MS-ESI (m/z) 464.2 [M+H]⁺. ¹H-NMR (400 MHz, DMSO-d₆) δ ppm 9.88 (s, 1H), 8.64 (d, *J = 5.2* Hz, 1H), 8.43 (d, *J =* 9.2 Hz, 1H), 8.24 (d, *J =* 9.1 Hz, 1H), 8.08 (d, *J* = 8.2 Hz, 1H), 7.91-7.86 (m, 2H), 7.66 (t, *J* = 7.7 Hz, 1H), 7.57 (dd, *J =* 9.2, 2.4 Hz, 1H), 7.43 (d, *J =* 2.3 Hz, 1H), 7.37 (d, *J =* 7.7 Hz, 1H), 7.31 (dd, *J =* 9.1, 2.4 Hz, 1H), 7.00 (t, *J* = 7.6 Hz, 1H), 6.82 (d, *J =* 7.9 Hz, 1H), 6.64 (t, *J =* 7.5 Hz, 1H), 6.59 (d, *J* = 5.2 Hz, 1H), 5.00 (s, 2H), 4.13 (t, *J =* 6.5 Hz, 2H), 1.90-1.77 (m, 2H), 1.05 (t, *J* = 7.4 Hz, 3H).

### Example 12: Preparation of Compound I-12

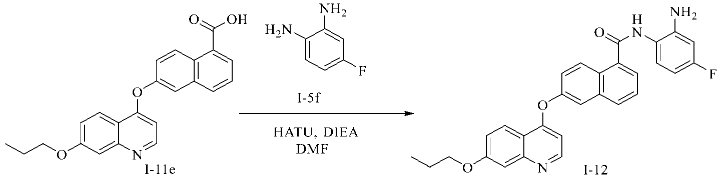

I-12 (yellow solid) was prepared from intermediate I-11e (200.00 mg, 0.54 mmol, 1.0 eq), HATU (307.96 mg, 0.81 mmol, 1.5 eq), DIEA (209.14 mg, 1.62 mmol, 3.0 eq), and I-5f (136.19 mg, 1.08 mmol, 2.0 eq) by steps similar to those in Example I-1. (100.00 mg, yield: 38.5%). LC-MS MS-ESI (m/z) 482.2 [M+H]⁺. ¹H-NMR (400 MHz, DMSO-d₆) δ ppm 9.80 (s, 1H), 8.64 (d, *J* = 5.2 Hz, 1H), 8.44 (d, *J =* 9.2 Hz, 1H), 8.24 (d, *J =* 9.1 Hz, 1H), 8.07 (d, *J =* 8.3 Hz, 1H), 7.93-7.88 (m, 2H), 7.71-7.60 (m, 1H), 7.57 (dd, *J =* 9.2, 2.5 Hz, 1H), 7.43 (d, *J =* 2.4 Hz, 1H), 7.37-7.26 (m, 2H), 6.62-6.56 (m, 2H), 6.42 (td, *J =* 8.5, 2.8 Hz, 1H), 5.32 (s, 2H), 4.13 (t, *J =* 6.5 Hz, 2H), 1.90-1.76 (m, 2H), 1.04 (t, *J =* 7.4 Hz, 3H).

### Preparation of intermediate: I-13c

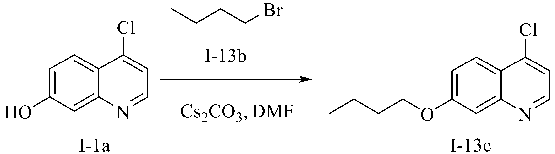

Intermediate I-13c (yellow solid) was prepared from commercially available I-1a (1.80 g, 10.00 mmol, 1.0 eq), Cs₂CO₃ (4.15 g, 30.00 mmol, 3.0 eq), and I-13b (4.11 g, 30.00 mmol, 3.0 eq) by steps similar to those in Example Intermediate I-1c. (1.72 g, yield: 73.0%). LC-MS MS-ESI (m/z) 236.1 [M+H]⁺.

### Preparation of intermediate: I-13e

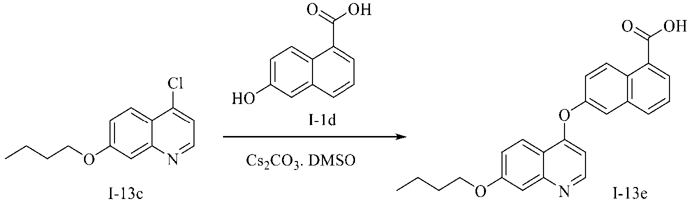

Intermediate I-13e (brown solid) was prepared from intermediate I-13c (prepared in-house, 705.00 mg, 3.00 mmol, 1.0 eq), Cs₂CO₃ (3.22 g, 9.90 mmol, 3.3 eq), and commercially available I-1d (564.60 mg, 3.00 mmol, 1.0 eq) by steps similar to those in Example Intermediate I-1e. (850.00 mg, yield: 73.1%). LC-MS MS-ESI (m/z) 388.2 [M+H]⁺.

### Example 13: Preparation of Compound I-13

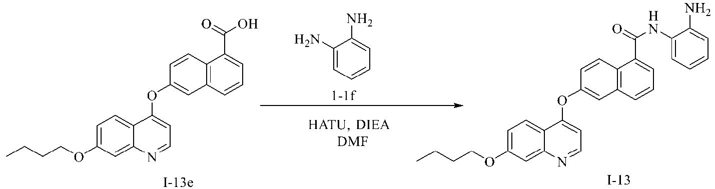

I-13 (yellow solid) was prepared from intermediate I-13e (108.50 mg, 0.28 mmol, 1.0 eq), HATU (159.68 mg, 0.42 mmol, 1.5 eq), DIEA (108.53 mg, 0.84 mmol, 3.0 eq), and I-1f (30.27 mg, 0.28 mmol, 1.0 eq) by steps similar to those in Example I-1. (37.00 mg, yield: 27.7%). LC-MS MS-ESI (m/z) 478.3 [M+H]⁺. ¹H-NMR (400 MHz, DMSO-d₆) δ ppm 9.88 (s, 1H), 8.63 (d, *J =* 5.2 Hz, 1H), 8.43 (d, *J =* 9.2 Hz, 1H), 8.23 (d, *J =* 9.1 Hz, 1H), 8.08 (d, *J* = 8.3 Hz, 1H), 7.91-7.86 (m, 2H), 7.70-7.63 (m, 1H), 7.57 (dd, *J =* 9.2, 2.4 Hz, 1H), 7.43 (d, *J* = 2.4 Hz, 1H), 7.37 (d, *J =* 7.4 Hz, 1H), 7.30 (dd, *J =* 9.1, 2.4 Hz, 1H), 7.00 (t, *J* = 7.1 Hz, 1H), 6.82 (d, *J =* 7.5 Hz, 1H), 6.64 (t, *J =* 7.2 Hz, 1H), 6.59 (d, *J* = 5.2 Hz, 1H), 5.00 (s, 2H), 4.17 (t, *J =* 6.4 Hz, 2H), 1.88-1.73 (m, 2H), 1.51 (dq, *J* = 14.7, 7.4 Hz, 2H), 0.97 (t, *J =* 7.4 Hz, 3H).

### Example 14: Preparation of Compound I-14

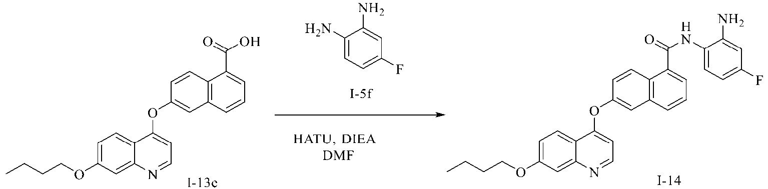

I-14 (yellow solid) was prepared from intermediate I-13e (1.40 g, 3.61 mmol, 1.0 eq), HATU (2.06 g, 5.41 mmol, 1.5 eq), DIEA (1.40 g, 10.83 mmol, 3.0 eq), and I-5f (455.22 mg, 3.61 mmol, 1.0 eq) by steps similar to those in Example I-1. (1015.00 mg, yield: 56.7%). LC-MS MS-ESI (m/z) 496.3 [M+H]⁺. ¹H-NMR (400 MHz, DMSO-d₆) δ ppm 9.80 (s, 1H), 8.63 (d, *J = 5.2* Hz, 1H), 8.43 (d, *J =* 9.2 Hz, 1H), 8.23 (d, *J =* 9.1 Hz, 1H), 8.07 (d, *J =* 8.3 Hz, 1H), 7.92-7.87 (m, 2H), 7.69-7.62 (m, 1H), 7.56 (dd, *J =* 9.2, 2.5 Hz, 1H), 7.43 (d, *J =* 2.4 Hz, 1H), 7.37-7.25 (m, 2H), 6.67-6.51 (m, 2H), 6.41 (td, *J =* 8.5, 2.9 Hz, 1H), 5.32 (s, 2H), 4.17 (t, *J =* 6.5 Hz, 2H), 1.87-1.71 (m, 2H), 1.61-1.44 (m, 2H), 0.98 (t, *J =* 7.4 Hz, 3H).

### Preparation of intermediate: I-15g

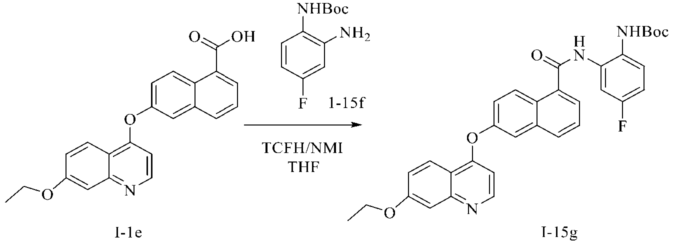

Intermediate I-15g (yellow solid) was prepared from intermediate I-1e (200.00 mg, 0.56 mmol, 1.0 eq), *N-methylimidazole* (206.89 mg, 2.52 mmol, 4.5 eq), I-15f (190.05 mg, 0.84 mmol, 1.5 eq), TCFH (235.70 mg, 0.84 mmol, 1.5 eq), and THF (10.0 mL) by steps similar to those in Example I-9g. (100.0 mg, yield: 31.5%). LC-MS MS-ESI (m/z) 568.2 [M+H]⁺.

### Example 15: Preparation of Compound I-15

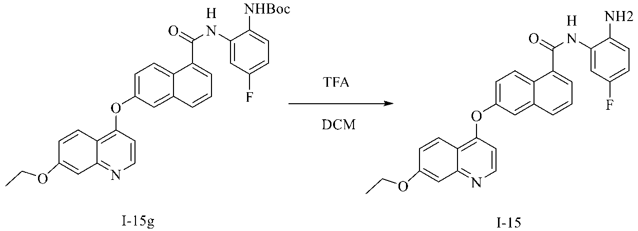

I-15 (yellow solid) was prepared from intermediate I-15g (100.0 mg, 0.18 mmol, 1.0 eq), TFA (1.5 mL), and DCM (5.0 mL) by steps similar to those in Example I-9. (60.00 mg, yield: 71.3%). LC-MS MS-ESI (m/z) 468.2 [M+H]⁺. ¹H-NMR (400 MHz, DMSO-d₆) δ ppm 9.90 (s, 1H), 8.61 (d, *J =* 5.2 Hz, 1H), 8.41 (d, *J =* 9.2 Hz, 1H), 8.21 (d, *J =* 9.1 Hz, 1H), 8.07 (d, *J =* 8.2 Hz, 1H), 7.92-7.82 (m, 2H), 7.64 (t, *J =* 7.7 Hz, 1H), 7.55 (dd, *J =* 9.2, 2.4 Hz, 1H), 7.42-7.35 (m, 2H), 7.28 (dd, *J =* 9.1, 2.4 Hz, 1H), 6.88-6.76 (m, 2H), 6.56 (d, *J = 5.2* Hz, 1H), 4.91 (s, 2H), 4.20 (q, *J =* 6.9 Hz, 2H), 1.41 (t, *J =* 6.9 Hz, 3H).

### Preparation of intermediate: I-16g

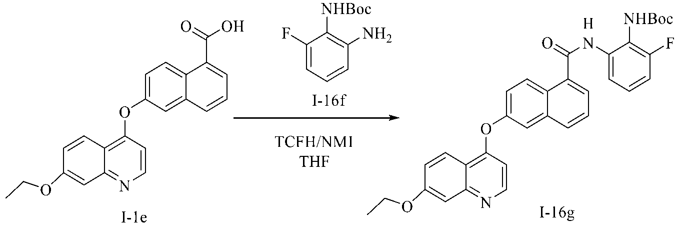

Intermediate I-16g (yellow solid) was prepared from intermediate I-1e (200.00 mg, 0.56 mmol, 1.0 eq), TCFH (235.70 mg, 0.84 mmol, 1.5 eq), NMI (206.89 mg, 2.52 mmol, 4.5 eq), and I-16f (190.05 mg, 0.84 mmol, 1.5 eq) by steps similar to those in Example Intermediate I-9g. (100.00 mg, yield: 31.5%). LC-MS MS-ESI (m/z) 568.2 [M+H]⁺.

### Example 16: Preparation of Compound I-16

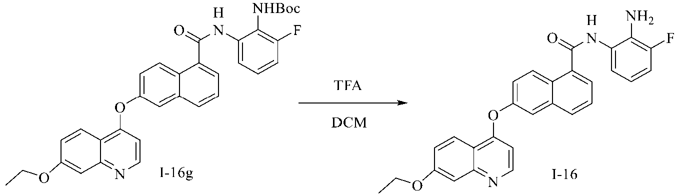

I-16 (yellow solid) was prepared from intermediate I-16g (100.00 mg, 0.18 mmol, 1.0 eq) and TFA (1.5 mL) by steps similar to those in Example I-9. (60.00 mg, yield: 71.3%). LC-MS MS-ESI (m/z) 468.2 [M+H]⁺. ¹H-NMR (400 MHz, DMSO-d₆) δ ppm 10.04 (s, 1H), 8.64 (d, *J =* 5.2 Hz, 1H), 8.42 (d, *J* = 9.2 Hz, 1H), 8.24 (d, *J* = 9.1 Hz, 1H), 8.09 (d, *J* = 8.3 Hz, 1H), 7.94-7.88 (m, 2H), 7.67 (t, *J* = 7.7 Hz, 1H), 7.58 (dd, *J* = 9.2, 2.4 Hz, 1H), 7.43 (d, *J =* 2.4 Hz, 1H), 7.34-7.26 (m, 2H), 7.07-6.91 (m, 1H), 6.65 (dd, *J =* 14.0, 8.0 Hz, 1H), 6.59 (d, *J =* 5.2 Hz, 1H), 4.99 (s, 2H), 4.23 (q, *J* = 6.9 Hz, 2H), 1.43 (t, *J =* 6.9 Hz, 3H).

### Preparation of intermediate: I-17a-3

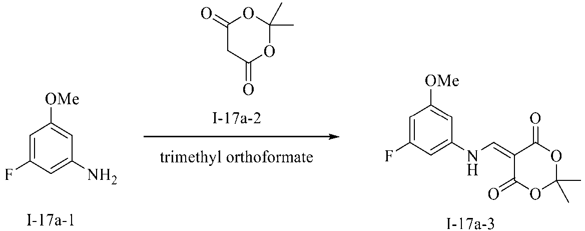

Commercially available I-17a-2 (51.10 g, 354.60 mmol, 1.0 eq) was dissolved in trimethyl orthoformate (300.0 mL), and the solution was purged 3 times with nitrogen and heated to 105 °C. After 1 h of stirring, I-17a-1 (50.00 g, 354.60 mmol) was added, and the mixture was stirred at that temperature for another hour and cooled to room temperature. n-Pentane (300 mL) was added, and the precipitated solid was collected by filtration, rinsed 3 times with n-pentane (30 mL), and dried in a blast drying oven at 50 °C for 8 h to give intermediate I-17a-3 as a yellow solid. (86.00 g, yield: 82.2%). LC-MS MS-ESI (m/z) 296.1 [M+H]⁺.

### Preparation of intermediate: I-17a-4

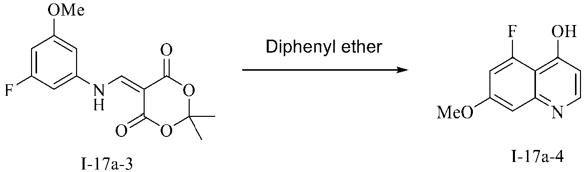

A mixture of I-17a-3 (60.00 g, 203.30 mmol, 1.0 eq) and diphenyl ether (500 mL) was heated to 220 °C, stirred for 1 h, and cooled to room temperature. Petroleum ether (500 mL) was added, and the precipitated solid was collected by filtration, rinsed 3 times with petroleum ether (50 mL), and dried. The crude product was separated through a reversed-phase C18 column (acetonitrile/water/0.1% TFA, 12-13%) to give 1-17a-4 as a white solid (18.00 g, yield: 46.0%). LC-MS MS-ESI (m/z) 194.0 [M+H]⁺.

### Preparation of intermediate: I-17a-5

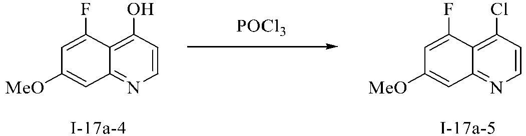

A mixture of intermediate I-17a-4 (18.00 g, 93.30 mmol, 1.0 eq) and phosphorus oxychloride (50 mL) was heated to 110 'C, stirred for 3 h, and cooled to room temperature. The reaction mixture was slowly added to ice/water to quench the reaction, and the pH was adjusted to 6-7 with a 10% NaOH solution. The precipitated solid was collected by filtration, rinsed 3 times with water (10 mL), and dried. The crude product was separated through a silica gel column (200- to 300-mesh silica gel, elution with EA/PE = 1:1) to give intermediate I-17a-5 as a yellow solid (16.10 g, yield: 81.8%). LC-MS MS-ESI (m/z) 212.0 [M+H]⁺.

### Preparation of intermediate: I-17a

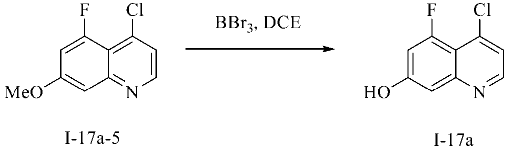

Intermediate I-17a-5 (12.00 g, 87.60 mmol, 1.0 eq) was dissolved in 1,2-dichloroethane (DCE, 100.0 mL). The mixture was cooled to 0 °C, and a 1.0 M solution of boron tribromide in dichloromethane (198.80 mL, 198.80 mmol, 2.27 eq) was added dropwise. After the addition, the mixture was heated to 100 °C, stirred for 8 h, and cooled to room temperature. Methanol (100 mL) was slowly added to quench the reaction. Water (300 mL) was added, and the pH was adjusted to 12-13 with a 10% NaOH solution. The organic phase and the aqueous phase were separated, and the pH of the aqueous phase was adjusted to 5-6 with 2 M dilute hydrochloric acid. The precipitated solid was collected by filtration, rinsed 3 times with water (10.0 mL), and dried in a blast drying oven at 50 °C for 8 h to give intermediate I-17a as a yellow solid (9.10 g, yield: 80.2%). LC-MS MS-ESI (m/z) 198.0 [M+H]⁺. ¹H-NMR (400 MHz, DMSO-d₆) δ ppm 10.82 (s, 1H), 8.67 (d, *J =* 4.8 Hz, 1H), 7.48 (d, *J =* 4.8 Hz, 1H), 7.19 (s, 1H), 7.08 (dd, *J =* 14.0, 2.3 Hz, 1H).

### Preparation of intermediate: I-17c

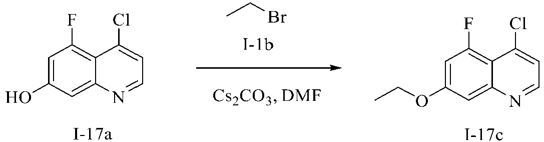

Intermediate I-17c (light yellow solid) was prepared from intermediate I-17a (800.00 mg, 4.05 mmol, 1.0 eq), Cs₂CO₃ (3.96 g, 12.15 mmol, 3.0 eq), and I-1b (1.32 g, 12.15 mmol, 3.0 eq) by steps similar to those in Example Intermediate I-1c. (810.00 mg, yield: 88.7%). LC-MS MS-ESI (m/z) 226.0 [M+H]⁺.

### Preparation of intermediate: I-17e

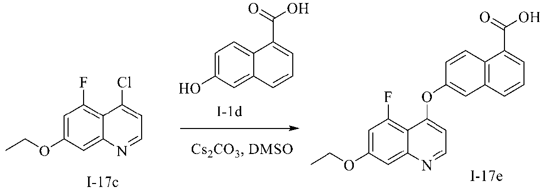

Intermediate I-17e (brown solid) was prepared from intermediate I-17c (450.00 mg, 2.00 mmol, 1.0 eq), Cs₂CO₃ (2.15 g, 6.60 mmol, 3.3 eq), and commercially available I-1d (376.40 mg, 2.00 mmol, 1.0 eq) by steps similar to those in Example Intermediate I-1e. (640.00 mg, yield: 84.8%). LC-MS MS-ESI (m/z) 378.2 [M+H]⁺.

### Example 17: Preparation of Compound I-17

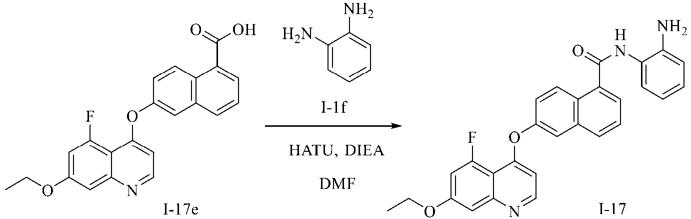

I-17 (yellow solid) was prepared from intermediate I-17e (340.00 mg, 0.90 mmol, 1.0 eq), HATU (513.57 mg, 1.35 mmol, 1.5 eq), DIEA (348.69 mg, 2.70 mmol, 3.0 eq), and I-1f (97.31 mg, 0.90 mmol, 1.0 eq) by steps similar to those in Example I-1. (99.00 mg, yield: 23.5%). LC-MS MS-ESI (m/z) 467.2 [M+H]⁺. ¹H-NMR (400 MHz, DMSO-d₆) δ ppm 9.86 (s, 1H), 8.63 (d, *J =* 5.0 Hz, 1H), 8.41 (d, *J =* 9.1 Hz, 1H), 8.04 (d, *J =* 8.1 Hz, 1H), 7.85 (d, *J =* 6.7 Hz, 1H), 7.81 (s, 1H), 7.63 (t, *J =* 7.5 Hz, 1H), 7.54 (d, *J =* 8.6 Hz, 1H), 7.35 (d, *J =* 7.5 Hz, 1H), 7.29 (s, 1H), 7.12 (d, *J =* 13.2 Hz, 1H), 6.98 (t, *J =* 7.2 Hz, 1H), 6.80 (d, *J =* 7.7 Hz, 1H), 6.71-6.52 (m, 2H), 4.98 (s, 2H), 4.33-4.12 (m, 2H), 1.40 (t, *J = 6.7* Hz, 3H).

### Example 18: Preparation of Compound I-18

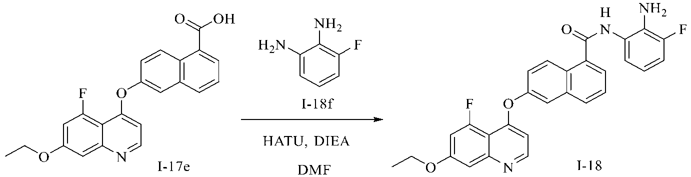

I-18 (yellow solid) was prepared from intermediate I-17e (340.00 mg, 0.90 mmol, 1.0 eq), HATU (513.57 mg, 1.35 mmol, 1.5 eq), DIEA (348.69 mg, 2.70 mmol, 3.0 eq), and I-18f (113.53 mg, 0.90 mmol, 1.0 eq) by steps similar to those in Example I-1. (282.00 mg, yield: 64.5%). LC-MS MS-ESI (m/z) 486.2 [M+H]⁺. ¹H-NMR (400 MHz, DMSO-d₆) δ ppm 10.03 (s, 1H), 8.65 (d, *J =* 5.0 Hz, 1H), 8.41 (d, *J =* 9.1 Hz, 1H), 8.08 (d, *J* = 8.0 Hz, 1H), 7.90 (d, *J =* 6.6 Hz, 1H), 7.83 (s, 1H), 7.66 (t, *J =* 7.4 Hz, 1H), 7.56 (d, *J* = 9.0 Hz, 1H), 7.38-7.23 (m, 2H), 7.14 (d, *J =* 13.3 Hz, 1H), 7.06-6.91 (m, 1H), 6.65 (d, *J* = 4.9 Hz, 2H), 4.99 (s, 2H), 4.24 (q, *J =* 6.9 Hz, 2H), 1.42 (t, *J =* 6.7 Hz, 3H).

### Example 19: Preparation of Compound I-19

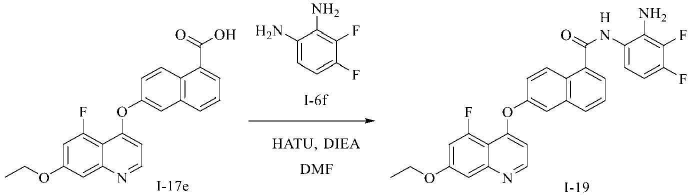

I-19 (yellow solid) was prepared from intermediate I-17e (340.00 mg, 0.90 mmol, 1.0 eq), HATU (513.57 mg, 1.35 mmol, 1.5 eq), DIEA (348.69 mg, 2.70 mmol, 3.0 eq), and I-6f (129.60 mg, 0.90 mmol, 1.0 eq) by steps similar to those in Example I-1. (164.00 mg, yield: 36.2%). LC-MS MS-ESI (m/z) 504.2 [M+H]⁺. ¹H-NMR (400 MHz, DMSO-d₆) δ ppm 9.95 (s, 1H), 8.66 (d, *J =* 5.2 Hz, 1H), 8.42 (d, *J =* 9.2 Hz, 1H), 8.07 (d, *J =* 8.2 Hz, 1H), 7.94 (d, *J =* 7.0 Hz, 1H), 7.83 (s, 1H), 7.66 (t, *J =* 7.6 Hz, 1H), 7.55 (dd, *J* = 9.2, 2.0 Hz, 1H), 7.31 (s, 1H), 7.28-7.18 (m, 1H), 7.14 (dd, *J =* 13.4, 1.9 Hz, 1H), 6.69-6.58 (m, 2H), 5.37 (s, 2H), 4.24 (q, *J =* 6.9 Hz, 2H), 1.42 (t, *J =* 6.9 Hz, 3H).

### Preparation of intermediate: I-20g

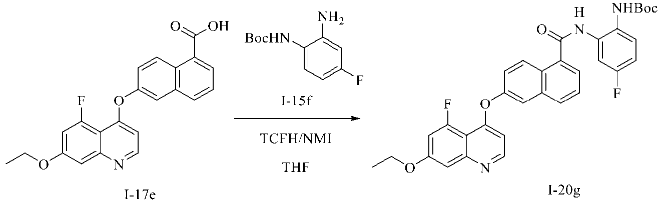

Intermediate I-20g (yellow solid) was prepared from intermediate I-17e (340.00 mg, 0.90 mmol, 1.0 eq), TCFH (378.00 mg, 1.35 mmol, 1.5 eq), NMI (332.50 mg, 4.05 mmol, 4.5 eq), and I-15f (203.58 mg, 0.90 mmol, 1.0 eq) by steps similar to those in Example I-9g. (152.00 mg, yield: 28.8%). LC-MS MS-ESI (m/z) 586.2 [M+H]⁺.

### Example 20: Preparation of Compound I-20

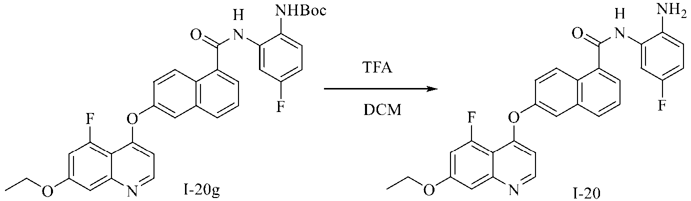

I-20 (yellow solid) was prepared from intermediate I-20g (152.0 mg, 0.26 mmol, 1.0 eq) and TFA (1.5 mL) by steps similar to those in Example I-9. (32.00 mg, yield: 25.3%). LC-MS MS-ESI (m/z) 486.2 [M+H]⁺. ¹H-NMR (400 MHz, DMSO-d₆) δ ppm 9.93 (s, 1H), 8.66 (d, *J = 5.2* Hz, 1H), 8.42 (d, *J =* 9.2 Hz, 1H), 8.07 (d, *J =* 8.2 Hz, 1H), 7.87 (d, *J* = 6.9 Hz, 1H), 7.83 (s, 1H), 7.66 (t, *J =* 7.7 Hz, 1H), 7.60-7.51 (m, 1H), 7.40 (d, *J =* 8.1 Hz, 1H), 7.31 (s, 1H), 7.20-7.08 (m, 1H), 6.92-6.75 (m, 2H), 6.66 (d, *J* = 5.2 Hz, 1H), 4.93 (s, 2H), 4.24 (q, *J =* 6.9 Hz, 2H), 1.42 (t, *J =* 6.9 Hz, 3H).

### Preparation of intermediate: I-21b

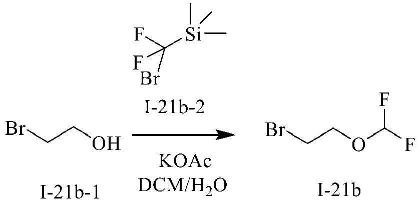

Commercially available I-21b-1 (3.0 g, 24.01 mmol, 1.0 eq) was dissolved in DCM/H₂O (30 mL/30 mL), and KOAc (14.50 g, 148.00 mmol, 6.16 eq) was added. After the mixture was cooled to 0 °C in an ice/salt bath, I-21b-2 (15 mL, 96.45 mmol, 4.0 eq) was added dropwise. Then the ice/salt bath was removed, and the mixture was stirred at room temperature for 18 h. The reaction mixture was quenched with saturated NaHCO₃ solution (100 mL) and extracted twice with DCM (50 mL). The organic phases were combined, dried over anhydrous Na₂SO₄, and concentrated under reduced pressure below 5 °C to give intermediate I-21b as a yellow oil (1.10 g, yield: 26.2%). LC-MS MS-ESI (m/z) 175.0 [M+H]⁺.

### Preparation of intermediate: I-21c

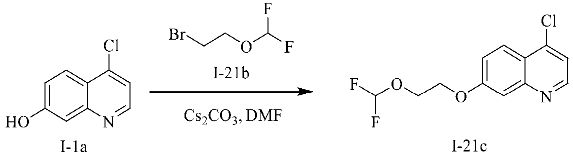

Intermediate I-21c (yellow solid) was prepared from commercially available I-1a (2.0 g, 11.13 mmol, 1.0 eq), Cs₂CO₃ (10.88 g, 33.39 mmol, 3.0 eq), and intermediate I-21b (prepared in-house, 2.92 g, 16.69 mmol, 1.5 eq) by steps similar to those in Example Intermediate I-1c. (2.46 g, yield: 80.7%). LC-MS MS-ESI (m/z) 274.0 [M+H]⁺.

### Preparation of intermediate: I-21e

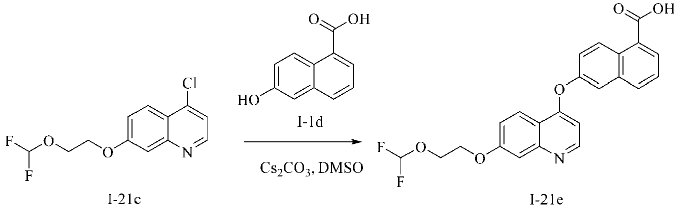

Intermediate I-21e (brown solid) was prepared from intermediate I-21c (2.46 g, 8.99 mmol, 1.0 eq), Cs₂CO₃ (9.67 g, 29.67 mmol, 3.3 eq), and commercially available I-1d (1.69 g, 8.99 mmol, 1.0 eq) by steps similar to those in Example Intermediate I-1e. (3.42 g, yield: 89.4%). LC-MS MS-ESI (m/z) 426.1 [M+H]⁺.

### Example 21: Preparation of Compound I-21

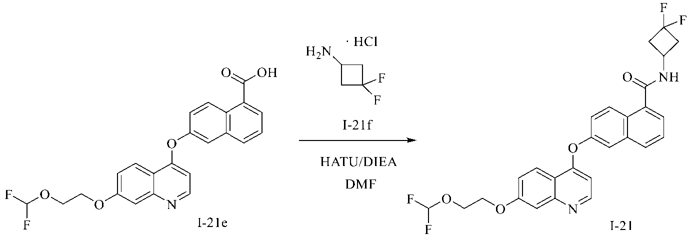

I-21 (yellow solid) was prepared from intermediate I-21e (1.27 g, 2.98 mmol, 1.0 eq), HATU (1.70 g, 4.47 mmol, 1.5 eq), DIEA (1.54 g, 11.92 mmol, 4.0 eq), and I-21f (427.93 mg, 2.98 mmol, 1.0 eq) by steps similar to those in Example I-1. (514.00 mg, yield: 33.4%). LC-MS MS-ESI (m/z) 515.2 [M+H]⁺. ¹H-NMR (400 MHz, DMSO-d₆) δ ppm 9.05 (d, *J =* 6.5 Hz, 1H), 8.62 (d, *J =* 5.2 Hz, 1H), 8.35 (d, *J* = 9.2 Hz, 1H), 8.23 (d, *J* = 9.1 Hz, 1H), 8.03 (d, *J =* 8.1 Hz, 1H), 7.85 (d, *J =* 2.4 Hz, 1H), 7.66 (d, *J =* 6.3 Hz, 1H), 7.63-7.56 (m, 1H), 7.53 (dd, *J =* 9.2, 2.5 Hz, 1H), 7.46 (d, *J =* 2.4 Hz, 1H), 7.32 (dd, *J =* 9.1, 2.4 Hz, 1H), 6.78 (t, ²*J* _{F-H}= 75.7 Hz, 1H), 6.57 (d, *J* = 5.2 Hz, 1H), 4.49-4.28 (m, 3H), 4.28-4.18 (m, 2H), 3.12-2.88 (m, 2H), 2.85-2.66 (m, 2H).

### Example 22: Preparation of Compound I-22

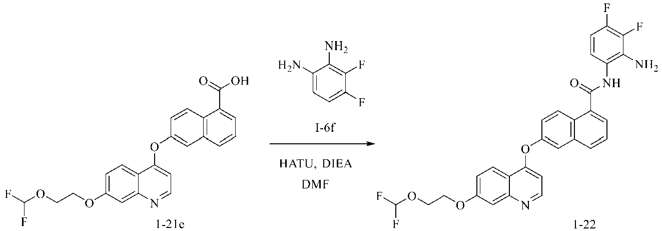

I-22 (yellow solid) was prepared from intermediate I-21e (1.27 g, 2.98 mmol, 1.0 eq), HATU (1.70 g, 4.47 mmol, 1.5 eq), DIEA (1.15 g, 8.94 mmol, 3.0 eq), and I-6f (429.42 mg, 2.98 mmol, 1.0 eq) by steps similar to those in Example I-1. (596.00 mg, yield: 36.3%). LC-MS MS-ESI (m/z) 552.2 [M+H]⁺. ¹H-NMR (400 MHz, DMSO-d₆) δ ppm 9.96 (s, 1H), 8.66 (d, *J = 5.2* Hz, 1H), 8.43 (d, *J* = 9.2 Hz, 1H), 8.27 (d, *J* = 9.2 Hz, 1H), 8.09 (d, *J* = 8.3 Hz, 1H), 7.96 (d, *J* = 7.0 Hz, 1H), 7.91 (d, *J* = 2.4 Hz, 1H), 7.75-7.63 (m, 1H), 7.58 (dd, *J* = 9.2, 2.5 Hz, 1H), 7.49 (d, *J* = 2.5 Hz, 1H), 7.35 (dd, *J* = 9.2, 2.5 Hz, 1H), 7.30-7.16 (m, 1H), 6.81 (t, ²*J* _{F-H} = 75.7 Hz, 1H), 6.70-6.55 (m, 2H), 5.38 (s, 2H), 4.40 (dd, *J =* 5.4, 2.9 Hz, 2H), 4.27 (dd, *J* = 5.2, 3.0 Hz, 2H).

### Preparation of intermediate: I-23c

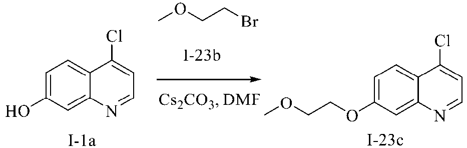

Intermediate I-23c (yellow solid) was prepared from commercially available I-1a (2.0 g, 11.13 mmol, 1.0 eq), Cs₂CO₃ (10.88 g, 33.39 mmol, 3.0 eq), and commercially available I-23b (4.64 g, 33.39 mmol, 3.0 eq) by steps similar to those in Example Intermediate I-1c. (2.10 g, yield: 79.4%). LC-MS MS-ESI (m/z) 238.1 [M+H]⁺.

### Preparation of intermediate: I-23e

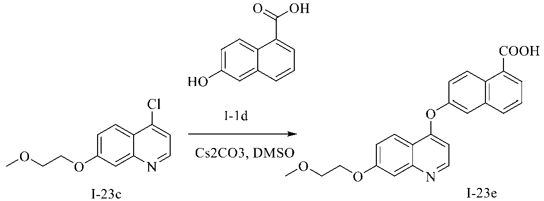

Intermediate I-23e (brown solid) was prepared from intermediate I-23c (2.10 g, 8.83 mmol, 1.0 eq), Cs₂CO₃ (9.50 g, 29.14 mmol, 3.3 eq), and commercially available I-1d (1.66 g, 8.83 mmol, 1.0 eq) by steps similar to those in Example Intermediate I-1e. (3.12 g, yield: 90.7%). LC-MS MS-ESI (m/z) 390.1 [M+H]⁺.

### Example 23: Preparation of Compound I-23

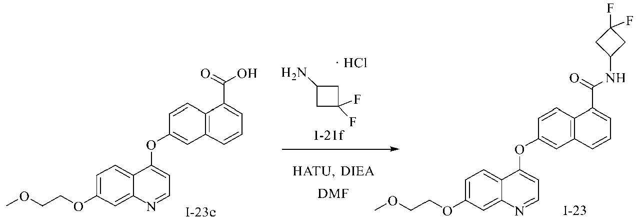

I-23 (yellow solid) was prepared from intermediate I-23e (1.50 g, 3.85 mmol, 1.0 eq), HATU (2.19 g, 5.77 mmol, 1.5 eq), DIEA (1.99 g, 15.40 mmol, 4.0 eq), and I-21f (552.86 mg, 3.85 mmol, 1.0 eq) by steps similar to those in Example I-1. (385.00 mg, yield: 20.9%). LC-MS MS-ESI (m/z) 479.2 [M+H]⁺. ¹H-NMR (400 MHz, CDCl₃) δ ppm 8.62 (d, *J = 5.2* Hz, 1H), 8.47 (d, *J =* 9.2 Hz, 1H), 8.29 (d, *J =* 9.2 Hz, 1H), 7.91 (d, *J =* 8.4 Hz, 1H), 7.67-7.61 (m, 2H), 7.53 (t, *J =* 7.7 Hz, 1H), 7.48-7.41 (m, 2H), 7.33 (d, *J =* 9.2 Hz, 1H), 6.51 (d, *J =* 5.2 Hz, 1H), 6.31 (d, *J =* 6.8 Hz, 1H), 4.62 (s, 1H), 4.37-4.29 (m, 2H), 3.92-3.82 (m, 2H), 3.52 (s, 3H), 3.27-3.14 (m, 2H), 2.73-2.60 (m, 2H).

### Example 24: Preparation of Compound I-24

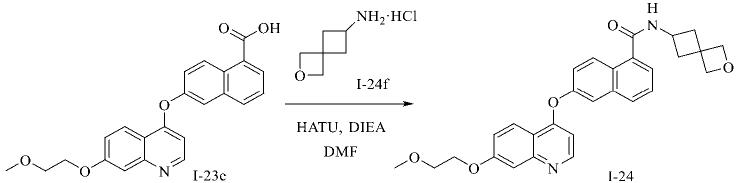

I-24 (yellow solid) was prepared from intermediate I-23e (155.76 mg, 0.40 mmol, 1.0 eq), HATU (228.14 mg, 0.6 mmol, 1.5 eq), DIEA (155.00 mg, 1.2 mmol, 3.0 eq), and I-24f (59.85 mg, 0.40 mmol, 1.0 eq) by steps similar to those in Example I-1. (85.00 mg, yield: 43.8%). LC-MS MS-ESI (m/z) 485.2 [M+H]⁺. ¹H-NMR (400 MHz, DMSO-d₆) δ ppm 8.82 (d, *J* = 7.3 Hz, 1H), 8.64 (d, *J =* 5.2 Hz, 1H), 8.33 (d, *J =* 9.2 Hz, 1H), 8.25 (d, *J* = 9.1 Hz, 1H), 8.05-8.00 (m, 1H), 7.87 (d, *J =* 2.4 Hz, 1H), 7.63-7.59 (m, 2H), 7.55 (dd, *J* = 9.2, 2.5 Hz, 1H), 7.47 (d, *J* = 2.4 Hz, 1H), 7.34 (dd, *J* = 9.1, 2.4 Hz, 1H), 6.58 (d, *J =* 5.2 Hz, 1H), 4.67 (s, 2H), 4.53 (s, 2H), 4.40-4.21 (m, 3H), 3.79-3.74 (m, 2H), 3.37 (s, 3H), 2.71-2.59 (m, 2H), 2.33-2.22 (m, 2H).

### Example 25: Preparation of Compound I-25

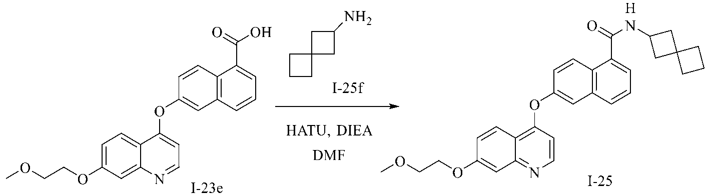

I-25 (yellow solid) was prepared from intermediate I-23e (155.76 mg, 0.40 mmol, 1.0 eq), HATU (228.14 mg, 0.6 mmol, 1.5 eq), DIEA (155.00 mg, 1.2 mmol, 3.0 eq), and I-25f (44.47 mg, 0.40 mmol, 1.0 eq) by steps similar to those in Example I-1. (93.00 mg, yield: 48.2%). LC-MS MS-ESI (m/z) 483.2 [M+H]⁺. ¹H-NMR (400 MHz, DMSO-d₆) δ ppm 8.78 (d, *J =* 6.8 Hz, 1H), 8.64 (d, *J* = 4.5 Hz, 1H), 8.32 (d, *J =* 8.8 Hz, 1H), 8.25 (d, *J* = 8.9 Hz, 1H), 8.02 (s, 1H), 7.86 (s, 1H), 7.66-7.50 (m, 3H), 7.46 (s, 1H), 7.33 (d, *J =* 8.1 Hz, 1H), 6.58 (d, *J =* 4.5 Hz, 1H), 4.46-4.19 (m, 3H), 3.84-3.72 (m, 2H), 3.36 (s, 3H), 2.45-2.35 (m, 2H), 2.13-2.00 (m, 4H), 1.98-1.88 (m, 2H), 1.87-1.76 (m, 2H).

### Example 26: Preparation of Compound I-26

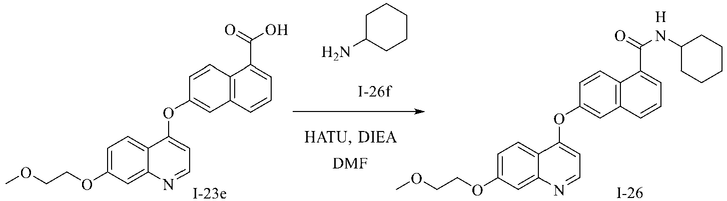

I-26 (yellow solid) was prepared from intermediate I-23e (150.00 mg, 0.39 mmol, 1.0 eq), HATU (220.40 mg, 0.58 mmol, 1.5 eq), DIEA (201.24 mg, 1.56 mmol, 4.0 eq), and I-26f (38.69 mg, 0.39 mmol, 1.0 eq) by steps similar to those in Example I-1. (40.00 mg, yield: 21.8%). LC-MS MS-ESI (m/z) 471.2 [M+H]⁺. ¹H-NMR (400 MHz, DMSO-d₆) δ ppm 8.64 (d, *J = 5.1* Hz, 1H), 8.49 (d, *J =* 7.9 Hz, 1H), 8.32 (d, *J* = 9.2 Hz, 1H), 8.25 (d, *J* = 9.1 Hz, 1H), 8.01 (d, *J =* 6.9 Hz, 1H), 7.86 (d, *J =* 2.1 Hz, 1H), 7.64-7.51 (m, 3H), 7.47 (d, *J =* 2.0 Hz, 1H), 7.34 (dd, *J =* 9.1, 2.1 Hz, 1H), 6.58 (d, *J =* 5.1 Hz, 1H), 4.39-4.25 (m, 2H), 3.86 (s, 1H), 3.82-3.72 (m, 2H), 3.37 (s, 3H), 2.00-1.89 (m, 2H), 1.85-173 (m, 2H), 1.63 (d, *J* = 12.1 Hz, 1H), 1.45-1.26 (m, 4H), 1.23-1.09 (s, 1H).

### Example 27: Preparation of Compound I-27

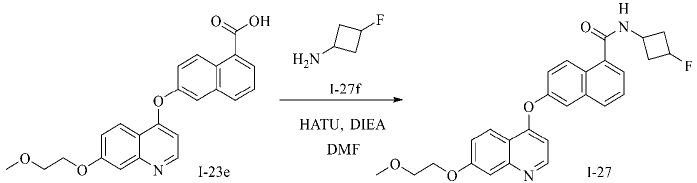

I-27 (yellow solid) was prepared from intermediate I-23e (150.00 mg, 0.39 mmol, 1.0 eq), HATU (220.40 mg, 0.58 mmol, 1.5 eq), DIEA (201.24 mg, 1.56 mmol, 4.0 eq), and I-27f (34.75 mg, 0.39 mmol, 1.0 eq) by steps similar to those in Example I-1. (40.00 mg, yield: 22.3%). LC-MS MS-ESI (m/z) 461.2 [M+H]⁺. ¹H-NMR (400 MHz, DMSO-d₆) δ ppm 8.96 (d, *J =* 6.9 Hz, 1H), 8.64 (d, *J =* 5.2 Hz, 1H), 8.34 (d, *J* = 9.2 Hz, 1H), 8.24 (d, *J* = 9.1 Hz, 1H), 8.03 (d, *J* = 7.9 Hz, 1H), 7.87 (d, *J =* 2.3 Hz, 1H), 7.69-7.58 (m, 2H), 7.54 (dd, *J =* 9.2, 2.4 Hz, 1H), 7.46 (d, *J =* 2.3 Hz, 1H), 7.33 (dd, *J =* 9.1, 2.4 Hz, 1H), 6.58 (d, *J =* 5.2 Hz, 1H), 5.41-5.15 (m, 1H), 4.64 (d, *J* = 3.7 Hz, 1H), 4.36-4.25 (m, 2H), 3.80-3.71 (m, 2H), 3.35 (s, 3H), 2.64-2.43 (m, 4H).

### Preparation of intermediate: I-28c

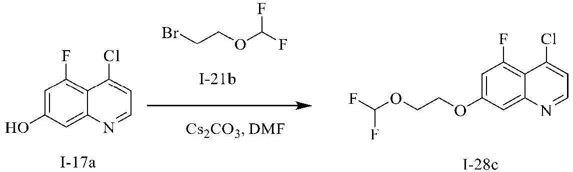

Intermediate I-28c (light yellow solid) was prepared from intermediate I-17a (394.00 mg, 2.00 mmol, 1.0 eq), Cs₂CO₃ (1.96 g, 6.00 mmol, 3.0 eq), and intermediate I-21b (prepared in-house, 1.05 g, 6.00 mmol, 3.0 eq) by steps similar to those in Example Intermediate I-1c. (541.00 mg, yield: 92.8%). LC-MS MS-ESI (m/z) 292.0 [M+H]⁺.

### Preparation of intermediate: I-28e

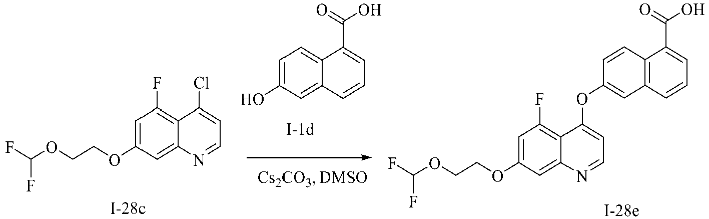

Intermediate I-28e (brown solid) was prepared from intermediate I-28c (541.00 mg, 1.86 mmol, 1.0 eq), Cs₂CO₃ (2.00 g, 6.14 mmol, 3.3 eq), and commercially available I-1d (349.68 mg, 1.86 mmol, 1.0 eq) by steps similar to those in Example Intermediate I-1e. (702.00 mg, yield: 85.1%). LC-MS MS-ESI (m/z) 444.1 [M+H]⁺.

### Example 28: Preparation of Compound I-28

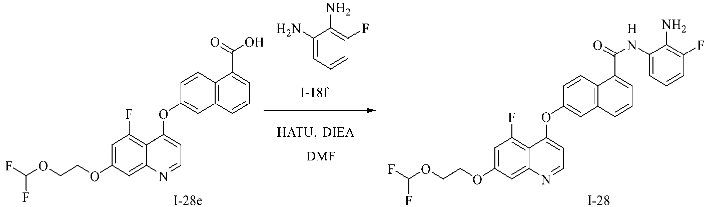

I-28 (yellow solid) was prepared from intermediate I-28e (640.00 mg, 1.44 mmol, 1.0 eq), HATU (820.60 mg, 2.16 mmol, 1.5 eq), DIEA (557.28 mg, 4.32 mmol, 3.0 eq), and I-18f (181.58 mg, 1.44 mmol, 1.0 eq) by steps similar to those in Example I-1. (259.00 mg, yield: 32.6%). LC-MS MS-ESI (m/z) 552.2 [M+H]⁺. ¹H-NMR (400 MHz, DMSO-d₆) δ ppm 10.04 (s, 1H), 8.68 (d, *J =* 4.9 Hz, 1H), 8.42 (d, *J =* 9.1 Hz, 1H), 8.09 (d, *J = 7.9* Hz, 1H), 7.91 (d, *J =* 6.7 Hz, 1H), 7.85 (s, 1H), 7.67 (t, *J =* 7.5 Hz, 1H), 7.58 (d, *J* = 8.7 Hz, 1H), 7.38 (s, 1H), 7.30 (d, *J* = 7.7 Hz, 1H), 7.21 (d, *J* = 13.0 Hz, 1H), 7.02-6.94 (m, 1H), 6.80 (t, ²*J* _{F-H} = 72.0 Hz, 1H), 6.73-6.63 (m, 2H), 5.00 (s, 2H), 4.48-4.38 (m, 2H), 4.31-4.22 (m, 2H).

### Example 29: Preparation of Compound I-29

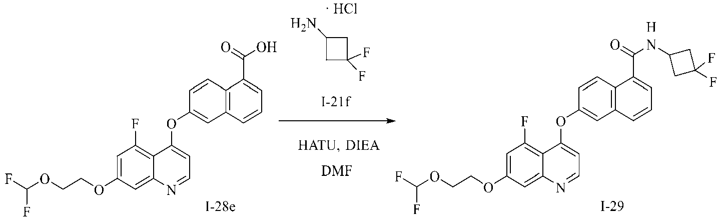

I-29 (yellow solid) was prepared from intermediate I-28e (443.00 mg, 1.00 mmol, 1.0 eq), HATU (570.00 mg, 1.50 mmol, 1.5 eq), DIEA (516.00 mg, 4.0 mmol, 4.0 eq), and I-21f (143.56 mg, 1.0 mmol, 1.0 eq) by steps similar to those in Example I-1. (182.00 mg, yield: 34.2%). LC-MS MS-ESI (m/z) 533.2 [M+H]⁺. ¹H-NMR (400 MHz, DMSO-d₆) δ ppm 9.06 (d, *J =* 6.4 Hz, 1H), 8.66 (d, *J =* 5.1 Hz, 1H), 8.36 (d, *J =* 9.2 Hz, 1H), 8.03 (d, *J* = 7.9 Hz, 1H), 7.81 (s, 1H), 7.67 (d, *J =* 6.8 Hz, 1H), 7.64-7.57 (m, 1H), 7.53 (d, *J =* 9.2 Hz, 1H), 7.37 (s, 1H), 7.20 (d, *J =* 13.2 Hz, 1H), 6.79 (t, ²*J* _{F-H} = 75.7 Hz, 1H), 6.65 (d, *J* = 5.1 Hz, 1H), 4.52-4.31 (m, 3H), 4.25 (s, 2H), 3.15-2.95 (m, 2H), 2.90-2.67 (m, 2H).

### Example 30: Preparation of Compound I-30

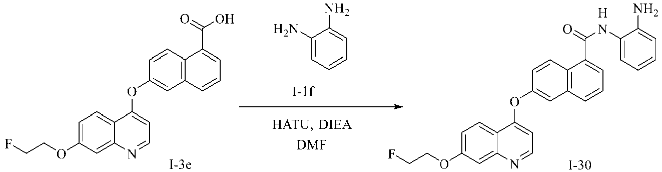

I-30 (yellow solid) was prepared from intermediate I-3e (1.60 g, 4.24 mmol, 1.0 eq), HATU (2.42 g, 6.36 mmol, 1.5 eq), DIEA (1.64 g, 12.72 mmol, 3.0 eq), and I-1f (458.34 mg, 4.24 mmol, 1.0 eq) by steps similar to those in Example I-1. (1.20 g, yield: 60.5%). LC-MS MS-ESI (m/z) 468.2 [M+H]⁺. ¹H-NMR (400 MHz, DMSO-d₆) δ ppm 9.88 (s, 1H), 8.65 (d, *J = 5.2* Hz, 1H), 8.45 (d, *J =* 9.2 Hz, 1H), 8.27 (d, *J =* 9.1 Hz, 1H), 8.08 (d, *J* = 8.3 Hz, 1H), 7.89 (t, *J* = 4.5 Hz, 2H), 7.71-7.62 (m, 1H), 7.58 (dd, *J* = 9.2, 2.5 Hz, 1H), 7.48 (t, *J* = 9.4 Hz, 1H), 7.42-7.29 (m, 2H), 7.05-6.96 (m, 1H), 6.85-6.80 (m, 1H), 6.68-6.62 (m, 1H), 6.61 (d, *J =* 5.2 Hz, 1H),5.00 (s, 2H), 4.94-4.87 (m, 1H), 4.79 (dd, *J* = 4.5, 2.9 Hz, 1H), 4.52-4.46 (m, 1H), 4.47-4.37 (m, 1H).

### Example 31: Preparation of Compound I-31

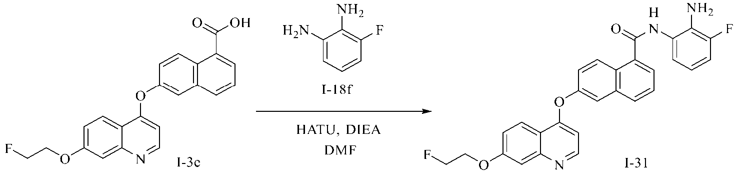

I-31 (yellow solid) was prepared from intermediate I-3e (250.00 mg, 0.66 mmol, 1.0 eq), HATU (376.20 mg, 0.99 mmol, 1.5 eq), DIEA (255.42 mg, 1.98 mmol, 3.0 eq), and I-18f (83.23 mg, 0.66 mmol, 1.0 eq) by steps similar to those in Example I-1. (187.00 mg, yield: 58.1%). LC-MS MS-ESI (m/z) 486.2 [M+H]⁺. ¹H-NMR (400 MHz, DMSO-d₆) δ ppm 10.04 (s, 1H), 8.66 (d, *J =* 5.2 Hz, 1H), 8.43 (d, *J* = 9.2 Hz, 1H), 8.27 (d, *J =* 9.1 Hz, 1H), 8.10 (d, *J =* 8.3 Hz, 1H), 7.95-7.87 (m, 2H), 7.73-7.62 (m, 1H), 7.59 (dd, *J =* 9.2, 2.5 Hz, 1H), 7.49 (d, *J = 2.5* Hz, 1H), 7.36 (dd, *J =* 9.1, 2.5 Hz, 1H), 7.29 (d, *J =* 8.0 Hz, 1H), 7.05- 6.95 (m, 1H), 6.70-6.63 (m, 1H), 6.61 (d, *J =* 5.2 Hz, 1H), 4.99 (s, 2H), 4.93-4.88 (m, 1H), 4.83-4.75 (m, 1H), 4.53-4.47 (m, 1H), 4.45-4.37 (m, 1H).

### Example 32: Preparation of Compound I-32

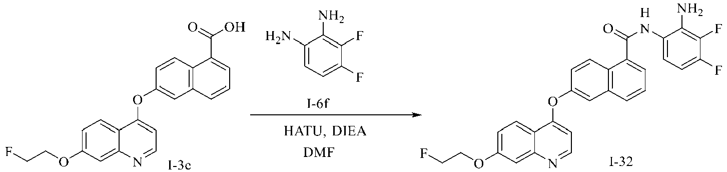

I-32 (yellow solid) was prepared from intermediate I-3e (200.00 mg, 0.53 mmol, 1.0 eq), HATU (302.10 mg, 0.79 mmol, 1.5 eq), DIEA (205.11 mg, 1.59 mmol, 3.0 eq), and I-6f (76.37 mg, 0.53 mmol, 1.0 eq) by steps similar to those in Example I-1. (80.00 mg, yield: 30.0%). LC-MS MS-ESI (m/z) 504.2 [M+H]⁺. ¹H-NMR (400 MHz, DMSO-d₆) δ ppm 9.93 (s, 1H), 8.63 (d, *J =* 5.2 Hz, 1H), 8.41 (d, *J* = 9.2 Hz, 1H), 8.25 (d, *J* = 9.1 Hz, 1H), 8.07 (d, *J = 8.3* Hz, 1H), 7.93 (d, *J* = 7.0 Hz, 1H), 7.89 (d, *J* = 2.3 Hz, 1H), 7.69-7.62 (m, 1H), 7.56 (dd, *J* = 9.2, 2.4 Hz, 1H), 7.47 (d, *J* = 2.4 Hz, 1H), 7.34 (dd, *J* = 9.1, 2.5 Hz, 1H), 7.26-7.15 (m, 1H), 6.66-6.54 (m, 2H), 5.35 (s, 2H), 4.92-4.83 (m, 1H), 4.82-4.70 (m, 1H), 4.54-4.45 (m, 1H), 4.43-4.32 (m, 1H).

### Example 33: Preparation of Compound I-33

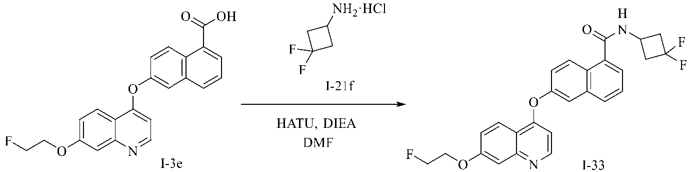

I-33 (yellow solid) was prepared from intermediate I-3e (150.95 mg, 0.4 mmol, 1.0 eq), HATU (228.14 mg, 0.6 mmol, 1.5 eq), DIEA (155.00 mg, 1.2 mmol, 3.0 eq), and I-21f (57.42 mg, 0.40 mmol, 1.0 eq) by steps similar to those in Example I-1. (66.00 mg, yield: 35.3%). LC-MS MS-ESI (m/z) 467.2 [M+H]⁺. ¹H-NMR (400 MHz, DMSO-d₆) δ ppm 9.09 (d, *J =* 6.6 Hz, 1H), 8.66 (d, *J =* 5.2 Hz, 1H), 8.38 (d, *J =* 9.2 Hz, 1H), 8.27 (d, *J =* 9.1 Hz, 1H), 8.06 (d, *J* = 8.2 Hz, 1H), 7.89 (d, *J* = 2.5 Hz, 1H), 7.71-7.67 (m, 1H), 7.66-7.60 (m, 1H), 7.57 (dd, *J =* 9.2, 2.5 Hz, 1H), 7.50 (d, *J =* 2.4 Hz, 1H), 7.37 (dd, *J =* 9.2, 2.5 Hz, 1H), 6.60 (d, *J* = 5.2 Hz, 1H), 4.97-4.74 (m, 2H), 4.54-4.31 (m, 3H), 3.13-2.94 (m, 2H), 2.85-2.70 (m, 2H).

### Example 34: Preparation of Compound I-34

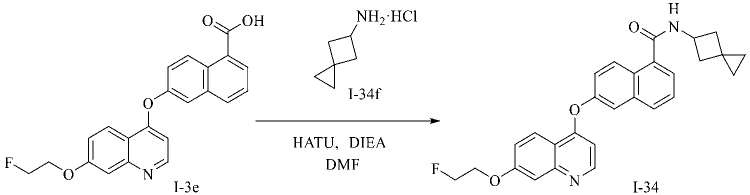

I-34 (yellow solid) was prepared from intermediate I-3e (150.95 mg, 0.4 mmol, 1.0 eq), HATU (228.14 mg, 0.60 mmol, 1.5 eq), DIEA (155.00 mg, 1.2 mmol, 3.0 eq), and I-34f (53.45 mg, 0.40 mmol, 1.0 eq) by steps similar to those in Example I-1. (57.00 mg, yield: 31.2%). LC-MS MS-ESI (m/z) 457.2 [M+H]⁺. ¹H-NMR (400 MHz, DMSO-d₆) δ ppm 8.96 (d, *J* = 7.2 Hz, 1H), 8.66 (d, *J =* 5.1 Hz, 1H), 8.35 (d, *J =* 9.2 Hz, 1H), 8.28 (d, *J =* 9.1 Hz, 1H), 8.04 (d, *J =* 7.7 Hz, 1H), 7.88 (d, *J =* 2.1 Hz, 1H), 7.69-7.58 (m, 2H), 7.56 (dd, *J =* 9.2, 2.2 Hz, 1H), 7.50 (d, *J =* 2.0 Hz, 1H), 7.37 (dd, *J* = 9.1, 2.2 Hz, 1H), 6.60 (d, *J =* 5.2 Hz, 1H), 4.86 (d, *J =* 47.8 Hz, 2H), 4.75-4.65 (m, 1H), 4.47 (d, *J =* 30.3 Hz, 2H), 2.46-2.28 (m, 4H), 0.57-0.40 (m, 4H).

### Example 35: Preparation of Compound I-35

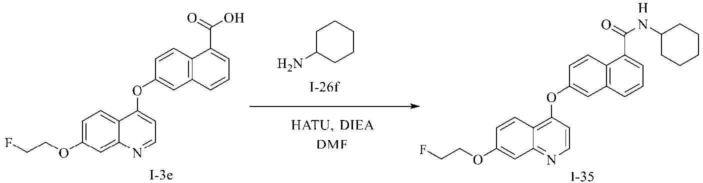

I-35 (yellow solid) was prepared from intermediate I-3e (150.95 mg, 0.4 mmol, 1.0 eq), HATU (228.14 mg, 0.60 mmol, 1.5 eq), DIEA (155.00 mg, 1.2 mmol, 3.0 eq), and I-26f (39.68 mg, 0.40 mmol, 1.0 eq) by steps similar to those in Example I-1. (40.00 mg, yield: 21.8%). LC-MS MS-ESI (m/z) 459.2 [M+H]⁺. ¹H-NMR (400 MHz, DMSO-d₆) δ ppm 8.64 (d, *J =* 5.1 Hz, 1H), 8.49 (d, *J =* 7.9 Hz, 1H), 8.29 (dd, *J =* 19.6, 9.2 Hz, 2H), 8.00 *(d, J =* 5.2 Hz, 1H), 7.86 (d, *J =* 1.7 Hz, 1H), 7.63 -7.51 (m, 3H), 7.48 (s, 1H), 7.35 (dd, *J* = 9.1, 1.9 Hz, 1H), 6.57 (d, *J =* 5.1 Hz, 1H), 4.85 (d, *J =* 47.9 Hz, 2H), 4.46 (d, *J =* 30.2 Hz, 2H), 3.86 (s, 1H), 1.96-1.87 (m, 2H), 1.81-1.70 (m, 2H), 1.65-1.57 (m, 1H), 1.42-1.26 (m, 4H), 1.20-1.08 (m, 1H).

### Preparation of intermediate: I-36e

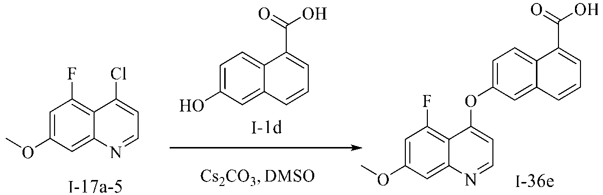

Intermediate I-36e (brown solid) was prepared from intermediate 1-17a-5 (800.00 mg, 3.78 mmol, 1.0 eq), Cs₂CO₃ (4.07 g, 12.47 mmol, 3.3 eq), and commercially available I-1d (710.64 mg, 3.78 mmol, 1.0 eq) by steps similar to those in Example Intermediate I-1e. (1.00 g, yield: 72.8%). LC-MS MS-ESI (m/z) 364.1 [M+H]⁺.

### Example 36: Preparation of Compound I-36

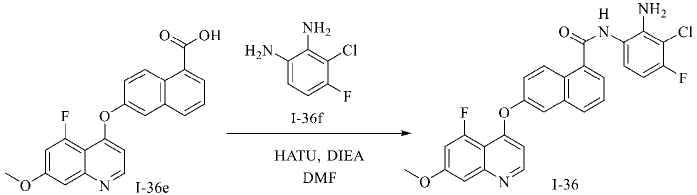

I-36 (yellow solid) was prepared from intermediate I-36e (200.00 mg, 0.55 mmol, 1.0 eq), HATU (313.50 mg, 0.82 mmol, 1.5 eq), DIEA (212.85 mg, 1.65 mmol, 3.0 eq), and commercially available I-36f (131.70 mg, 0.82 mmol, 1.5 eq) by steps similar to those in Example I-1. (120.00 mg, yield: 43.1%). LC-MS MS-ESI (m/z) 506.1 [M+H]⁺. ¹H-NMR (400 MHz, DMSO-d₆) δ ppm 9.93 (s, 1H), 8.66 (d, *J =* 4.7 Hz, 1H), 8.44 (d, *J =* 9.0 Hz, 1H), 8.07 (d, *J =* 7.9 Hz, 1H), 7.96 (d, *J =* 6.6 Hz, 1H), 7.83 (s, 1H), 7.66 (t, *J =* 7.3 Hz, 1H), 7.55 (d, *J =* 8.7 Hz, 1H), 7.45-7.30 (m, 2H), 7.16 (d, *J =* 13.2 Hz, 1H), 6.72-6.60 (m, 2H), 5.55 (s, 2H), 3.96 (s, 3H).

### Preparation of intermediate: I-37g

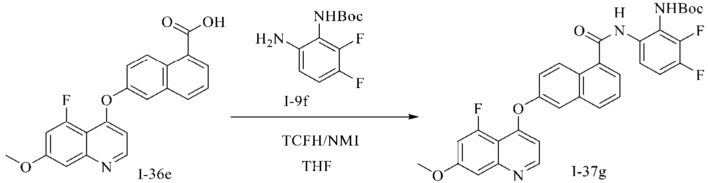

Intermediate I-37g (yellow solid) was prepared from intermediate I-36e (700.00 mg, 1.93 mmol, 1.0 eq), NMI (712.63 mg, 8.68 mmol, 4.5 eq), I-9f (705.74 mg, 2.89 mmol, 1.5 eq), TCFH (809.20 mg, 2.89 mmol, 1.5 eq), and THF (20.0 mL) by steps similar to those in Example I-9g. (462.00 mg, yield: 40.6%). LC-MS MS-ESI (m/z) 590.2 [M+H]⁺.

### Example 37: Preparation of Compound I-37

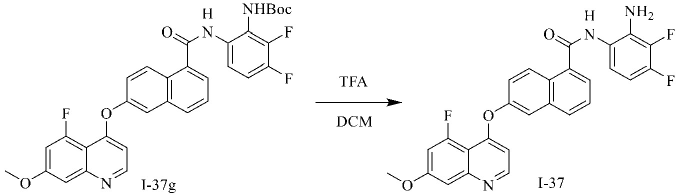

I-37 (yellow solid) was prepared from intermediate I-37g (462.00 mg, 0.78 mmol, 1.0 eq), TFA (6 mL), and DCM (20 mL) by steps similar to those in Example I-9. (263.0 mg, yield 68.9%). LC-MS MS-ESI (m/z) 490.1 [M+H]⁺. ¹H-NMR (400 MHz, DMSO-d₆) δ ppm 9.95 (s, 1H), 8.67 (d, *J* = 5.3 Hz, 1H), 8.42 (d, *J* = 9.2 Hz, 1H), 8.08 (d, *J* = 8.2 Hz, 1H), 7.94 (d, *J* = 6.8 Hz, 1H), 7.84 (d, *J* = 2.4 Hz, 1H), 7.76-7.60 (m, 1H), 7.56 (dd, *J =* 9.2, 2.5 Hz, 1H), 7.34 (d, *J* = 2.2 Hz, 1H), 7.27-7.19 (m, 1H), 7.17 (dd, *J =* 13.4, 2.4 Hz, 1H), 6.77-6.51 (m, 2H), 5.37 (s, 2H), 3.96 (s, 3H).

### Preparation of intermediate: I-38c

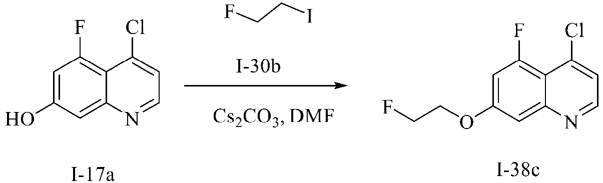

Intermediate I-38c (light yellow solid) was prepared from intermediate I-17a (396.00 mg, 2.00 mmol, 1.0 eq), Cs₂CO₃ (1.95 g, 6.00 mmol, 3.0 eq), and I-30b (1.04 g, 6.00 mmol, 3.0 eq) by steps similar to those in Example Intermediate I-1c. (460.00 mg, yield: 94.4%). LC-MS MS-ESI (m/z) 244.0 [M+H]⁺.

### Preparation of intermediate: I-38e

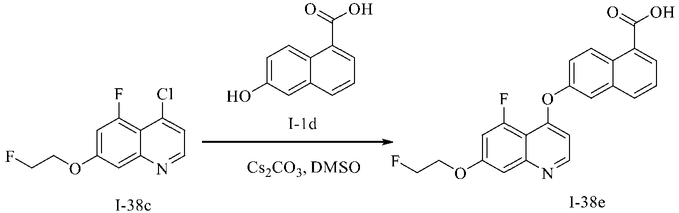

Intermediate I-38e (brown solid) was prepared from intermediate I-38c (460.00 mg, 1.89 mmol, 1.0 eq), Cs₂CO₃ (2.03 g, 6.24 mmol, 3.3 eq), and commercially available I-1d (355.70 mg, 1.89 mmol, 1.0 eq) by steps similar to those in Example Intermediate I-1e. (680.00 mg, yield: 91.0%). LC-MS MS-ESI (m/z) 396.1 [M+H]⁺.

### Example 38: Preparation of Compound I-38

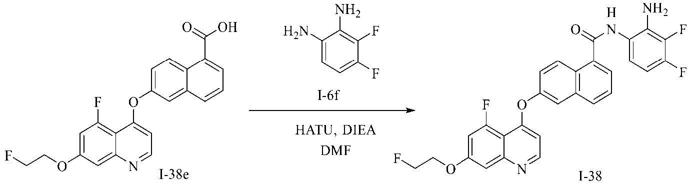

I-38 (yellow solid) was prepared from intermediate I-38e (680.00 mg, 1.72 mmol, 1.0 eq), HATU (980.40 mg, 2.58 mmol, 1.5 eq), DIEA (665.64 mg, 5.16 mmol, 3.0 eq), and commercially available I-6f (247.85 mg, 1.72 mmol, 1.0 eq) by steps similar to those in Example I-1. (396.00 mg, yield: 44.1%). LC-MS MS-ESI (m/z) 522.1 [M+H]⁺. ¹H-NMR (400 MHz, DMSO-d₆) δ ppm 9.96 (s, 1H), 8.67 (d, *J =* 5.2 Hz, 1H), 8.42 (d, *J =* 9.2 Hz, 1H), 8.08 (d, *J* = 8.3 Hz, 1H), 7.94 (d, *J* = 7.0 Hz, 1H), 7.84 (d, *J* = 2.3 Hz, 1H), 7.71 -7.62 (m, 1H), 7.56 (dd, *J =* 9.2, 2.5 Hz, 1H), 7.38 (d, *J =* 2.1 Hz, 1H), 7.26-7.18 (m, 2H), 6.71-6.57 (m, 2H), 5.37 (s, 2H), 4.94-4.71 (m, 2H), 4.55-4.38 (m, 2H).

### Preparation of intermediate: I-39a-3

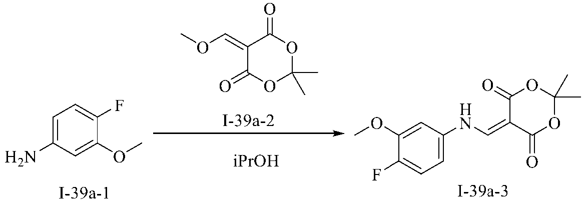

I-39a-1 (10.00 g, 70.85 mmol, 1.0 eq) was dissolved in isopropanol (200 mL), and I-39a-2 (15.80 g, 84.87 mmol, 1.2 eq) was added. The resulting mixture was heated to 70 °C and stirred for 2 h. The mixture was cooled to room temperature, and n-pentane (200 mL) was added. The precipitated solid was collected by filtration, rinsed 3 times with isopropanol/n-pentane (1:1, 10 mL), and dried in a blast drying oven at 50 °C for 8 h to give intermediate I-39a-3 as a white solid (17.0 g, yield: 81.2%). LC-MS MS-ESI (m/z) 296.1 [M+H]⁺.

### Preparation of intermediate: I-39a-4

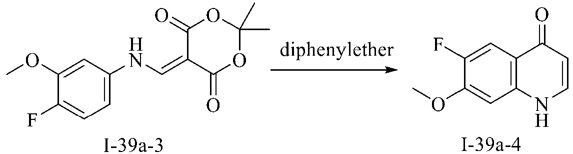

Intermediate I-39a-4 (white solid) was prepared from intermediate I-39a-3 (12.00 g, 40.64 mmol, 1.0 eq) and diphenyl ether (300 mL) by steps similar to those in Example I-17a-4. (5.50 g, yield: 70.0%). LC-MS MS-ESI (m/z) 194.0 [M+H]⁺.

### Preparation of intermediate: I-39a-5

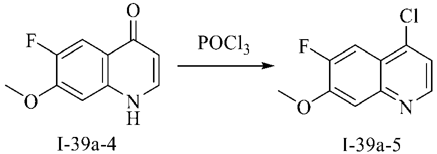

Intermediate I-39a-5 (white solid) was prepared from intermediate I-39a-4 (5.50 g, 28.47 mmol, 1.0 eq) and phosphorus oxychloride (30.0 mL) by steps similar to those in Example 1-17a-5. (4.50 g, yield: 74.7%). LC-MS MS-ESI (m/z) 212.0 [M+H]⁺.

### Preparation of intermediate: I-39a

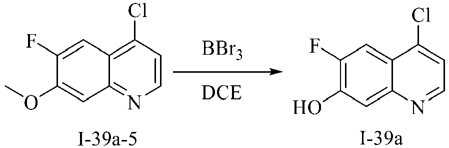

Intermediate I-39a (yellow solid) was prepared from intermediate I-39a-5 (4.50 g, 21.27 mmol, 1.0 eq), a 1.0 M solution of boron tribromide in dichloromethane (75 mL, 75.00 mmol, 3.53 eq), and 1,2-dichloroethane (DCE, 210 mL) by steps similar to those in Example I-17a. (2.10 g, yield: 50.0%). LC-MS MS-ESI (m/z) 198.0 [M+H]⁺. ¹H-NMR (400 MHz, DMSO-d₆) δ ppm 11.22 (s, 1H), 8.68 (d, *J =* 4.0 Hz, 1H), 7.86 (d, *J =* 12.0 Hz, 1H),7.57 (d, *J =* 8.0 Hz, 1H), 7.51 (d, *J =* 8.0 Hz, 1H).

### Preparation of intermediate: I-39c

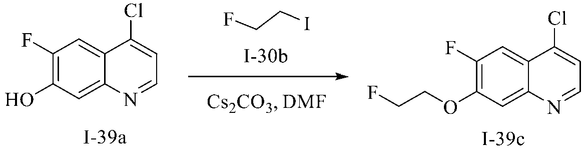

Intermediate I-39c (light yellow solid) was prepared from intermediate I-39a (396.00 mg, 2.00 mmol, 1.0 eq), Cs₂CO₃ (1.95 g, 6.00 mmol, 3.0 eq), and I-30b (1.04 g, 6.00 mmol, 3.0 eq) by steps similar to those in Example Intermediate I-1c. (480.00 mg, yield: 98.5%). LC-MS MS-ESI (m/z) 244.0 [M+H]⁺.

### Preparation of intermediate: I-39e

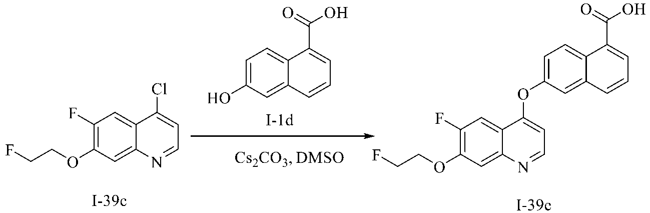

Intermediate I-39e (brown solid) was prepared from intermediate I-39c (480.00 mg, 1.97 mmol, 1.0 eq), Cs₂CO₃ (2.12 g, 6.50 mmol, 3.3 eq), and commercially available I-1d (370.75 mg, 1.97 mmol, 1.0 eq) by steps similar to those in Example Intermediate I-1e. (710.00 mg, yield: 91.1%). LC-MS MS-ESI (m/z) 396.1 [M+H]⁺.

### Example 39: Preparation of Compound I-39

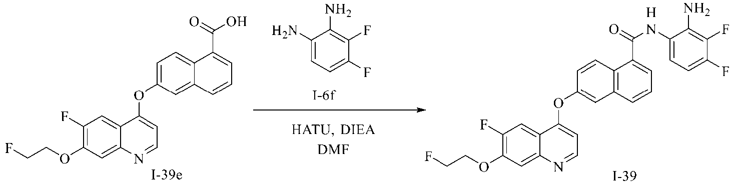

I-39 (yellow solid) was prepared from intermediate I-39e (710.00 mg, 1.80 mmol, 1.0 eq), HATU (1.03 g, 2.70 mmol, 1.5 eq), DIEA (696.60 mg, 5.40 mmol, 3.0 eq), and commercially available I-6f (259.38 mg, 1.80 mmol, 1.0 eq) by steps similar to those in Example I-1. (54.00 mg, yield: 5.8%). LC-MS MS-ESI (m/z) 522.1 [M+H]⁺. ¹H-NMR (400 MHz, DMSO-d₆) δ ppm 9.96 (s, 1H), 8.65 (d, *J =* 5.1 Hz, 1H), 8.44 (d, *J =* 9.2 Hz, 1H), 8.09 (t, *J =* 10.8 Hz, 2H), 8.02-7.87 (m, 2H), 7.79-7.62 (m, 2H), 7.58 (d, *J =* 8.8 Hz, 1H), 7.29-7.17 (m, 1H), 6.76-6.53 (m, 2H), 5.37 (s, 2H), 4.88 (d, *J =* 47.8 Hz, 2H), 4.56 (d, *J = 29.7* Hz, 2H).

### Preparation of intermediate: I-40c

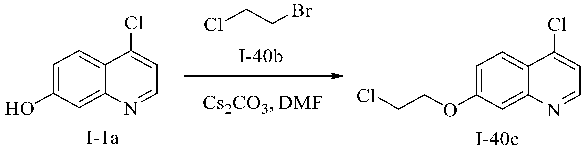

Intermediate I-40c (light yellow solid) was prepared from I-1a (1.79 g, 10.0 mmol, 1.0 eq), Cs₂CO₃ (9.77 g, 30.0 mmol, 3.0 eq), and I-40b (4.30 g, 30.0 mmol, 3.0 eq) by steps similar to those in Example Intermediate I-1c. (2.25 g, yield: 92.9%). LC-MS MS-ESI (m/z) 242.0 [M+H]⁺.

### Preparation of intermediate: I-40e

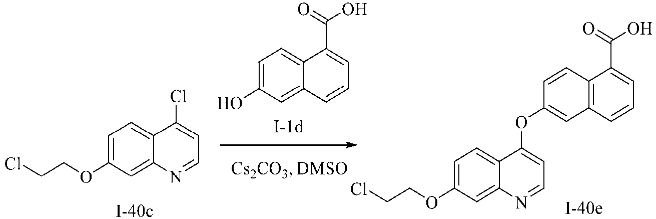

Intermediate I-40e (brown solid) was prepared from intermediate 1-40c (2.25 g, 9.29 mmol, 1.0 eq), Cs₂CO₃ (9.98 g, 30.65 mmol, 3.3 eq), and commercially available I-1d (1.75 g, 9.29 mmol, 1.0 eq) by steps similar to those in Example Intermediate I-1e. (3.20 g, yield: 87.46%). LC-MS MS-ESI (m/z) 394.1 [M+H]⁺.

### Example 40: Preparation of Compound I-40

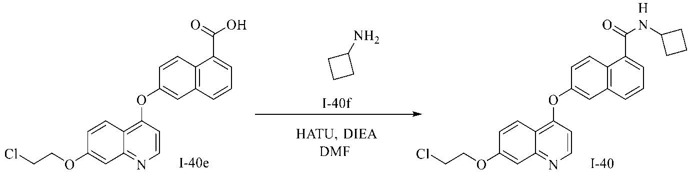

I-40 (yellow solid) was prepared from intermediate I-40e (157.53 mg, 0.40 mmol, 1.0 eq), HATU (228.14 mg, 0.6 mmol, 1.5 eq), DIEA (155.00 mg, 1.2 mmol, 3.0 eq), and I-40f (28.45 mg, 0.40 mmol, 1.0 eq) by steps similar to those in Example I-1. (42.00 mg, yield: 23.5%). LC-MS MS-ESI (m/z) 447.1 [M+H]⁺. ¹H-NMR (400 MHz, DMSO-d₆) δ ppm 8.86 (d, *J* = 7.6 Hz, 1H), 8.66 (d, *J =* 5.1 Hz, 1H), 8.34 (d, *J* = 9.2 Hz, 1H), 8.28 (d, *J* = 9.1 Hz, 1H), 8.03 (d, *J =* 7.0 Hz, 1H), 7.88 (d, *J =* 2.3 Hz, 1H), 7.64-7.58 (m, 2H), 7.55 (dd, *J =* 9.2, 2.4 Hz, 1H), 7.49 (d, *J =* 2.2 Hz, 1H), 7.37 (dd, *J* = 9.1, 2.3 Hz, 1H), 6.60 (d, *J = 5.2* Hz, 1H), 4.55-4.45 (m, 3H), 4.12-4.03 (m m, 2H), 2.35-2.25 (m, 2H), 2.15-2.02 (m, 2H), 1.76-1.67 (m, 2H).

### Preparation of intermediate: I-41c

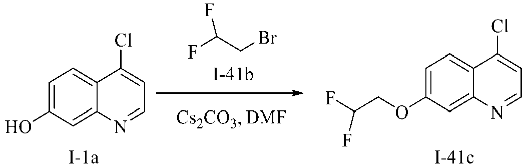

Intermediate I-41c (yellow solid) was prepared from commercially available I-1a (1.80 g, 10.02 mmol, 1.0 eq), Cs₂CO₃ (9.79 g, 30.06 mmol, 3.0 eq), and I-41b (4.36 g, 30.06 mmol, 3.0 eq) by steps similar to those in Example Intermediate I-1c. (1.70 g, yield: 69.6%). LC-MS MS-ESI (m/z) 244.0 [M+H]⁺.

### Preparation of intermediate: I-41e

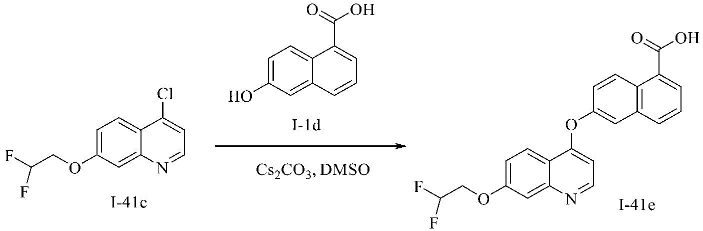

Intermediate I-41e (brown solid) was prepared from intermediate 1-41c (prepared in-house, 731.00 mg, 3.00 mmol, 1.0 eq), Cs₂CO₃ (3.22 g, 9.90 mmol, 3.3 eq), and commercially available I-1d (564.60 mg, 3.00 mmol, 1.0 eq) by steps similar to those in Example Intermediate I-1e. (800.00 mg, yield: 67.4%). LC-MS MS-ESI (m/z) 396.1 [M+H]⁺.

### Example 41: Preparation of Compound I-41

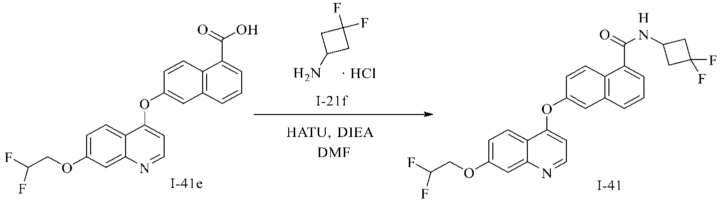

I-41 (yellow solid) was prepared from intermediate I-41e (110.70 mg, 0.28 mmol, 1.0 eq), HATU (159.68 mg, 0.42 mmol, 1.5 eq), DIEA (108.53 mg, 0.84 mmol, 3.0 eq), and I-41f (40.20 mg, 0.28 mmol, 1.0 eq) by steps similar to those in Example I-1. (40.00 mg, yield: 29.5%). LC-MS MS-ESI (m/z) 485.1 [M+H]⁺. ¹H-NMR (400 MHz, DMSO-d₆) δ ppm 9.09 (d, *J =* 6.5 Hz, 1H), 8.68 (d, *J =* 5.1 Hz, 1H), 8.39 (d, *J =* 9.2 Hz, 1H), 8.29 (d, *J* = 9.2 Hz, 1H), 8.06 (d, *J =* 8.1 Hz, 1H), 7.89 (d, *J =* 2.2 Hz, 1H), 7.70 (d, *J =* 6.7 Hz, 1H), 7.67-7.60 (m, 1H), 7.59-7.53 (m, 2H), 7.40 (dd, *J =* 9.2, 2.3 Hz, 1H), 6.62 (d, *J =* 5.2 Hz, 1H), 6.52 (tt, *J* = 56.0, 3.2 Hz, 1H),4.57 (td, *J* = 14.7, 3.2 Hz, 2H), 4.45-4.30 (m, 1H), 3.13 -2.96 (m, 2H), 2.88-2.70 (m, 2H).

### Preparation of intermediate: I-42c

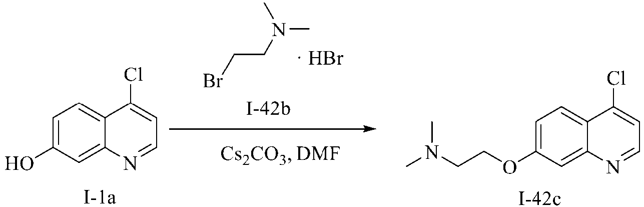

Intermediate I-42c (yellow solid) was prepared from commercially available I-1a (1.80 g, 10.02 mmol, 1.0 eq), Cs₂CO₃ (9.79 g, 30.06 mmol, 3.0 eq), and I-42b (2.33 g, 10.02 mmol, 1.0 eq) by steps similar to those in Example Intermediate I-1c. (1.60 g, yield: 63.7%). LC-MS MS-ESI (m/z) 251.1 [M+H]⁺.

### Preparation of intermediate: I-42e

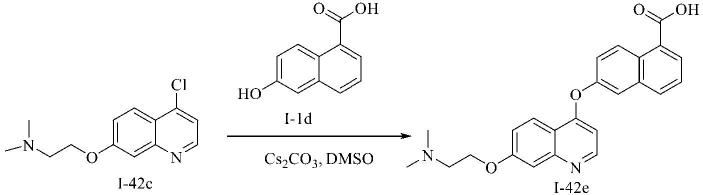

Intermediate I-42e (brown solid) was prepared from intermediate I-42c (prepared in-house, 752.10 mg, 3.00 mmol, 1.0 eq), Cs₂CO₃ (3.22 g, 9.90 mmol, 3.3 eq), and commercially available I-1d (564.60 mg, 3.00 mmol, 1.0 eq) by steps similar to those in Example Intermediate I-1e. (800.00 mg, yield: 66.2%). LC-MS MS-ESI (m/z) 403.2 [M+H]⁺.

### Example 42: Preparation of Compound I-42

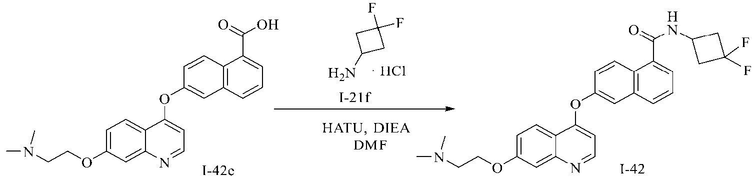

I-42 (yellow solid) was prepared from intermediate I-42e (112.67 mg, 0.28 mmol, 1.0 eq), HATU (159.68 mg, 0.42 mmol, 1.5 eq), DIEA (108.53 mg, 0.84 mmol, 3.0 eq), and I-21f (40.21 mg, 0.28 mmol, 1.0 eq) by steps similar to those in Example I-1. (40.00 mg, yield: 29.1%). LC-MS MS-ESI (m/z) 492.2 [M+H]⁺.

### Preparation of intermediate: I-43c

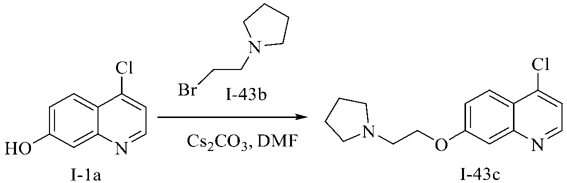

Intermediate I-43c (yellow solid) was prepared from commercially available I-1a (1.80 g, 10.02 mmol, 1.0 eq), Cs₂CO₃ (9.79 g, 30.06 mmol, 3.0 eq), and I-43b (1.78 g, 10.02 mmol, 1.0 eq) by steps similar to those in Example Intermediate I-1c. (1.60 g, yield: 57.7%). LC-MS MS-ESI (m/z) 277.1 [M+H]⁺.

### Preparation of intermediate: I-43e

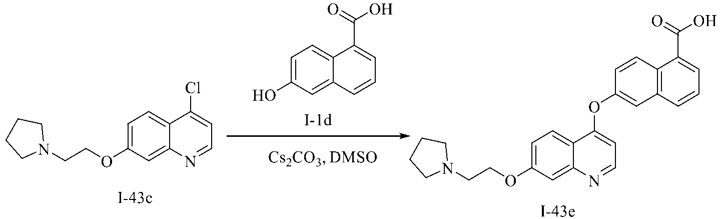

Intermediate I-43e (brown solid) was prepared from intermediate I-43c (prepared in-house, 830.40 mg, 3.00 mmol, 1.0 eq), Cs₂CO₃ (3.22 g, 9.90 mmol, 3.3 eq), and commercially available I-1d (564.60 mg, 3.00 mmol, 1.0 eq) by steps similar to those in Example Intermediate I-1e. (850.00 mg, yield: 66.1%). LC-MS MS-ESI (m/z) 429.2 [M+H]⁺.

### Example 43: Preparation of Compound I-43

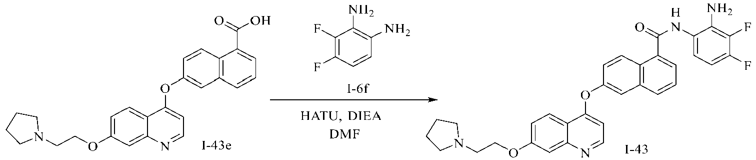

I-43 (yellow solid) was prepared from intermediate I-43e (119.98 mg, 0.28 mmol, 1.0 eq), HATU (159.68 mg, 0.42 mmol, 1.5 eq), DIEA (108.53 mg, 0.84 mmol, 3.0 eq), and I-6f (40.32 mg, 0.28 mmol, 1.0 eq) by steps similar to those in Example I-1. (40.00 mg, yield: 25.7%). LC-MS MS-ESI (m/z) 555.2 [M+H]⁺. ¹H-NMR (400 MHz, DMSO-d₆) δ ppm 9.96 (s, 1H), 8.64 (d, *J =* 5.2 Hz, 1H), 8.43 (d, *J =* 9.2 Hz, 1H), 8.24 (d, *J =* 9.1 Hz, 1H), 8.09 (d, *J* = 8.3 Hz, 1H), 7.95 (d, *J* = 6.6 Hz, 1H), 7.90 (d, *J* = 2.4 Hz, 1H), 7.71-7.63 (m, 1H), 7.57 (dd, *J =* 9.2, 2.5 Hz, 1H), 7.46 (d, *J =* 2.5 Hz, 1H), 7.32 (dd, *J =* 9.1, 2.5 Hz, 1H), 7.27 -7.19 (m, 1H), 6.72-6.56 (m, 2H), 5.38 (s, 2H), 4.27 (t, *J =* 5.8 Hz, 2H), 2.89 (t, *J =* 5.7 Hz, 2H), 2.65-2.52 (m, 4H), 1.83-1.58 (m m, 4H).

### Example 44: Preparation of Compound I-44

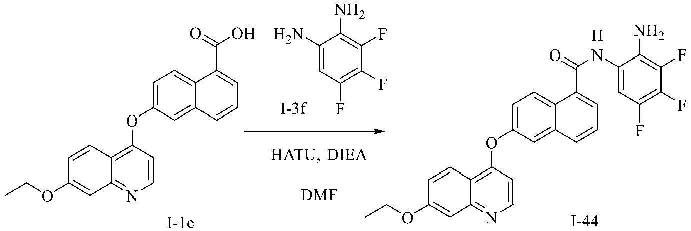

I-44 (yellow solid) was prepared from intermediate I-1e (205.0 mg, 0.57 mmol, 1.0 eq), HATU (325.10 mg, 0.855 mmol, 1.5 eq), DIEA (221.00 mg, 1.71 mmol, 3.0 eq), and I-3f (92.41 mg, 0.57 mmol, 1.0 eq) by steps similar to those in Example I-1. (65.00 mg, yield: 22.65%). LC-MS MS-ESI (m/z) 504.2 [M+H]⁺. ¹H-NMR (400 MHz, DMSO-d₆) δ ppm 10.03 (s, 1H), 8.64 (d, *J = 4.9* Hz, 1H), 8.43 (d, *J =* 9.1 Hz, 1H), 8.24 (d, *J =* 9.1 Hz, 1H), 8.10 (d, *J =* 8.1 Hz, 1H), 7.98-7.87 (m, 2H), 7.67 (t, *J =* 7.6 Hz, 1H), 7.60-7.46 (m, 2H), 7.42 (s, 1H), 7.30 (d, *J =* 9.0 Hz, 1H), 6.58 (d, *J =* 4.9 Hz, 1H), 5.27 (s, 2H), 4.23 (d, *J* = 6.9 Hz, 2H), 1.43 (t, *J =* 6.7 Hz, 3H).

In addition, other compounds of the present disclosure can be prepared by steps similar to those in the above examples.

### In Vitro Biological Evaluations

### Example A. Tests of Inhibitory Activity and Relative Selectivity of Compounds of the Present Disclosure for Aurora B and VEGFR2 Kinases

Preparation of working solutions of compounds in DMSO with gradient concentrations: 0.2 mM solutions of the compounds in DMSO were prepared and serially diluted with DMSO to form working solutions with 10 gradient concentrations (corresponding to final compound concentrations of 1000 nM, 300 nM, 100 nM, 30 nM, 10 nM, 3 nM, 1 nM, 0.3 nM, 0.1 nM, and 0.03 nM in the reaction system).

Tests of inhibitory activity against Aurora B kinase and VEGFR2: The half maximal inhibitory concentrations (IC₅₀) of the compounds against Aurora B kinase and VEGFR2 were determined using HTRF KinEASE-TK kit (Cisbio, Cat# 62TK0PEC) and HTRF KinEASE-STK kit (Cisbio, Cat# 61ST2BLE). A 2× kinase & Metal solution (Aurora B/Cat# 05-102 and VEGFR2/Cat# 08-191, both purchased from Carna) and a 2× Substrate & ATP solution (for Aurora B and VEGFR2, substrates S2 and TK were used, respectively) were prepared according to the kit instructions. The working solutions of the test compounds with gradient concentrations were added to a 384-well plate at 25 nL/well, and 2.5 µL of the 2× kinase & Metal solution was added to each well. The plate was sealed with a plate-sealing film, well shaken, centrifuged, and then incubated at 25 °C for 10 min. Then, the 2 × Substrate & ATP solution was added at 2.5 µL/well, and the plate was incubated again at 25 °C for 30 min. Finally, a 2 × XL665 & Antibody kinase detection reaction solution was added at 5 µL/well. After the plate was incubated at 25 °C for 60 min, the fluorescence signals at wavelengths of 665 nm and 620 nm in each well were measured by a microplate reader.

The 665 nm/620 nm fluorescence signal ratio (X) of each well was calculated. With the compound-free well as a negative control (Y) and the enzyme-free well as a positive control (Z), the inhibition rate of the compound in each test well against the corresponding kinase was calculated using the formula: inhibition (%) = 100% - (X-Z)/(Y-Z) × 100%, and the half maximal inhibitory concentration (IC₅₀) of each compound against the corresponding kinase was calculated by GraphPad 7.0 software.

The relative selectivity of Aurora B kinase inhibition intensity is represented by the ratio of the IC₅₀ of the compound against VEGFR2 to the IC₅₀ of the compound against Aurora B, i.e., IC_{50-VEGFR2}/IC_{50-Aurora B}. A higher ratio indicates that the compound has stronger inhibition intensity against Aurora B than against VEGFR2. Specific data are shown in Table 2 below:

**Table 2. The inhibitory activity and relative selectivity of the compounds of the present disclosure for Aurora B and VEGFR2 kinases**

| **Example** | **Aurora B IC₅₀ (nM)** | **Selectivity for Aurora B IC_{50-VEGFR2}/ IC_{50-Aurora B}** | **Example** | **Aurora B IC₅₀ (nM)** | **Selectivity for Aurora B IC_{50-VEGFR2}/ IC_{50-Aurora B}** |
|---|---|---|---|---|---|
| **I-5** | 2.833 | 49.6 | **I-6** | 3.178 | 18.64 |
| **I-9** | 1.795 | 17.57 | **I-10** | 3.521 | 13.34 |
| **I-17** | 4.01 | 8.16 | **I-18** | 6.894 | 9.75 |
| **I-19** | 5.62 | 9.08 | **I-24** | 1.704 | 109.61 |
| **I-21** | 2.118 | 12.27 | **I-28** | 4.12 | 9.68 |
| **I-23** | 1.238 | 9.49 | **I-32** | 2.013 | 8.07 |
| **I-25** | 1.547 | 38.05 | **I-36** | 2.92 | 7.75 |
| **I-27** | 0.896 | 10.23 | **I-42** | 3.863 | 13.9 |
| **I-33** | 2.654 | 376.79 | **I-44** | 6.715 | 12.14 |
| **I-35** | 5.262 | 9.55 | I-7 (control) | 417.46 6 | 0.17 |
| **I-37** | 2.63 | 14.19 | I-11 (control) | 33.946 | 0.78 |
| **I-41** | 2.218 | 127.51 | I-12 (control) | 18.994 | 5.12 |
| I-13 (control) | 127.21 5 | 0.27 | I-14 (control) | 53.088 | 18.84 |
| Control molecule 02 (No. 32) | 2.078 | 4.16 | Control molecule 01 (No. 31, chiauranib) | 10.83 | 1.54 |

Compounds No. 31 (chiauranib) and No. 32 are disclosed in examples of patent CN101906076A. With reference to the synthesis methods in the patent, the inventors synthesized the two compounds and used them as control molecules. The two compounds have the following structural formulas:

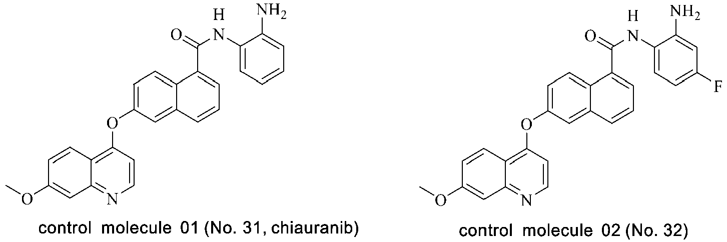

The results in Table 2 show that: the compounds of the present disclosure exhibited both excellent inhibitory activity against Aurora B kinase and very high selectivity for Aurora B kinase over VEGFR2. Specifically, compared with control molecule 01 (chiauranib), control molecule 02, and control examples I-7, I-11, I-12, I-13, and I-14, the compounds of the present disclosure exhibited not only stronger inhibitory effects on Aurora B kinase in terms of activity (their IC50 values against Aurora B were below 10 nM), but also very high selectivity for Aurora B kinase over VEGFR2 (their IC_{50-VEGFR2}/IC_{50-Aurora B} values were above 7), indicating that unexpected technical effects were achieved.

### Example B. Assessment of Effects of Compounds of the Present Disclosure on Tumor Cell Cycle

When the activity of Aurora B kinase is inhibited in tumor cells, the arrangement and separation of chromosomes are blocked, leading to cytokinesis failure and the formation of tetraploid or polyploid cells. Moreover, severe genomic instability and mitotic catastrophe result in apoptosis. The intracellular DNA content can be mapped with propidium iodide (PI), a fluorescent dye that specifically binds to nucleic acid, so that cell states can be distinguished into sub-G₀/G₁ (apoptosis), G₀/G₁, S, G₂/M (tetraploid), and polyploid states. Among them, sub-G₀/G₁ represents the DNA content < 2N, which generally indicates that the cells are undergoing apoptosis (random DNA degradation and cell membrane permeation); the polyploid state is a state with the DNA content > 4N.

### Experimental process:

Molt-4 cells (human acute lymphoblastic leukemia cells, CRL-1582) were purchased from the American Type Culture Collection (ATCC). Molt-4 cells in the logarithmic growth phase were collected from suspension culture and counted. The cells were seeded into a 6-well plate at 10⁶ cells/well and cultured overnight under normal conditions. The test compounds were prepared as 1 mM and 3 mM solutions in DMSO, and the solutions in DMSO were diluted 50-fold with culture medium. Finally, the above solutions were added to the culture system in a volume ratio of 1:19, achieving a final compound concentration of 1 µM or 3 µM in each well. After 48 h of incubation, the cells were collected by centrifugation. Each cell sample was resuspended in 300 µL of PBS, and the suspension was added dropwise to 700 µL of pre-cooled absolute ethanol. Then the mixture was gently inverted several times for thorough mixing to complete cell collection and fixation. The samples were left to stand at 4 °C for more than 12 h and then analyzed by flow cytometry.

A working dye solution was prepared by homogeneously mixing PBS, a 10 mg/mL PI stock solution, and a 10 mg/mL RNase A solution in a ratio of 1000:5:2. The fixed cell samples were centrifuged at 4 °C and 1000 rpm for 10 min. The supernatants were completely removed using a pipette, and the cells were washed twice with PBS and resuspended in the above working dye solution at 300 µL/tube. After incubation in a dark place at 37 °C for 30 min, the cells were filtered using a 200-mesh stainless steel strainer, and the filtrate was loaded onto a flow cytometer for cell cycle analysis (10,000 cells counted per sample).

Histograms showing DNA contents were obtained from the flow cytometry analysis, and the cell cycle distribution in each sample was analyzed using the flow cytometry data analysis software FlowJo. The mean (X) and standard deviation (SD) were calculated, and data were represented as X ± SD. The apoptosis and the proportions of tetraploid and polyploid cells after Molt-4 cells were treated with different doses of the test compounds are shown in Table 3 below.

**Table 3. The results of the assessment of the effects of the compounds of the present disclosure on the Molt-4 cell cycle**

| **Example** | **Aurora B IC₅₀ (nM)** | **Dose (µM)** | **Apoptosis (%)** | **Tetraploid (%)** | **Polyploid (%)** | **Total** |
|---|---|---|---|---|---|---|
| | | | **DNA<2N** | **DNA=4N** | **DNA>4N** | |
| **I-9** | 1.795 | 1 | 24.60±8.06 | 6.07 ±7.25 | 55.15±15.20 | 85.82 |
| | | 3 | 21.35±5.87 | 5.39±6.10 | 56.10±12.02 | 82.84 |
| **I-37** | 2.63 | 1 | 22.55±0.21 | 4.68±3.46 | 55.25±5.02 | 82.48 |
| | | 3 | 23.75±2.33 | 5.75±3.15 | 53.75±4.17 | 83.25 |
| **Control molecule 01 (chiauranib)** | 10.83 | 1 | 10.23 ±0.67 | 14.80±6.51 | 3.73±2.95 | 28.76 |
| | | 3 | 16.40±6.65 | 16.25±8.13 | 6.40±5.66 | 39.05 |
| **Control molecule 02** | 2.078 | 1 | 10.07±1.18 | 18.05±0.35 | 7.94±3.77 | 36.06 |
| | | 3 | 23.80±2.26 | 8.40±4.96 | 55.70±1.41 | 87.90 |
| **Blank control** | - | - | 6.76±0.27 | 10.37±1.04 | 5.44±0.58 | 22.57 |

The results in Table 3 show that: at both doses of 1 µM and 3 µM, the compounds of the present disclosure exhibited significantly improved effects of inducing tetraploidy, polyploidy, and apoptosis in Molt-4 cells compared with control molecule 01 (chiauranib) and control molecule 02, indicating that the compounds of the present disclosure had a stronger inhibitory effect on Aurora B kinase activity in the tumor cells, thereby affecting the cell cycles of the tumor cells; the compounds of the present disclosure can provide better comprehensive benefits in terms of efficacy/safety for related diseases.

### Example C. Brain Permeation Assay of Compounds of the Present Disclosure

The objective of this example was to test whether the compounds of the present disclosure are able to cross the blood-brain barrier (BBB). The compounds of the present disclosure were dissolved in a vehicle containing 5% dimethyl sulfoxide, 60% 1,3-propanediol, and 35% purified water, and intragastrically administered to B/C female mice aged 6 weeks (purchased from Guangdong Medical Laboratory Animal Center) at 5 mg/kg. The animals were sacrificed 4 h after the administration, and blood and brain tissue samples were collected. The blood samples were anticoagulated with K₂-EDTA and centrifuged at 5000 rpm for 5 min to separate plasma. The brain tissue samples were accurately weighed and homogenized with a certain amount of phosphate-buffered saline (PBS) solution, and the biological samples were stored at -80 °C before analysis. After sample extraction, the compound concentration was determined using a liquid chromatography-tandem mass spectrometry (LC-MS/MS) system. Brain permeation was defined as the ratio of the compound concentration in brain tissue to the concentration in plasma.

**Table 4. Data from the brain permeation experiment**

| **Compound** | **Control molecule 01 (chiauranib)** | **Control molecule 02** | **I-37** | **I-15** | **I-17** | **I-1** | **I-9** | **I-20** |
|---|---|---|---|---|---|---|---|---|
| **Brain permeation** | 0.28 | 0.27 | 0.60 | 0.76 | 0.60 | 0.73 | 0.60 | 0.67 |

The results in Table 4 show that: in the brain permeation assay, the compounds of the present disclosure exhibited ratios of compound concentration in brain tissue to plasma concentration significantly higher than those of control molecule 01 (chiauranib) and control molecule 02. The results of this assay show that: the compounds of the present disclosure have superior brain barrier permeability, and thus are capable of crossing the blood-brain barrier and have the potential to achieve effective plasma concentrations in brain tissue, thereby demonstrating their applicability in the treatment and prevention of diseases associated with the central nervous system.

### Example D. Experiment of Anti-Tumor Efficacy of Compound of the Present Disclosure on PANC02 Mouse Pancreatic Cancer Model

A mouse PANC02 pancreatic cancer graft tumor model was constructed: the experimental animals were C57BL/6 mice (female, 6 weeks old), the tumor cells were PANC02 cells (mouse pancreatic cancer cells), and the cell culture medium was a DMEM culture medium containing 1% double antibiotic and 10% fetal bovine serum. PANC02 cells in the logarithmic growth phase and in good condition were collected and inoculated subcutaneously into the left flank of C57BL/6 mice at a concentration of 0.9 × 10⁶ cells/0.2 mL. The long diameters (a, mm) and short diameters (b, mm) of the tumors were measured using a vernier caliper, and tumor volume (V, mm³) was calculated using the formula: V = 1/2a × b². Tumor volume was measured 2-3 times weekly using the vernier caliper. When the mean tumor volumes of the mice reached 190 mm³ or above, the mice were divided into 4 groups of 8 and dosed, and the day of grouping was represented by day 0. The groups were as follows: a solvent control group (0.2% aqueous solution of CMC-Na), a compound I-9 group (10 mg/kg/day), a control molecule 01 (chiauranib) group (10 mg/kg/day), and a control molecule 02 group (10 mg/kg/day). All four groups received intragastric administration once daily for consecutive days, and the experiment was terminated on the day of the last dose.

The tumor volume results were represented as mean and standard error (Mean ± S.E.M). Intergroup comparisons were performed using the Mann Whitney test. If *p* < 0.05, the difference is considered to be statistically significant. The tumor volume inhibition rate (TGI) was calculated using the formula: TGI (%) = [1 - (Ti - T0)/(Vi - V0)] × 100% (Ti: mean tumor volume of the treatment group on day i of administration; T0: mean tumor volume of the treatment group on day 0 of administration; Vi: mean tumor volume of the solvent control group on day i of administration; V0: mean tumor volume of the solvent control group on day 0 of administration).

The mean tumor volumes and the tumor volume inhibition rates in the groups of mice on different days of administration are shown in Table 5, and the statistical results of the mean tumor volumes are shown in FIG. 1.

**Table 5. Mean tumor volumes and tumor volume inhibition rates**

| **Group** | **Mean tumor volumes ((mm³ ± SEM))** | | **Tumor volume inhibition rate (%TGI)** |
|---|---|---|---|
| | **Grouping and administration Day 0** | **Grouping and administration Day 9** | **Grouping and administration Day 9** |
| **Solvent control group** | 193±39.6 | 535.8±39.6 | -- |
| **Compound I-9 group** | 193.9±38.5 | 145.9±38.5 | 114.0% |
| **Control molecule 01 (chiauranib) group** | 191.5±33.9 | 278.4±35.6 | 74.6% |
| **Control molecule 02 group** | 192.7±36 | 299.3 ±68.2 | 68.9% |

As can be seen from FIG. 1: on day 9 of grouping and administration, the mice in the compound I-9 group had significantly reduced tumor volumes (p < 0.01) compared with those in the control molecule 01 (chiauranib) group; the mice in the compound I-9 group had significantly reduced tumor volumes (p < 0.05) compared with those in the control molecule 02 group; the mice in the compound I-9 group had significantly reduced tumor volumes (p < 0.001) compared with those in the solvent control group.

As can be seen from Table 5, the tumor volume inhibition rate (TGI: 114.0%) of the representative compound I-9 group was significantly better than that (TGI: 74.6%) of the control molecule 01 (chiauranib) group and that (TGI: 68.9%) of the control molecule 02 group.

In conclusion, the compound of the present disclosure has superior *in vivo* anti-tumor efficacy and is capable of significantly inhibiting tumor growth.

The above description merely represents preferred embodiments of the present disclosure. It should be noted that those of ordinary skill in the art can make several improvements and modifications without departing from the principles of the present disclosure, and these improvements and modifications shall fall within the protection scope of the present disclosure.

## Claims

1. A compound represented by formula (I) or a stereoisomer, tautomer, polymorph, co-crystal, solvate, metabolite, prodrug, deuterated compound, or pharmaceutically acceptable salt thereof: 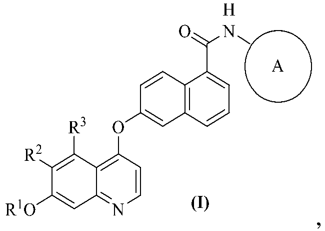 wherein:
R¹ is selected from methyl and ethyl; the methyl or ethyl is unsubstituted or substituted with one or more identical or different R^{a};
each R^{a} is independently selected from halogen, C₁₋₆ alkoxy, -NR'R", and halogenated C₁₋₆ alkoxy; wherein R' and R" are each independently selected from H, C₁₋₆ alkyl, and C₃₋₆ cycloalkyl, or R' and R", together with the N atom to which they are attached, form a 4-8 membered nitrogen-containing heterocyclyl; the 4-8 membered nitrogen-containing heterocyclyl may additionally comprise 1-2 heteroatoms selected from N, O, and S;
R² and R³ are each independently selected from: H, halogen, and C₁₋₆ alkyl;
ring A is selected from C₄₋₁₀ cycloalkyl and 4-10 membered heterocyclyl; the 4-10 membered heterocyclyl comprises 1 to 4 heteroatoms selected from N, O, and S; the C₄₋₁₀ cycloalkyl or 4-10 membered heterocyclyl is unsubstituted or substituted with one or more identical or different R^{b};
each R^{b} is independently selected from halogen, C₁₋₆ alkyl, and halogenated C₁₋₆ alkyl; or, ring A is selected from the groups: 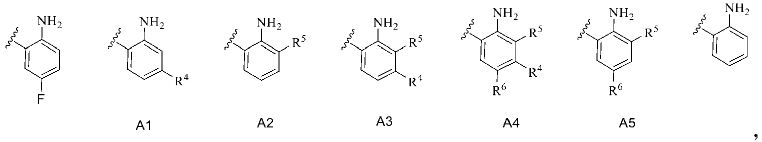 wherein:
in A1, R⁴ is selected from F, Cl, C₁₋₆ alkyl, and halogenated C₁₋₆ alkyl;
in A2, R⁵ is selected from halogen, C₁₋₆ alkyl, and halogenated C₁₋₆ alkyl;
in A3, R⁴ and R⁵ are each independently selected from halogen, C₁₋₆ alkyl, and halogenated C₁₋₆ alkyl;
in A4, R⁶ is selected from F and Cl, and R⁴ and R⁵ are each independently selected from halogen, C₁₋₆ alkyl, and halogenated C₆ alkyl; when R⁶ is Cl, R¹ is methyl or ethyl;
in A5, R⁵ and R⁶ are each independently selected from halogen, C₁₋₆ alkyl, and halogenated C₁₋₆ alkyl;
when ring A is 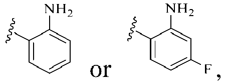 R¹ is ethyl, or methyl or ethyl substituted with one or more R³.

2. The compound or the stereoisomer, tautomer, polymorph, co-crystal, solvate, metabolite, prodrug, deuterated compound, or pharmaceutically acceptable salt thereof according to claim 1, wherein:
R¹ is selected from methyl and ethyl; the methyl or ethyl is unsubstituted or substituted with 1, 2, or 3 identical or different R³;
preferably, each R³ is independently selected from halogen, C₁₋₄ alkoxy, -NR'R", and halogenated C₁₋₄ alkoxy; wherein R' and R" are each independently selected from H, C₁₋₄ alkyl, and C₃₋₅ cycloalkyl, or R' and R", together with the N atom to which they are attached, form a 4-8 membered nitrogen-containing heterocyclyl; the 4-8 membered nitrogen-containing heterocyclyl may additionally comprise 1-2 heteroatoms selected from N, O, and S;
preferably, each R³ is independently selected from halogen, C₅₋₆ alkoxy, -NR'R", and halogenated C₅₋₆ alkoxy; wherein R' and R" are each independently selected from H, C₅₋₆ alkyl, and C₅₋₆ cycloalkyl, or R' and R", together with the N atom to which they are attached, form a 4-8 membered nitrogen-containing heterocyclyl; the 4-8 membered nitrogen-containing heterocyclyl may additionally comprise 1-2 heteroatoms selected from N, O, and S;
preferably, each R³ is independently selected from halogen, C₁₋₃ alkoxy, -NR'R", and halogenated C₁₋₃ alkoxy; wherein R' and R" are each independently selected from H, C₁₋₃ alkyl, and C₃₋₄ cycloalkyl, or R' and R", together with the N atom to which they are attached, form a 4-8 membered nitrogen-containing heterocyclyl; the 4-8 membered nitrogen-containing heterocyclyl may additionally comprise 1-2 heteroatoms selected from N, O, and S;
preferably, each R^{a} is independently selected from halogen, methoxy, ethoxy, propoxy, -NR'R", halogenated methoxy, halogenated ethoxy, and halogenated propoxy; wherein R' and R" are each independently selected from H, methyl, and ethyl, or R' and R", together with the N atom to which they are attached, form a 4-8 membered nitrogen-containing heterocyclyl; the 4-8 membered nitrogen-containing heterocyclyl may additionally comprise 1-2 heteroatoms selected from N, O, and S;
preferably, each R^{a} is independently selected from F, Cl, Br, methoxy, ethoxy, propoxy, -NR'R", halogenated methoxy, halogenated ethoxy, and halogenated propoxy; wherein R' and R" are each independently selected from H, methyl, and ethyl, or R' and R", together with the N atom to which they are attached, form a 4-7 membered nitrogen-containing heterocyclyl; the 4-7 membered nitrogen-containing heterocyclyl may additionally comprise 1-2 heteroatoms selected from N, O, and S;
preferably, each R^{a} is independently selected from F, Cl, Br, methoxy, ethoxy, -NR'R", and methoxy or ethoxy substituted with 1-3 F or Cl; wherein R' and R" are each independently selected from methyl and ethyl, or R' and R", together with the N atom to which they are attached, form a 4-7 membered nitrogen-containing heterocyclyl; the 4-7 membered nitrogen-containing heterocyclyl may additionally comprise 1-2 heteroatoms selected from N, O, and S;
preferably, each R^{a} is independently selected from F, Cl, methoxy, ethoxy, -NR'R", -OCH₂F, -OCHF₂, -OCF₃, -OCH₂CH₂F, -OCH₂CHF₂, and -OCH₂CF₃; wherein R' and R" are each independently selected from methyl and ethyl, or R' and R", together with the N atom to which they are attached, form 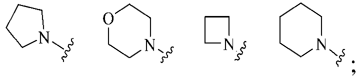
preferably, each R^{a} is independently selected from F, Cl, methoxy, N,N-dimethylamino, N-methylamino, -OCHF₂, and 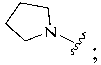
preferably, R¹ is selected from methyl, ethyl, -CH₂CH₂F, -CH₂CHF₂, -CH₂CH₂Cl, methoxyethyl, 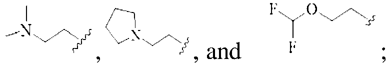
preferably, when ring A is 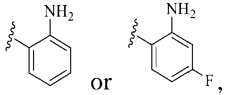 R¹ is ethyl or -CH₂CH₂F;
preferably, R¹ is selected from methyl and ethyl, and the methyl or ethyl is unsubstituted.

3. The compound or the stereoisomer, tautomer, polymorph, co-crystal, solvate, metabolite, prodrug, deuterated compound, or pharmaceutically acceptable salt thereof according to claim 1, wherein:
ring A is selected from C₄₋₇ monocycloalkyl, C₅₋₁₀ spirobicycloalkyl, 4-7 membered monoheterocyclyl, and 6-10 membered spirobiheterocyclyl; the 4-7 membered monoheterocyclyl or 6-10 membered spirobiheterocyclyl comprises 1 to 4 heteroatoms selected from N, O, and S; the C₄₋₇ monocycloalkyl, C₅₋₁₀ spirobicycloalkyl, 4-7 membered monoheterocyclyl, or 6-10 membered spirobiheterocyclyl is unsubstituted or substituted with 1, 2, or 3 identical or different R^{b};
ring A is selected from C₄₋₆ monocycloalkyl, C₆₋₈ spirobicycloalkyl, 4-7 membered monoheterocyclyl, and 6-8 membered spirobiheterocyclyl; the 4-7 membered monoheterocyclyl or 6-8 membered spirobiheterocyclyl comprises 1 to 2 heteroatoms selected from N, O, and S; the C₄₋₆ monocycloalkyl, C₆₋₈ spirobicycloalkyl, 4-7 membered monoheterocyclyl, or 6-8 membered spirobiheterocyclyl is unsubstituted or substituted with 1, 2, or 3 identical or different R^{b};
preferably, ring A is selected from 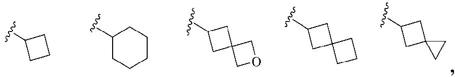 and the 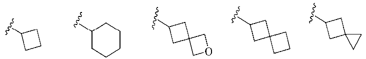 are unsubstituted or substituted with 1, 2, or 3 identical or different R^{b};
preferably, each R^{b} is independently selected from halogen and C₁₋₆ alkyl;
preferably, each R^{b} is independently selected from F, Cl, Br, C₁₋₄ alkyl, and C₅₋₆ alkyl;
preferably, each R^{b} is independently selected from F, Cl, Br, methyl, ethyl, C₃ alkyl, C₄ alkyl, C₅ alkyl, and C₆ alkyl;
preferably, each R^{b} is independently selected from F, Cl, Br, methyl, ethyl, propyl, isopropyl, butyl, tert-butyl, and sec-butyl;
preferably, each R^{b} is independently selected from F, Cl, Br, and methyl;
preferably, each R^{b} is independently selected from F;
preferably, ring A is selected from

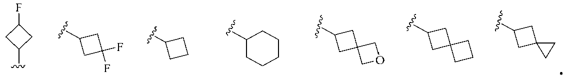

4. The compound or the stereoisomer, tautomer, polymorph, co-crystal, solvate, metabolite, prodrug, deuterated compound, or pharmaceutically acceptable salt thereof according to claim 1, wherein:
in A1, R⁴ is selected from F, Cl, C₁₋₄ alkyl, C₅₋₆ alkyl, halogenated C₁₋₄ alkyl, and halogenated C₅₋₆ alkyl;
preferably, in A1, R⁴ is selected from F, Cl, C₄ alkyl, and halogenated C₁₋₄ alkyl;
preferably, in A1, R⁴ is selected from F, Cl, methyl, ethyl, propyl, isopropyl, C₁₋₄ alkyl, halogenated methyl, halogenated ethyl, halogenated propyl, halogenated isopropyl, and halogenated C₄ alkyl;
preferably, in A1, R⁴ is selected from F, Cl, methyl, ethyl, methyl substituted with fluorine or chlorine, and ethyl substituted with fluorine or chlorine;
preferably, in A1, R⁴ is selected from F, Cl, and methyl;
or,
in A2, R⁵ is selected from F, Cl, Br, C₁₋₄ alkyl, C₅₋₆ alkyl, halogenated C₁₋₄ alkyl, and halogenated C₅₋₆ alkyl;
preferably, in A2, R⁵ is selected from F, Cl, Br, C₁₋₄ alkyl, and halogenated C₁₋₄ alkyl;
preferably, in A2, R⁵ is selected from F, Cl, Br, C₅₋₆ alkyl, and halogenated C₅₋₆ alkyl;
preferably, in A2, R⁵ is selected from F, Cl, Br, methyl, ethyl, propyl, isopropyl, C₄ alkyl, halogenated methyl, halogenated ethyl, halogenated propyl, halogenated isopropyl, and halogenated C₄ alkyl;
preferably, in A2, R⁵ is selected from F, Cl, Br, methyl, ethyl, methyl substituted with fluorine or chlorine, and ethyl substituted with fluorine or chlorine;
preferably, in A2, R⁵ is selected from F, Cl, Br, and methyl;
preferably, in A2, R⁵ is selected from F;
or,
in A3, R⁴ and R⁵ are each independently selected from halogen, C₁₋₄ alkyl, C₅₋₆ alkyl, halogenated C₁₋₄ alkyl, and halogenated C₅₋₆ alkyl;
preferably, in A3, R⁴ and Rare each independently selected from F, Cl, Br, C₄ alkyl, and halogenated C₁₋₄ alkyl;
preferably, in A3, R⁴ and Rare each independently selected from F, Cl, Br, methyl, ethyl, propyl, isopropyl, C₄ alkyl, halogenated methyl, halogenated ethyl, halogenated propyl, halogenated isopropyl, and halogenated C₄ alkyl;
preferably, in A3, R⁴ and Rare each independently selected from F, Cl, Br, methyl, ethyl, methyl substituted with fluorine or chlorine, and ethyl substituted with fluorine or chlorine;
preferably, in A3, R⁴ and R⁵ are each independently selected from F and Cl;
preferably, in A3, R⁴ is F, and R⁵ is F or Cl;
or,
in A4, R⁶ is selected from F and Cl, and R⁴ and R⁵ are each independently selected from halogen, C₁₋₄ alkyl, C₅₋₆ alkyl, halogenated C₁₋₄ alkyl, and halogenated C₅₋₆ alkyl; when R⁶ is Cl, R¹ is methyl or ethyl;
preferably, in A4, R⁶ is selected from F and Cl, and R⁴ and R⁵ are each independently selected from F, Cl, Br, C₁₋₄ alkyl, and halogenated C₁₋₄ alkyl; when R⁶ is Cl, R' is methyl or ethyl;
preferably, in A4, R⁶ is selected from F and Cl, and R⁴ and R⁵ are each independently selected from F, Cl, Br, methyl, ethyl, propyl, isopropyl, C₄ alkyl, halogenated methyl, halogenated ethyl, halogenated propyl, halogenated isopropyl, and halogenated C₄ alkyl; when R⁶ is Cl, R¹ is methyl or ethyl;
preferably, in A4, R⁶ is selected from F and Cl; R⁴ and R⁵ are each independently selected from F, Cl, Br, methyl, ethyl, methyl substituted with fluorine or chlorine, and ethyl substituted with fluorine or chlorine; when R⁶ is Cl, R¹ is methyl or ethyl;
preferably, in A4, R⁶ is selected from F and Cl, and R⁴ and R⁵ are each independently selected from F and Cl; when R⁶ is Cl, R¹ is methyl or ethyl;
or,
in A5, R⁵ and R⁶ are each independently selected from halogen, C₁₋₄ alkyl, C₅₋₆ alkyl, halogenated C₁₋₄ alkyl, and halogenated C₅₋₆ alkyl;
preferably, in A5, R⁵ and R⁶ are each independently selected from F, Cl, Br, C₄ alkyl, and halogenated C₁₋₄ alkyl;
preferably, in A5, R⁵ and R⁶ are each independently selected from F, Cl, Br, methyl, ethyl, propyl, isopropyl, C₄ alkyl, halogenated methyl, halogenated ethyl, halogenated propyl, halogenated isopropyl, and halogenated C₄ alkyl;
preferably, in A5, R⁵ and R⁶ are each independently selected from F, Cl, Br, methyl, ethyl, methyl substituted with fluorine or chlorine, and ethyl substituted with fluorine or chlorine;
preferably, in A5, R⁵ and R⁶ are each independently selected from F and Cl; 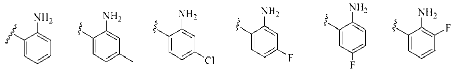
preferably, ring A is selected from 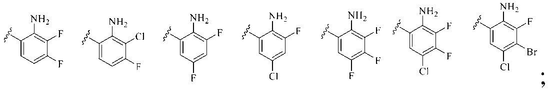
when ring A is selected from 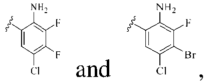 R¹ is methyl or ethyl;
preferably, ring A is selected from

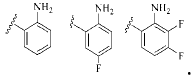

5. The compound or the stereoisomer, tautomer, polymorph, co-crystal, solvate, metabolite, prodrug, deuterated compound, or pharmaceutically acceptable salt thereof according to claim 1, wherein:
R² and R³ are each independently selected from H, halogen, C₁₋₄ alkyl, and C₅₋₆ alkyl;
preferably, R² and R³ are each independently selected from H, F, Cl, Br, methyl, ethyl, propyl, isopropyl, butyl, pentyl, and hexyl;
preferably, R² and R³ are each independently selected from H, F, Cl, and Br;
preferably, R² and R³ are each independently selected from H and F;
preferably, R² and R³ are both H;
or preferably, one of R² and R³ is H, and the other is F;
preferably, R² is H, and R³ is F;
or preferably, R² is F, and R³ is H.

6. The compound or the stereoisomer, tautomer, polymorph, co-crystal, solvate, metabolite, prodrug, deuterated compound, or pharmaceutically acceptable salt thereof according to any one of claims 1-5, wherein the compound is selected from:

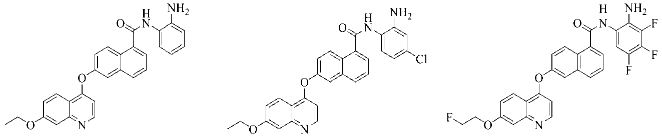

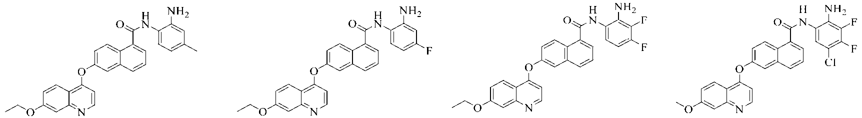

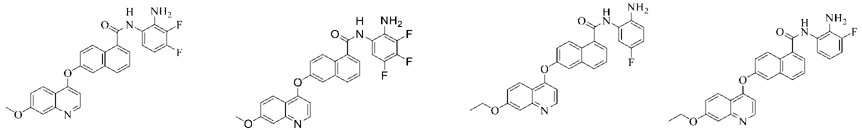

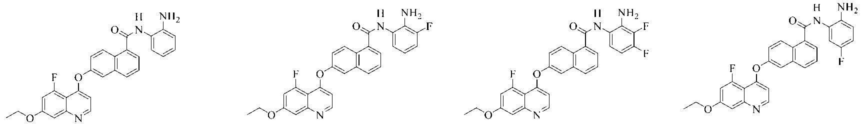

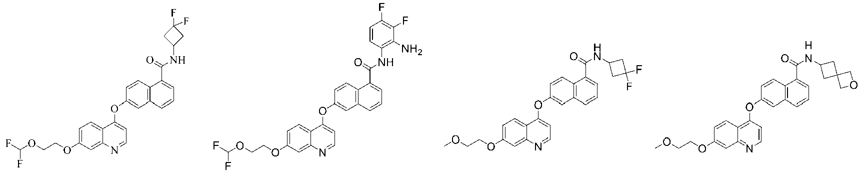

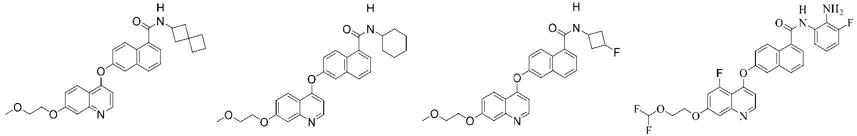

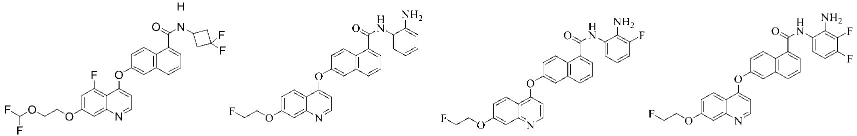

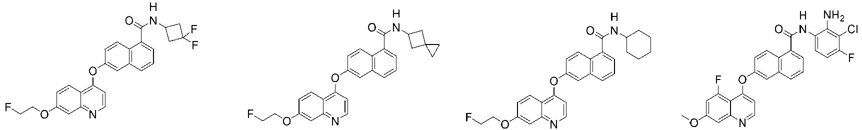

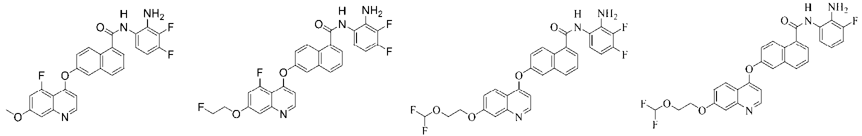

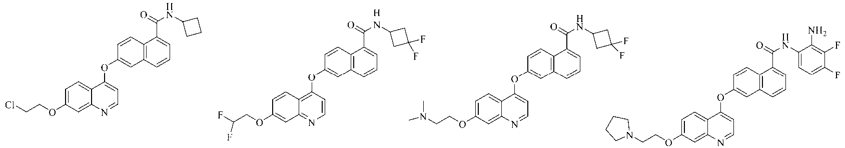

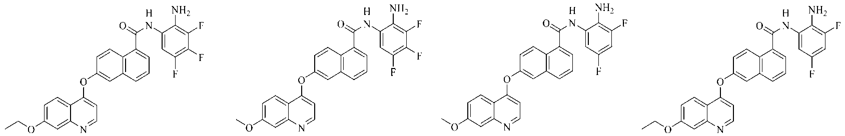

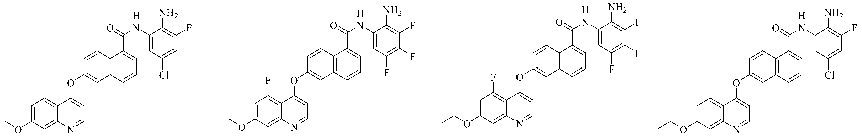

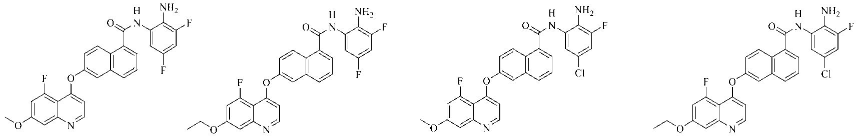

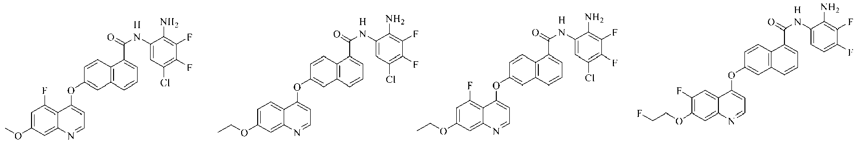

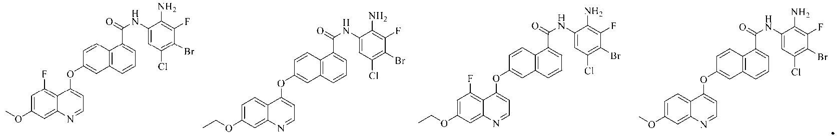

7. A pharmaceutical composition, comprising the compound or the stereoisomer, tautomer, polymorph, co-crystal, solvate, metabolite, prodrug, deuterated compound, or pharmaceutically acceptable salt thereof according to any one of claims 1-6, and optionally a pharmaceutically acceptable carrier and/or adjuvant and/or diluent.

8. Use of the compound or the stereoisomer, tautomer, polymorph, co-crystal, solvate, metabolite, prodrug, deuterated compound, or pharmaceutically acceptable salt thereof according to any one of claims 1-6 or the pharmaceutical composition according to claim 7 in the manufacture of a medicament for treating and/or preventing an Aurora B kinase-associated disease,
preferably, the Aurora B kinase-associated disease is selected from tumors and hyperproliferative diseases.

9. A method for treating and/or preventing an Aurora B kinase-associated disease, comprising administering a therapeutically and/or prophylactically effective amount of the compound or the stereoisomer, tautomer, polymorph, co-crystal, solvate, metabolite, prodrug, deuterated compound, or pharmaceutically acceptable salt thereof according to any one of claims 1-6 or the pharmaceutical composition according to claim 7 to an individual in need thereof,
preferably, the Aurora B kinase-associated disease is selected from tumors and hyperproliferative diseases.

10. The compound or the stereoisomer, tautomer, polymorph, co-crystal, solvate, metabolite, prodrug, deuterated compound, or pharmaceutically acceptable salt thereof according to any one of claims 1-6 or the pharmaceutical composition according to claim 7 for use in the treatment and/or prevention of an Aurora B kinase-associated disease, preferably, the Aurora B kinase-associated disease is selected from tumors and hyperproliferative diseases.
